# EUROPEAN PATENT APPLICATION

(11) **EP 2 018 855 A1**
(43) Date of publication of application: **28.01.2009**
(21) Application number: 07743343.1
(22) Date of filing: 14.05.2007
(51) Int. Cl.: A61K 31/215, A61P 9/00, A61P 25/00, A61P 29/00, A61P 31/04, A61P 37/02, A61P 43/00

(54) **PHARMACEUTICAL AGENT**

(30) Priority: 15.05.2006 JP 2006135883
(71) Applicant: Takeda Pharmaceutical Company Limited, Osaka-shi, Osaka 541-0045 (JP)
(72) Inventor: II, Masayuki, 2-chome, Yodokawa-ku, Osaka-shi, Osaka 540-8686 (JP); MATSUDA, Hideyasu, Chuo-ku,Osaka-shi, Osaka 540-8645 (JP); MOURI, Kouji, Osaka-shi, Osaka 540-8645 (JP)
(74) Representative: Kalhammer, Georg
(86) International application number: PCT/JP2007/059908
(87) International publication number: WO 2007/132825

(57) **Abstract**

The present invention provides an agent for the prophylaxis or treatment of complications after coronary-artery bypass surgery or cardiac diseases, autoimmune diseases, central nervous system diseases, inflammatory diseases, sepsis, severe sepsis or septic shock in a patient who undergoes coronary-artery bypass surgery, which comprises a compound represented by the formula (I): 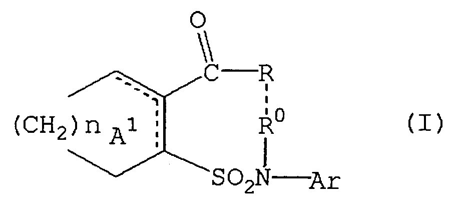 wherein each symbol is as defined in the specification, or a compound represented by the formula (II): 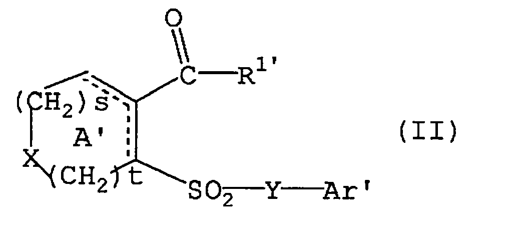 wherein each symbol is as defined in the specification, or a salt thereof or a prodrug thereof, and an agent for the prophylaxis or treatment of sepsis and the like, as well as complications after coronary-artery bypass surgery, which is prepared for administration of ethyl (6R)-6-[(2-chloro-4-fluoroanilino)sulfonyl]-1-cyclohexene-1-carboxylate or a salt thereof or a prodrug thereof in a specific dose at a specific administration time.

## Description

### Technical field

The present invention relates to an agent for the prophylaxis or treatment of complications after coronary-artery bypass surgery, which comprises a specific cycloalkene compound having an inducible nitric oxide synthase-derived nitric oxide (NO) production suppressive action and/or a suppressive action on the production of inflammatory cytokines such as TNF-α, IL-1, IL-6 and the like, and an agent for the prophylaxis or treatment of cardiac diseases, autoimmune diseases, central nervous system diseases, inflammatory diseases, sepsis, severe sepsis, septic shock or complications after coronary-artery bypass surgery, which is prepared for administration of the cycloalkene compound in a specific dose at a specific administration time.

### Background of the Invention

Patent reference 1 describes that (i) a compound represented by the formula:

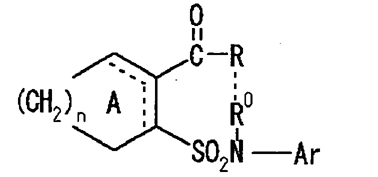

wherein
R is an aliphatic hydrocarbon group optionally having substituent(s), an aromatic hydrocarbon group optionally having substituent(s), a heterocyclic group optionally having substituent(s), a group represented by the formula: -OR¹ wherein R¹ is a hydrogen atom or an aliphatic hydrocarbon group optionally having substituent(s), or a group represented by the formula:

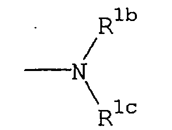

wherein R^{1b} is a hydrogen atom or an aliphatic hydrocarbon group optionally having substituent(s), and R^{1c} is the same as or different from R^{1b}, a hydrogen atom or an aliphatic hydrocarbon group optionally having substituent(s),
R⁰ is a hydrogen atom or an aliphatic hydrocarbon group, or R and R⁰ in combination form a bond,
ring A is a cycloalkene substituted by 1 to 4 substituents selected from (1) an aliphatic hydrocarbon group optionally having substituent(s), (2) an aromatic hydrocarbon group optionally having substituent(s), (3) a group represented by the formula: -OR¹¹ wherein R¹¹ is a hydrogen atom or an aliphatic hydrocarbon group optionally having substituent(s), and (4) a halogen atom,
Ar is an aromatic hydrocarbon group optionally having substituent(s),
a group represented by the formula:

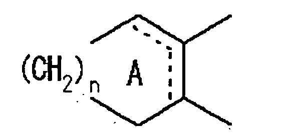

is a group represented by the formula:

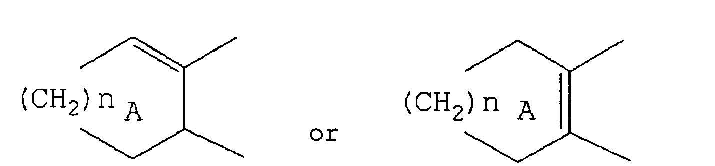

and n is an integer of 1 to 4, and
(ii) a compound represented by the formula:

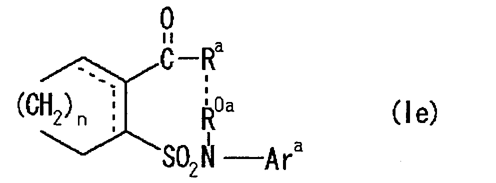

wherein
R^{a} is an aliphatic hydrocarbon group optionally having substituent(s), an aromatic hydrocarbon group optionally having substituent(s), a heterocyclic group optionally having substituent(s), a group represented by the formula: -OR^{1a} wherein R^{1a} is a hydrogen atom or an aliphatic hydrocarbon group optionally having substituent(s), or a group represented by the formula:

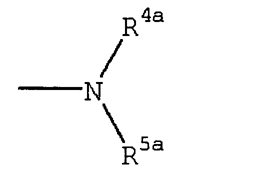

wherein R^{4a} and R^{5a} are the same or different and each is a hydrogen atom or an aliphatic hydrocarbon group optionally having substituent(s),
R^{0a} is a hydrogen atom or an aliphatic hydrocarbon group, or
R^{a} and R^{0a} in combination form a bond,
Ar^{a} is an aromatic hydrocarbon group optionally having substituent(s),
a group represented by the formula:

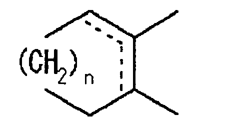

is a group represented by the formula:

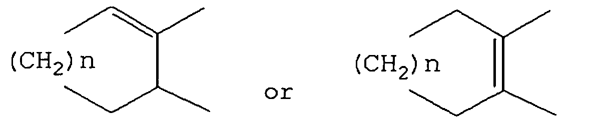

and n is an integer of 1 to 4,
a salt thereof and a prodrug thereof have a nitric oxide (NO) production-inhibiting effect and an inhibitory effect on the production of inflammatory cytokines, such as TNF-α, IL-1, IL-6 and the like, and are useful as an agent for the prophylaxis or treatment of diseases including cardiac diseases,
autoimmune diseases, inflammatory diseases, central nervous system diseases, infectious diseases, sepsis, septic shock and the like; and
patent reference 2 describes that a compound represented by the formula:

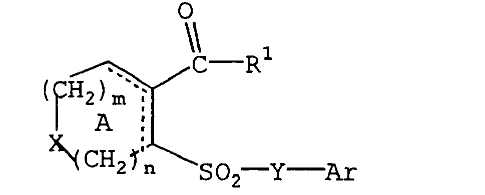

wherein
R¹ is an aliphatic hydrocarbon group optionally having substituent(s), an aromatic hydrocarbon group optionally having substituent(s), a heterocyclic group optionally having substituent(s), a group represented by the formula: -OR^{1a} wherein R^{1a} is a hydrogen atom or an aliphatic hydrocarbon group optionally having substituent(s), or a group represented by the formula:

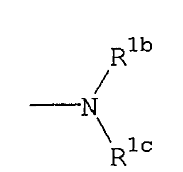

wherein R^{1b} and R^{1c} are the same or different and each is a hydrogen atom or an aliphatic hydrocarbon group optionally having substituent(s),
X is a methylene group, NH, a sulfur atom or an oxygen atom,
Y is a methylene group optionally having substituent(s) or NH optionally having substituent(s),
ring A is a 5- to 8-membered ring optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR² wherein R² is a hydrogen atom or an aliphatic hydrocarbon group optionally having substituent(s), and (4) a halogen atom,
Ar is an aromatic hydrocarbon group optionally having substituent(s),
a group represented by the formula:

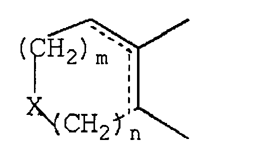

is a group represented by the formula:

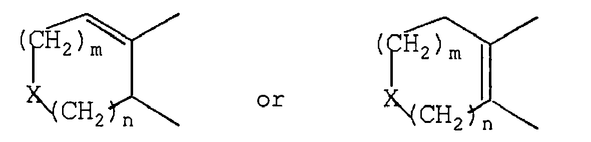

m is an integer of 0 to 2,
n is an integer of 1 to 3, and
the total of m and n is 4 or less;
provided that when X is a methylene group, then Y should be a methylene group optionally having substituent(s),
a salt thereof and a prodrug thereof
have a nitric oxide (NO) production-inhibiting effect and an inhibitory effect on the production of inflammatory cytokines, such as TNF-α, IL-1, IL-6 and the like, and are useful as an agent for the prophylaxis or treatment of diseases including cardiac diseases, autoimmune diseases, inflammatory diseases, central nervous system diseases, infectious diseases, sepsis, septic shock and the like.
Patent reference 3 describes that the above-mentioned compound is useful as a TLR signaling inhibitor or an agent for the prophylaxis or treatment of severe sepsis.
patent reference 1: WO99/46242
patent reference 2: WO01/10826
patent reference 3: WO03/84527

### Disclosure of the Invention

### Problems to be Solved by the Invention

The present invention aims to provide a pharmaceutical agent effective for the prophylaxis or treatment of complications after coronary-artery bypass surgery, and a pharmaceutical agent capable of efficiently preventing or treating sepsis and the like.

### Means of Solving the Problems

The present inventors have conducted intensive studies in an attempt to solve the aforementioned problems and found that the above-mentioned cycloalkene compound is unexpectedly also effective for ischemic reperfusion disorders such as complications after coronary-artery bypass surgery and the like, postoperative complications, tissue disorders and rejection after organ transplantation, and further that sepsis and the like can be unexpectedly efficiently prevented or treated by the administration of the cycloalkene compound in a specific dose at a specific administration time. Further studies made by the present inventors based on these findings have resulted in the completion of the present invention.

Accordingly, the present invention relates to [1] an agent for the prophylaxis or treatment of complications after coronary-artery bypass surgery, which comprises a compound represented by the formula (I):

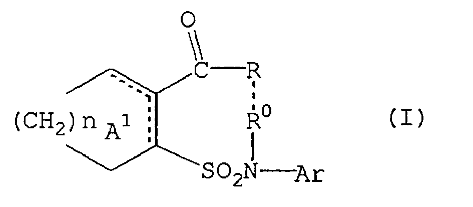

wherein
R is an aliphatic hydrocarbon group optionally having substituent(s), an aromatic hydrocarbon group optionally having substituent(s), a heterocyclic group optionally having substituent(s), a group represented by the formula: -OR¹ wherein R¹ is a hydrogen atom or an aliphatic hydrocarbon group optionally having substituent(s), or a group represented by the formula:

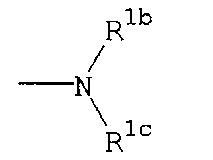

wherein R^{1b} and R^{1c} are the same or different and each is a hydrogen atom or an aliphatic hydrocarbon group optionally having substituent(s),
R⁰ is a hydrogen atom or an aliphatic hydrocarbon group, or R and R⁰ in combination form a bond,
ring A¹ is a cycloalkene optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR¹¹ wherein R¹¹ is a hydrogen atom or an aliphatic hydrocarbon group optionally having substituent(s), and (4) a halogen atom,
Ar is an aromatic hydrocarbon group optionally having substituent(s),
a group represented by the formula:

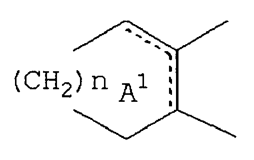

is a group represented by the formula:

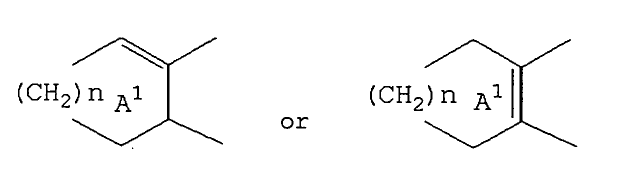

and n is an integer of 1 to 4,
or a salt thereof or a prodrug thereof, or
a compound represented by the formula (II):

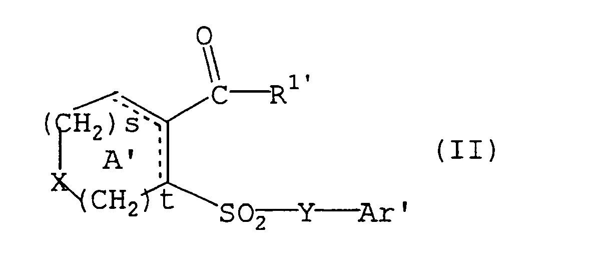

wherein
R1' is an aliphatic hydrocarbon group optionally having substituent(s), an aromatic hydrocarbon group optionally having substituent(s), a heterocyclic group optionally having substituent(s), a group represented by the formula: -OR^{1a'} wherein R^{1a'} is a hydrogen atom or an aliphatic hydrocarbon group optionally having substituent(s), or a group represented by the formula:

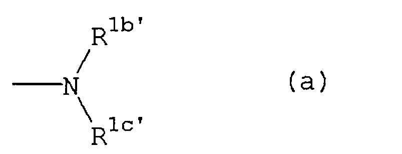

wherein R^{1b'} and R^{1c'} are the same or different and each is a hydrogen atom or an aliphatic hydrocarbon group optionally having substituent(s),
X is a methylene group, NH, a sulfur atom or an oxygen atom,
Y is a methylene group optionally having substituent(s) or NH optionally having substituent(s),
ring A' is a 5- to 8-membered ring optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR^{2'} wherein R^{2'} is a hydrogen atom or an aliphatic hydrocarbon group optionally having substituent(s), and (4) a halogen atom, Ar' is an aromatic hydrocarbon group optionally having substituent(s),
a group represented by the formula:

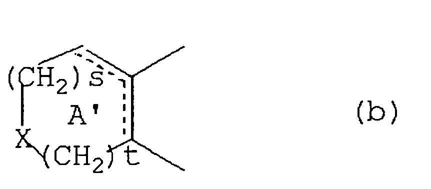

is a group represented by the formula:

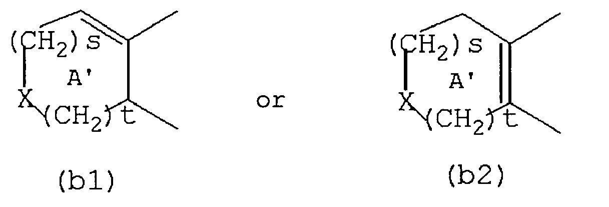

s is an integer of 0 to 2,
t is an integer of 1 to 3, and
the total of s and t is 4 or less;
provided that when X is a methylene group, then Y should be a methylene group optionally having substituent(s),
a salt thereof and a prodrug thereof;
[2] an agent for the prophylaxis or treatment of cardiac diseases, autoimmune diseases, central nervous system diseases, inflammatory diseases, sepsis, severe sepsis or septic shock in a patient who undergoes coronary-artery bypass surgery, which comprises a compound represented by the formula (I):

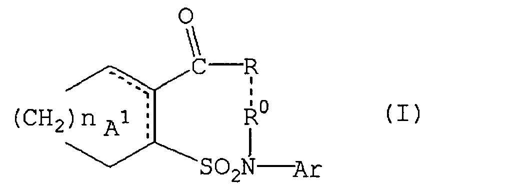

wherein
R is an aliphatic hydrocarbon group optionally having substituent(s), an aromatic hydrocarbon group optionally having substituent(s), a heterocyclic group optionally having substituent(s), a group represented by formula: -OR¹ wherein R¹ is a hydrogen atom or an aliphatic hydrocarbon group optionally having substituent(s), or a group represented by the formula:

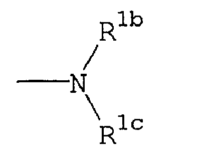

wherein R^{1b} and R^{1c} are the same or different and each is a hydrogen atom or an aliphatic hydrocarbon group optionally having substituent(s),
R⁰ is a hydrogen atom or an aliphatic hydrocarbon group, or R and R⁰ in combination form a bond,
ring A¹ is a cycloalkene optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR¹¹ wherein R¹¹ is a hydrogen atom or an aliphatic hydrocarbon group optionally having substituent(s), and (4) a halogen atom,
Ar is an aromatic hydrocarbon group optionally having substituent(s),
a group represented by the formula:

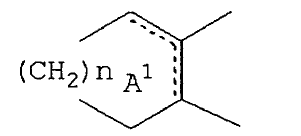

is a group represented by the formula:

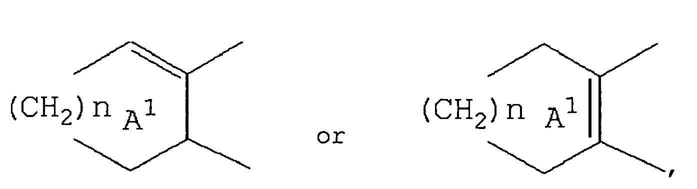

and n is an integer of 1 to 4,
or a salt thereof or a prodrug thereof, or
a compound represented by the formula (II):

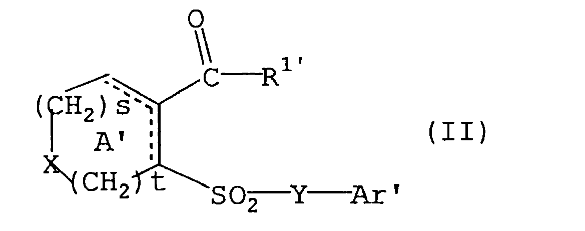

wherein
R^{1'} is an aliphatic hydrocarbon group optionally having substituent(s), an aromatic hydrocarbon group optionally having substituent(s), a heterocyclic group optionally having substituent(s), a group represented by the formula: -OR^{1a'} wherein R^{1a'} is a hydrogen atom or an aliphatic hydrocarbon group optionally having substituent(s), or a group represented by the formula:

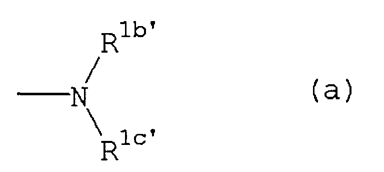

wherein R^{1b}' and R^{1c'} are the same or different and each is a hydrogen atom or an aliphatic hydrocarbon group optionally having substituent(s),
X is a methylene group, NH, a sulfur atom or an oxygen atom,
Y is a methylene group optionally having substituent(s) or NH optionally having substituent(s), ring A' is a 5- to 8-membered ring optionally having 1 to 4 substituents selected from the group consisting of (1) an aliphatic hydrocarbon group optionally having substituent(s), (2) an aromatic hydrocarbon group optionally having substituent(s), (3) a group represented by formula: -OR^{2'} wherein R^{2'} is a hydrogen atom or an aliphatic hydrocarbon
group optionally having substituent(s), and (4) a halogen atom, Ar' is an aromatic hydrocarbon group optionally having substituent(s),
a group represented by the formula:

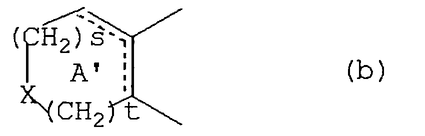

is a group represented by the formula:

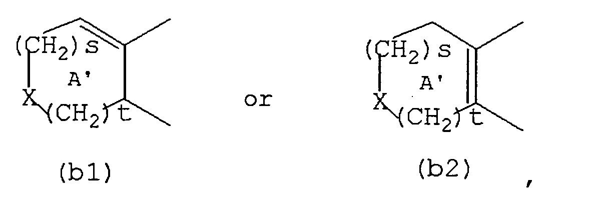

s is an integer of 0 to 2,
t is an integer of 1 to 3, and
the total of s and t is 4 or less;
provided that when X is a methylene group, then Y should be a methylene group optionally having substituent(s),
a salt thereof and a prodrug thereof,
[3] the agent of the above-mentioned [1] or [2], wherein the formula (I) is the formula (Ia):

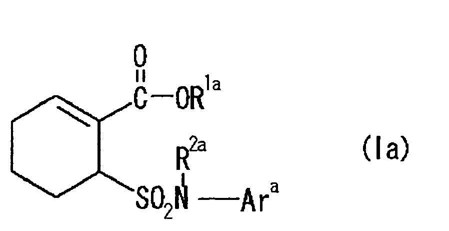

wherein R^{1a} is a Cₗ₋₆ alkyl group, R^{2a} is a hydrogen atom or a C₁₋₆ alkyl group, Ar^{a} is a phenyl group substituted by 1 or 2 halogen atoms, and the formula (II) is the formula (IIa):

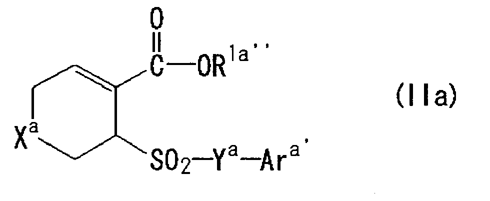

wherein R^{1a''} is a C₁₋₆ alkyl group, X^{a} is a methylene group or an oxygen atom, Y^{a} is a methylene group or -NH-, Ar^{a'} is a phenyl group optionally having 1 or 2 substituents selected from the group consisting of a halogen atom and a C₁₋₆ alkoxy group, provided that when X^{a} is a methylene group, then Y^{a} should be a methylene group,
[4] the agent of the above-mentioned [1] or [2], which is used in combination with at least one drug selected from the group consisting of antibacterial agents, antifungal agents, antivirus drugs, non-steroidal antiinflammatory drugs, steroids, anticoagulants, cytopathy suppressive agents and anti-sepsis drugs,
[5] a method for preventing or treating complications after coronary-artery bypass surgery, which comprises administering an effective amount of a compound represented by the formula (I):

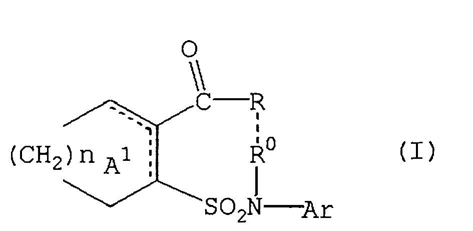

wherein
R is an aliphatic hydrocarbon group optionally having substituent(s), an aromatic hydrocarbon group optionally having substituent(s), a heterocyclic group optionally having substituent(s), a group represented by the formula: -OR¹ wherein R¹ is a hydrogen atom or an aliphatic hydrocarbon group optionally having substituent(s), or a group represented by the formula:

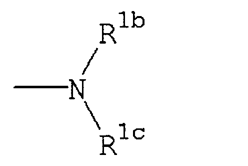

wherein R^{1b} and R^{1c} are the same or different and each is a hydrogen atom an aliphatic hydrocarbon group optionally having substituent(s),
R⁰ is a hydrogen atom or an aliphatic hydrocarbon group, or R and R⁰ in combination form a bond,
ring A¹ is a cycloalkene optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR¹¹ wherein R¹¹ is a hydrogen atom or an aliphatic hydrocarbon group optionally having substituent(s), and (4) a halogen atom,
Ar is an aromatic hydrocarbon group optionally having substituent(s),
a group represented by the formula:

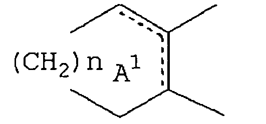

is a group represented by the formula:

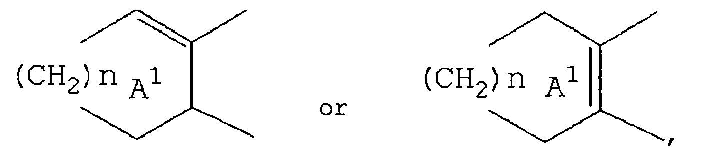

and n is an integer of 1 to 4,
or a salt thereof or a prodrug thereof, or
a compound represented by the formula (II):

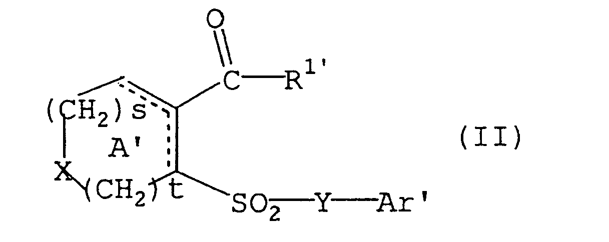

wherein
R^{1'} is an aliphatic hydrocarbon group optionally having substituent(s), an aromatic hydrocarbon group optionally having substituent(s), a heterocyclic group optionally having substituent(s), a group represented by the formula: -OR^{1a'} wherein R^{1a'} is a hydrogen atom or an aliphatic hydrocarbon group optionally having substituent(s), or a group represented by the formula:

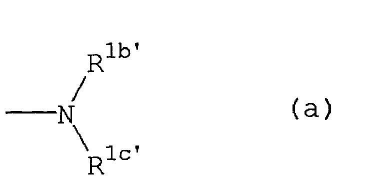

wherein R^{1b'} and R^{1c'} are the same or different and each is a hydrogen atom or an aliphatic hydrocarbon group optionally having substituent(s),
X is a methylene group, NH, a sulfur atom or an oxygen atom,
Y is a methylene group optionally having substituent(s) or NH optionally having substituent(s), ring A' is a 5- to 8-membered ring optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR^{2'} wherein R^{2'} is a hydrogen atom or an aliphatic hydrocarbon group optionally having substituent(s), and (4) a halogen atom, Ar' is a hydrocarbon group optionally having substituent(s), a group represented by the formula:

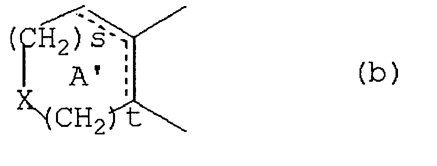

is a group represented by the formula:

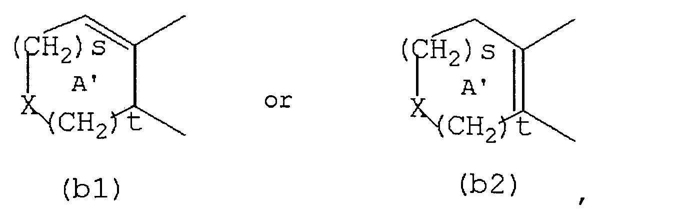

s is an integer of 0 to 2,
t is an integer of 1 to 3, and
the total of s and t is 4 or less;
provided that when X is a methylene group, then Y should be a methylene group optionally having substituent(s),
or a salt thereof or a prodrug thereof, to a mammal,
[6] use of a compound represented by the formula (I):

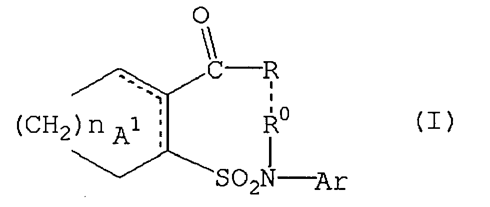

wherein
R is an aliphatic hydrocarbon group optionally having substituent(s), an aromatic hydrocarbon group optionally having substituent(s), a heterocyclic group optionally having substituent(s), a group represented by the formula: -OR¹ wherein R¹ is a hydrogen atom or an aliphatic hydrocarbon group optionally having substituent(s), or a group represented by the formula:

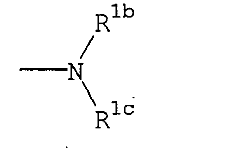

wherein R^{1b} and R^{1c} are the same or different and each is a hydrogen atom or an aliphatic hydrocarbon group optionally having substituent(s),
R⁰ is a hydrogen atom or an aliphatic hydrocarbon group, or R and R⁰ in combination form a bond,
ring A¹ is a cycloalkene optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR¹¹ wherein R¹¹ is a hydrogen atom or an aliphatic hydrocarbon group optionally having substituent(s), and (4) a halogen atom,
Ar is an aromatic hydrocarbon group optionally having substituent(s),
a group represented by the formula:

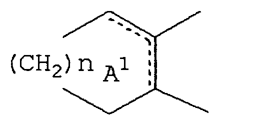

is a group represented by the formula:

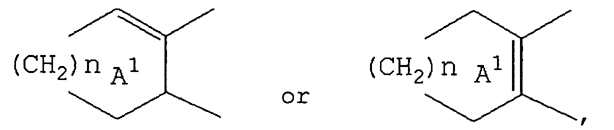

and n is an integer of 1 to 4,
or a salt thereof or a prodrug thereof, or
a compound represented by the formula (II):

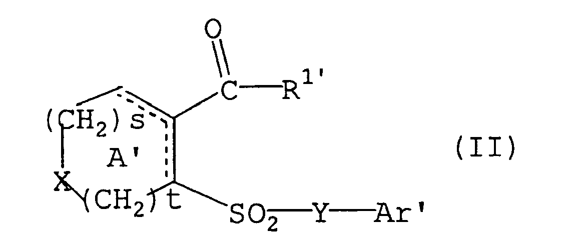

wherein
R^{1'} is an aliphatic hydrocarbon group optionally having substituent(s), an aromatic hydrocarbon group optionally having substituent(s), a heterocyclic group optionally having substituent(s), a group represented by the formula: -OR^{1a'} wherein R^{1a'} is a hydrogen atom or an aliphatic hydrocarbon group optionally having substituent(s), or a group represented by the formula:

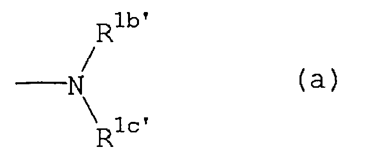

wherein R^{1b'} and R^{1c'} are the same or different and each is a hydrogen atom or an aliphatic hydrocarbon group optionally having substituent(s),
X is a methylene group, NH, a sulfur atom or an oxygen atom,
Y is a methylene group optionally having substituent(s) or NH optionally having substituent(s), ring A' is a 5- to 8-membered ring optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR^{2'} wherein R^{2'} is a hydrogen atom or an aliphatic hydrocarbon
group optionally having substituent(s), and (4) a halogen atom, Ar' is an aromatic hydrocarbon group optionally having substituent(s),
a group represented by the formula:

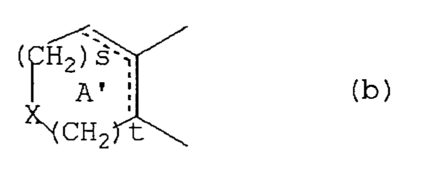

is a group represented by the formula:

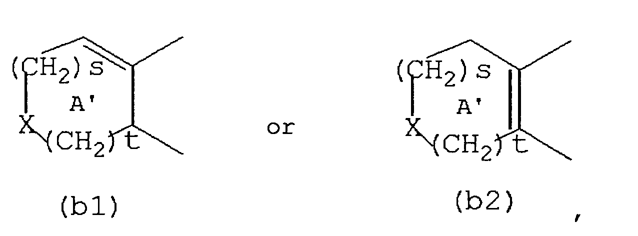

s is an integer of 0 to 2,
t is an integer of 1 to 3, and
the total of s and t is 4 or less;
provided that when X is a methylene group, then Y should be a methylene group optionally having substituent(s),
or a salt thereof or a prodrug thereof,
for the production of an agent for the prophylaxis or treatment of complications after coronary-artery bypass surgery,
[7] a compound represented by the formula (I):

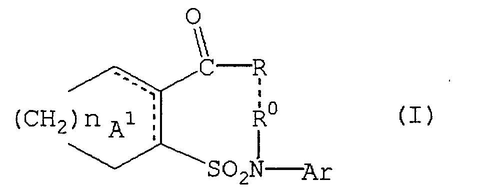

wherein
R is an aliphatic hydrocarbon group optionally having substituent(s), an aromatic hydrocarbon group optionally having substituent(s), a heterocyclic group optionally having substituent(s), a group represented by the formula: -OR¹ wherein R¹ is a hydrogen atom or an aliphatic hydrocarbon group optionally having substituent(s), or a group represented by the formula:

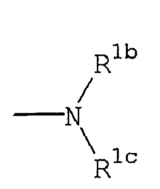

wherein R^{1b} and R^{1c} are the same or different and each is a hydrogen atom or an aliphatic hydrocarbon group optionally having substituent(s),
R⁰ is a hydrogen atom or an aliphatic hydrocarbon group, or R and R⁰ in combination form a bond,
ring A¹ is a cycloalkene optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR¹¹ wherein R¹¹ is a hydrogen atom or an aliphatic hydrocarbon group optionally having substituent(s), and (4) a halogen atom,
Ar is an aromatic hydrocarbon group optionally having substituent(s),
a group represented by the formula:

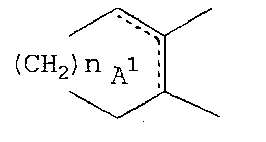

is a group represented by the formula:

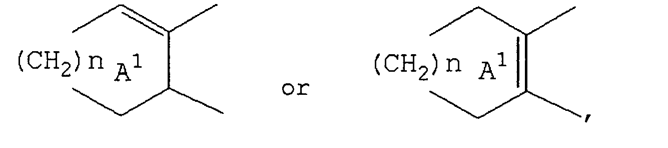

and n is an integer of 1 to 4,
or a salt thereof or a prodrug thereof, or
a compound represented by the formula (II):

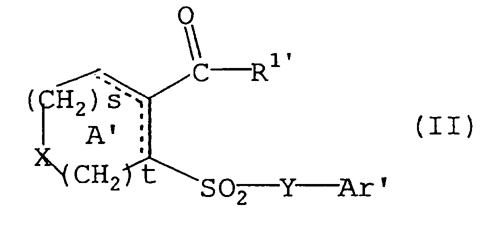

wherein
R^{1'} is an aliphatic hydrocarbon group optionally having substituent(s), an aromatic hydrocarbon group optionally having substituent(s), a heterocyclic group optionally having substituent(s), a group represented by the formula: -OR^{1a'} wherein R^{1a'} is a hydrogen atom or an aliphatic hydrocarbon group optionally having substituent(s), or a group represented by the formula:

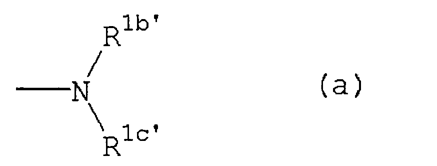

wherein R^{1b'} and R^{1c'} are the same or different and each is a hydrogen atom or an aliphatic hydrocarbon group optionally having substituent(s),
X is a methylene group, NH, a sulfur atom or an oxygen atom,
Y is a methylene group optionally having substituent(s) or NH optionally having substituent(s),
ring A' is a 5- to 8-membered ring optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR^{2'} wherein R^{2'} is a hydrogen atom or an aliphatic hydrocarbon group optionally having substituent(s), and (4) a halogen atom, Ar' is an aromatic hydrocarbon group optionally having substituent(s),
a group represented by the formula:

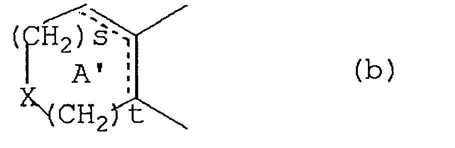

is a group represented by the formula:

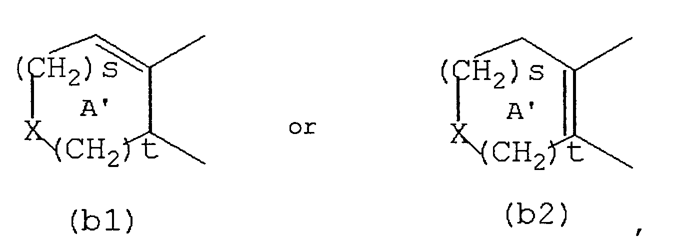

s is an integer of 0 to 2,
t is an integer of 1 to 3, and
the total of s and t is 4 or less;
provided that when X is a methylene group, then Y should be a methylene group optionally having substituent(s),
or a salt thereof or a prodrug thereof,
for the use as an agent for the prophylaxis or treatment of complications after coronary-artery bypass surgery,
[8] a method for the prophylaxis or treatment of cardiac disease, autoimmune diseases, central nervous system diseases, inflammatory disease, sepsis, severe sepsis or septic shock, which comprises administering an effective amount of a compound represented by the formula (I):

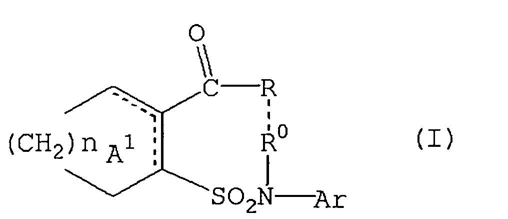

wherein
R is an aliphatic hydrocarbon group optionally having substituent(s), an aromatic hydrocarbon group optionally having substituent(s), a heterocyclic group optionally having substituent(s), a group represented by the formula: -OR¹ wherein R¹ is a hydrogen atom or an aliphatic hydrocarbon group optionally having substituent(s), or a group represented by the formula:

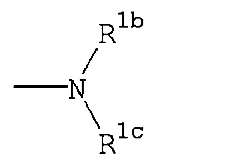

wherein R^{1b} and R^{1c} are the same or different and each is a hydrogen atom or an aliphatic hydrocarbon group optionally having substituent(s),
R⁰ is a hydrogen atom or an aliphatic hydrocarbon group, or R and R⁰ in combination form a bond,
ring A¹ is a cycloalkene optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR¹¹ wherein R¹¹ is a hydrogen atom or an aliphatic hydrocarbon group optionally having substituent(s), and (4) a halogen atom,
Ar is an aromatic hydrocarbon group optionally having substituent(s),
a group represented by the formula:

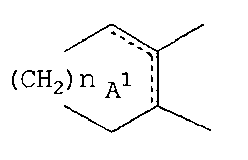

is a group represented by the formula:

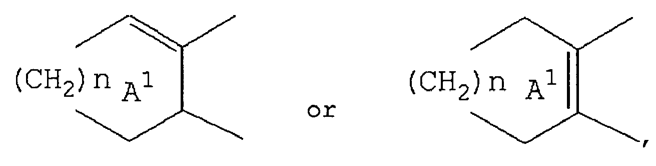

and n is an integer of 1 to 4,
or a salt thereof or a prodrug thereof, or a compound represented by the formula (II):

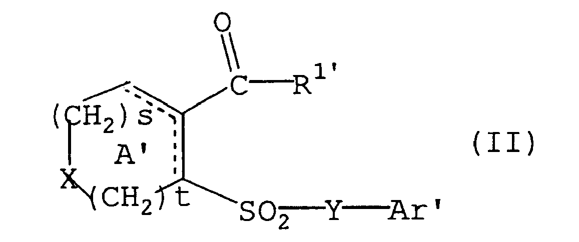

wherein
R^{1'} is an aliphatic hydrocarbon group optionally having substituent(s), an aromatic hydrocarbon group optionally having substituent(s), a heterocyclic group optionally having substituent(s), a group represented by the formula: -OR^{1a'} wherein R^{1a'} is a hydrogen atom or an aliphatic hydrocarbon group optionally having substituent(s), or a group represented by the formula:

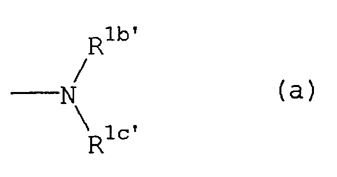

wherein R^{1b'} and R^{1c'} are the same or different and each is a hydrogen atom or an aliphatic hydrocarbon group optionally having substituent(s),
X is a methylene group, NH, a sulfur atom or an oxygen atom,
Y is a methylene group optionally having substituent(s) or NH optionally having substituent(s), ring A' is a 5- to 8-membered ring optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR^{2'} wherein R^{2'} is a hydrogen atom or an aliphatic hydrocarbon
group optionally having substituent(s), and (4) a halogen atom, Ar' is an aromatic hydrocarbon group optionally having substituent(s),
a group represented by the formula:

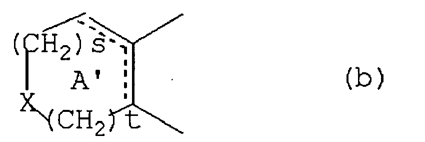

is a group represented by the formula:

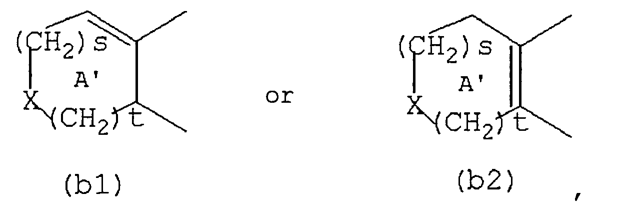

s is an integer of 0 to 2,
t is an integer of 1 to 3, and
the total of s and t is 4 or less;
provided that when X is a methylene group, then Y should be a methylene group optionally having substituent(s), or a salt thereof or a prodrug thereof, to a patient who undergoes coronary-artery bypass surgery,
[9] use of a compound represented by the formula (I):

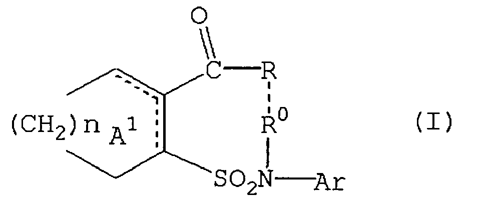

wherein
R is an aliphatic hydrocarbon group optionally having substituent(s), an aromatic hydrocarbon group optionally having substituent(s), a heterocyclic group optionally having substituent(s), a group represented by the formula: -OR¹ wherein R¹ is a hydrogen atom or an aliphatic hydrocarbon group optionally having substituent(s), or a group represented by the formula:

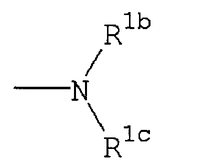

wherein R^{1b} and R^{1c} are the same or different and each is a hydrogen atom or an aliphatic hydrocarbon group optionally having substituent(s),
R⁰ is a hydrogen atom or an aliphatic hydrocarbon group, or R and R⁰ in combination form a bond,
ring A¹ is a cycloalkene optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR¹¹ wherein R¹¹ is a hydrogen atom or an aliphatic hydrocarbon group optionally having substituent(s), and (4) a halogen atom,
Ar is an aromatic hydrocarbon group optionally having substituent(s),
a group represented by the formula:

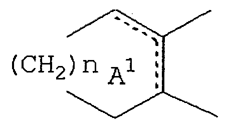

is a group represented by the formula:

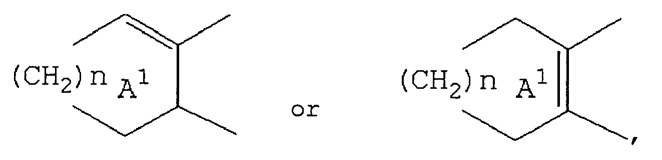

and n is an integer of 1 to 4,
or a salt thereof or a prodrug thereof, or a compound represented by the formula (II):

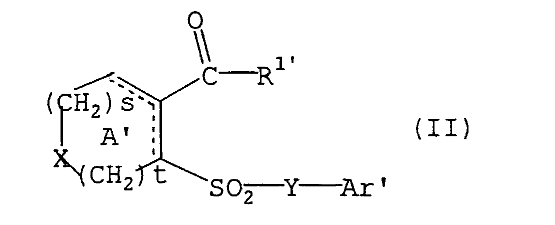

wherein
R^{1'} is an aliphatic hydrocarbon group optionally having substituent(s), an aromatic hydrocarbon group optionally having substituent(s), a heterocyclic group optionally having substituent(s), a group represented by the formula: -OR^{1a'} wherein R^{1a}' is a hydrogen atom or an aliphatic hydrocarbon group optionally having substituent(s), or a group represented by the formula:

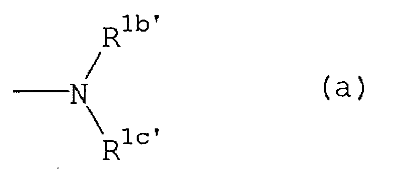

wherein R^{1b'} and R^{1c'} are the same or different and each is a hydrogen atom or an aliphatic hydrocarbon group optionally having substituent(s),
X is a methylene group, NH, a sulfur atom or an oxygen atom,
Y is a methylene group optionally having substituent(s) or NH optionally having substituent(s), ring A' is a 5- to 8-membered ring optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR^{2'} wherein R^{2'} is a hydrogen atom or an aliphatic hydrocarbon
group optionally having substituent(s), and (4) a halogen atom, Ar' is an aromatic hydrocarbon group optionally having substituent(s),
a group represented by the formula:

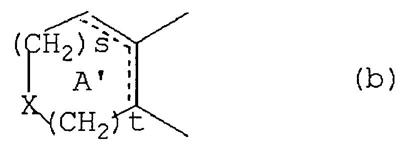

is a group represented by the formula:

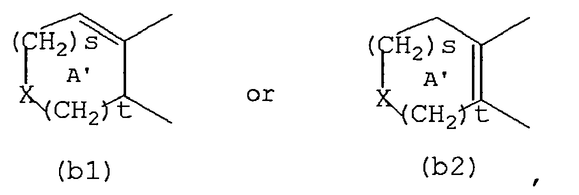

s is an integer of 0 to 2,
t is an integer of 1 to 3, and
the total of s and t is 4 or less;
provided that when X is a methylene group, then Y should be a methylene group optionally having substituent(s),
or a salt thereof or a prodrug thereof,
for the production of an agent for the prophylaxis or treatment of cardiac disease, autoimmune diseases, central nervous system diseases, inflammatory disease, sepsis, severe sepsis or septic shock in a patient who undergoes coronary-artery bypass surgery,
[10] a compound represented by the formula (I):

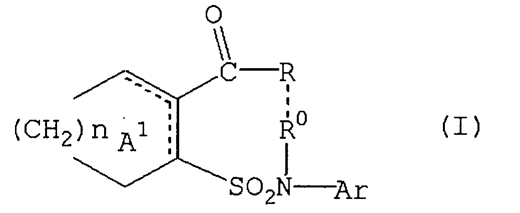

wherein
R is an aliphatic hydrocarbon group optionally having substituent(s), an aromatic hydrocarbon group optionally having substituent(s), a heterocyclic group optionally having substituent(s), a group represented by the formula: -OR¹ wherein R¹ is a hydrogen atom or an aliphatic hydrocarbon group optionally having substituent(s), or a group represented by the formula:

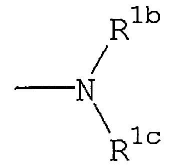

wherein R^{1b} and R^{1c} are the same or different and each is a hydrogen atom or an aliphatic hydrocarbon group optionally having substituent(s),
R⁰ is a hydrogen atom or an aliphatic hydrocarbon group, or R and R⁰ in combination form a bond,
ring A¹ is a cycloalkene optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR¹¹ wherein R¹¹ is a hydrogen atom or an aliphatic hydrocarbon group optionally having substituent(s), and (4) a halogen atom,
Ar is an aromatic hydrocarbon group optionally having substituent(s),
a group represented by the formula:

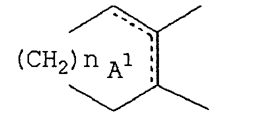

is a group represented by the formula:

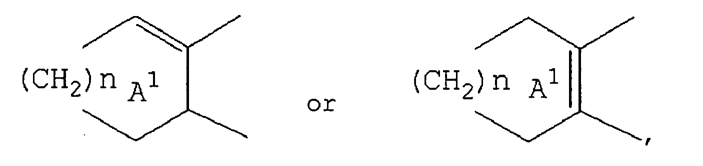

and n is an integer of 1 to 4, or a salt thereof or a prodrug thereof, or
a compound represented by the formula (II):

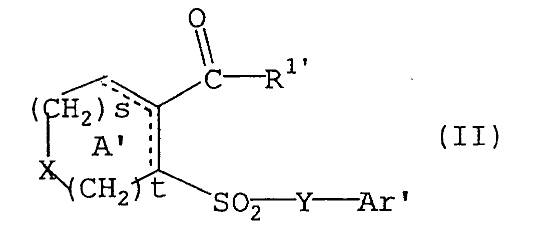

wherein
R^{1'} is an aliphatic hydrocarbon group optionally having substituent(s), an aromatic hydrocarbon group optionally having substituent(s), a heterocyclic group optionally having substituent(s), a group represented by the formula: -OR^{1a'} wherein R^{1a'} is a hydrogen atom or an aliphatic hydrocarbon group optionally having substituent(s), or a group represented by the formula:

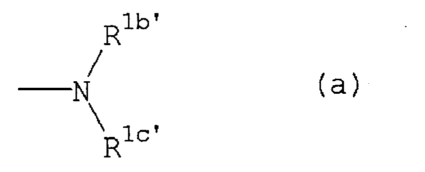

wherein R^{1b'} and R^{1c'} are the same or different and each is a hydrogen atom is an aliphatic hydrocarbon group optionally having substituent(s),
X is a methylene group, NH, a sulfur atom or an oxygen atom,
Y is a methylene group optionally having substituent(s) or NH optionally having substituent(s),
ring A' is a 5- to 8-membered ring optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR^{2'} wherein R^{2'} is a hydrogen atom or an aliphatic hydrocarbon group optionally having substituent(s), and (4) a halogen atom, Ar' is an aromatic hydrocarbon group optionally having substituent(s),
a group represented by the formula:

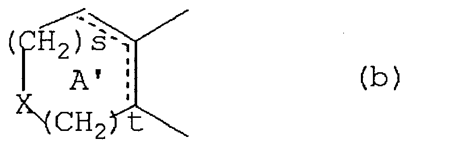

is a group represented by the formula:

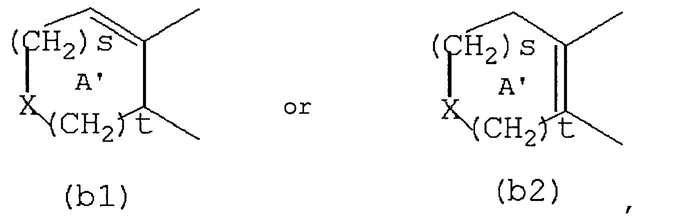

s is an integer of 0 to 2,
t is an integer of 1 to 3, and
the total of s and t is 4 or less;
provided that when X is a methylene group, then Y should be a methylene group optionally having substituent(s),
or a salt thereof or a prodrug thereof, for the use of as an agent for the prophylaxis or treatment of cardiac disease, autoimmune diseases, central nervous system diseases, inflammatory disease, sepsis, severe sepsis or septic shock in a patient who undergoes coronary-artery bypass surgery,

[11] an agent for the prophylaxis or treatment of cardiac disease, autoimmune disease, central nervous system disease, inflammatory disease, sepsis, severe sepsis, septic shock or complications after coronary-artery bypass surgery, comprising ethyl (6R)-6-[(2-chloro-4-fluoroanilino)sulfonyl]-1-cyclohexene-1-carboxylate or a salt thereof or a prodrug thereof, which is prepared for administration of ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight for about 15 min to about 240 min,
[12] a method for the prophylaxis or treatment of cardiac disease, autoimmune diseases, central nervous system diseases, inflammatory disease, sepsis, severe sepsis, septic shock or complications after coronary-artery bypass surgery, which comprises administering ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight for about 15 min to about 240 min, to a mammal,
[13] use of ethyl (6R)-6-[(2-chloro-4-fluoroanilino)sulfonyl]-1-cyclohexene-1-carboxylate or a salt thereof or a prodrug thereof, for the production of an agent for the prophylaxis or treatment of cardiac disease, autoimmune diseases, central nervous system diseases, inflammatory disease, sepsis, severe sepsis, septic shock or complications after coronary-artery bypass surgery, wherein the agent is prepared for administration of ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight for about 15 min to about 240 min,
[14] ethyl (6R)-6-[(2-chloro-4-fluoroanilino)sulfonyl]-1-cyclohexene-1-carboxylate or a salt thereof or a prodrug thereof for the use as an agent for the prophylaxis or treatment of cardiac disease, autoimmune diseases, central nervous system diseases, inflammatory disease, sepsis, severe sepsis, septic shock or complications after coronary-artery bypass surgery, wherein the agent is prepared for administration of ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight for about 15 min to about 240 min,
[15] an agent for the prophylaxis or treatment of cardiac disease, autoimmune diseases, central nervous system diseases, inflammatory disease, sepsis, severe sepsis, septic shock or complications after coronary-artery bypass surgery, comprising ethyl (6R)-6-[(2-chloro-4-fluoroanilino)sulfonyl]-1-cyclohexene-1-carboxylate or a salt thereof or a prodrug thereof, which is prepared for administration of ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight per administration for about 15 min to about 240 min per administration, 1 to 6 times a day, for 1 day to 1 month,
[16] a method for the prophylaxis or treatment of cardiac disease, autoimmune diseases, central nervous system diseases, inflammatory disease, sepsis, severe sepsis, septic shock or complications after coronary-artery bypass surgery, which comprises administering ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight per administration for about 15 min to about 240 min per administration, 1 to 6 times a day, for 1 day to 1 month, to a mammal,
[17] use of ethyl (6R)-6-[(2-chloro-4-fluoroanilino)sulfonyl]-1-cyclohexene-1-carboxylate or a salt thereof or a prodrug thereof, for the production of an agent for the prophylaxis or treatment of cardiac disease, autoimmune diseases, central nervous system diseases, inflammatory disease, sepsis, severe sepsis, septic shock or complications after coronary-artery bypass surgery, wherein the agent is prepared for administration of ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight per administration for about 15 min to about 240 min per administration, 1 to 6 times a day, for 1 day to 1 month,
[18] ethyl (6R)-6-[(2-chloro-4-fluoroanilino)sulfonyl]-1-cyclohexene-1-carboxylate or a salt thereof or a prodrug thereof, for the use as an agent for the prophylaxis or treatment of cardiac disease, autoimmune diseases, central nervous system diseases, inflammatory disease, sepsis, severe sepsis, septic shock or complications after coronary-artery bypass surgery, wherein the agent is prepared for administration of ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight per administration for about 15 min to about 240 min per administration, 1 to 6 times a day, for 1 day to 1 month,
[19] an agent for the prophylaxis or treatment of cardiac disease, autoimmune diseases, central nervous system diseases, inflammatory disease, sepsis, severe sepsis, septic shock or complications after coronary-artery bypass surgery, comprising ethyl (6R)-6-[(2-chloro-4-fluoroanilino)sulfonyl]-1-cyclohexene-1-carboxylate or a salt thereof or a prodrug thereof, which is prepared for continuous drip of ethyl (6R)-6-[(2-chloro-4-fluoroanilino)sulfonyl]-1-cyclohexene-1-carboxylate or a salt thereof or a prodrug thereof at a dose of about 0.01 mg/kg body weight/hr to about 0.3 mg/kg body weight/hr for about 1 hr to about 1 month,
[20] a method for the prophylaxis or treatment of cardiac disease, autoimmune diseases, central nervous system diseases, inflammatory disease, sepsis, severe sepsis, septic shock or complications after coronary-artery bypass surgery, which comprises giving continuous drip of ethyl (6R)-6-[(2-chloro-4-fluoroanilino)sulfonyl]-1-cyclohexene-1-carboxylate or a salt thereof or a prodrug thereof at a dose of about 0.01 mg/kg body weight/hr to about 0.3 mg/kg body weight/hr for about 1 hr to about 1 month, to a mammal,
[21] use of ethyl (6R)-6-[(2-chloro-4-fluoroanilino)sulfonyl]-1-cyclohexene-1-carboxylate or a salt thereof or a prodrug thereof, for the production of an agent for the prophylaxis or treatment of cardiac disease, autoimmune diseases, central nervous system diseases, inflammatory disease, sepsis, severe sepsis, septic shock or complications after coronary-artery bypass surgery, wherein the agent is prepared for continuous drip of ethyl (6R)-6-[(2-chloro-4-fluoroanilino)sulfonyl]-1-cyclohexene-1-carboxylate or a salt thereof or a prodrug thereof at a dose of about 0.01 mg/kg body weight/hr to about 0.3 mg/kg body weight/hr for about 1 hr to about 1 month,
[22] ethyl (6R)-6-[(2-chloro-4-fluoroanilino)sulfonyl]-1-cyclohexene-1-carboxylate or a salt thereof or a prodrug thereof, for the use as an agent for the prophylaxis or treatment of cardiac disease, autoimmune diseases, central nervous system diseases, inflammatory disease, sepsis, severe sepsis, septic shock or complications after coronary-artery bypass surgery, wherein the agent is prepared for continuous drip of ethyl (6R)-6-[(2-chloro-4-fluoroanilino)sulfonyl]-1-cyclohexene-1-carboxylate or a salt thereof or a prodrug thereof at a dose of about 0.01 mg/kg body weight/hr to about 0.3 mg/kg body weight/hr for about 1 hr to about 1 month,
[23] an agent for the prophylaxis or treatment of cardiac disease, autoimmune diseases, central nervous system diseases, inflammatory disease, sepsis, severe sepsis, septic shock or complications after coronary-artery bypass surgery, comprising ethyl (6R)-6-[(2-chloro-4-fluoroanilino)sulfonyl]-1-cyclohexene-1-carboxylate or a salt thereof or a prodrug thereof, which is prepared for administration of ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight for about 15 min to about 240 min, and then for continuous drip of ethyl (6R)-6-[(2-chloro-4-fluoroanilino)sulfonyl]-1-cyclohexene-1-carboxylate or a salt thereof or a prodrug thereof at a dose of about 0.01 mg/kg body weight/hr to about 0.3 mg/kg body weight/hr for about 1 hr to about 1 month,
[24] a method for the prophylaxis or treatment of cardiac disease, autoimmune diseases, central nervous system diseases, inflammatory disease, sepsis, severe sepsis, septic shock or complications after coronary-artery bypass surgery, which comprises administering ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight for about 15 min to about 240 min, and then giving continuous drip of ethyl (6R)-6-[(2-chloro-4-fluoroanilino)sulfonyl]-1-cyclohexene-1-carboxylate or a salt thereof or a prodrug thereof at a dose of about 0.01 mg/kg body weight/hr to about 0.3 mg/kg body weight/hr for about 1 hr to about 1 month, to a mammal,
[25] use of ethyl (6R)-6-[(2-chloro-4-fluoroanilino)sulfonyl]-1-cyclohexene-1-carboxylate or a salt thereof or a prodrug thereof, for the production of an agent for the prophylaxis or treatment of cardiac disease, autoimmune diseases, central nervous system diseases, inflammatory disease, sepsis, severe sepsis, septic shock or complications after coronary-artery bypass surgery, wherein the agent is prepared for administration of ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight for about 15 min to about 240 min, and then for continuous drip of ethyl (6R)-6-[(2-chloro-4-fluoroanilino)sulfonyl]-1-cyclohexene-1-carboxylate or a salt thereof or a prodrug thereof at a dose of about 0.01 mg/kg body weight/hr to about 0.3 mg/kg body weight/hr for about 1 hr to about 1 month,
[26] ethyl (6R)-6-[(2-chloro-4-fluoroanilino)sulfonyl]-1-cyclohexene-1-carboxylate or a salt thereof or a prodrug thereof, for the use as an agent for the prophylaxis or treatment of cardiac disease, autoimmune diseases, central nervous system diseases, inflammatory disease, sepsis, severe sepsis, septic shock or complications after coronary-artery bypass surgery, wherein the agent is prepared for administration of ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight for about 15 min to about 240 min, and then for continuous drip of ethyl (6R)-6-[(2-chloro-4-fluoroanilino)sulfonyl]-1-cyclohexene-1-carboxylate or a salt thereof or a prodrug thereof at a dose of about 0.01 mg/kg body weight/hr to about 0.3 mg/kg body weight/hr for about 1 hr to about 1 month,
[27] the agent of the above-mentioned [11], [15], [19] or [23], wherein ethyl (6R)-6-[(2-chloro-4-fluoroanilino)sulfonyl]-1-cyclohexene-1-carboxylate or a salt thereof or a prodrug thereof is used in the form of an emulsion composition having a pH adjusted to about 3.7 to about 5.5, which comprises the compound and a buffer,
[28] the method of the above-mentioned [12], [16], [20] or [24], wherein ethyl (6R)-6-[(2-chloro-4-fluoroanilino)sulfonyl]-1-cyclohexene-1-carboxylate or a salt thereof or a prodrug thereof is used in the form of an emulsion composition having a pH adjusted to about 3.7 to about 5.5, which comprises the compound and a buffer,
[29] the use of the above-mentioned [13], [17], [21] or [25], wherein ethyl (6R)-6-[(2-chloro-4-fluoroanilino)sulfonyl]-1-cyclohexene-1-carboxylate or a salt thereof or a prodrug thereof is used in the form of an emulsion composition having a pH adjusted to about 3.7 to about 5.5, which comprises the compound and a buffer, and
[30] the compound of the above-mentioned [14], [18], [22] or [26], wherein ethyl (6R)-6-[(2-chloro-4-fluoroanilino)sulfonyl]-1-cyclohexene-1-carboxylate or a salt thereof or a prodrug thereof is used in the form of an emulsion composition having a pH adjusted to about 3.7 to about 5.5, which comprises the compound and a buffer.
Furthermore, the present invention relates to,
[31] the agent of the above-mentioned [27], the method of the above-mentioned [28], the use of the above-mentioned [29] or the compound of the above-mentioned [30] or a salt thereof or a prodrug thereof, where the buffer is one or more buffer selected from the group consisting of acetate buffer, lactate buffer, citrate buffer and phosphate buffer,
[32] the agent, method, use, or compound or salt thereof or prodrug thereof of the above-mentioned [31], wherein the buffer is acetate buffer, and the acetate buffer contains acetic acid and sodium acetate,
[33] the agent of the above-mentioned [27], the method of the above-mentioned [28], the use of the above-mentioned [29] or the compound of the above-mentioned [30] or a salt thereof or a prodrug thereof, wherein the concentration of the buffer is not more than about 100 mM,
[34] the agent of the above-mentioned [27], the method of the above-mentioned [28], the use of the above-mentioned [29] or the compound of the above-mentioned [30] or a salt thereof or a prodrug thereof, wherein the emulsion composition further contains a component selected from the group consisting of oil component, emulsifier, water and a mixture thereof,
[35] the agent, method, use, or compound or salt thereof or prodrug thereof of the above-mentioned [34], wherein the emulsion composition is oil-in-water type,
[36] the agent of the above-mentioned [27], the method of the above-mentioned [28], the use of the above-mentioned [29] or the compound of the above-mentioned [30] or a salt thereof or a prodrug thereof, wherein the emulsion composition is comprised of dispersion phase particles comprising ethyl (6R)-6-[(2-chloro-4-fluoroanilino)sulfonyl]-1-cyclohexene-1-carboxylate or a salt thereof or a prodrug thereof, an oil component and an emulsifier, and water comprising the above-mentioned buffer, in which the dispersion phase particles are dispersed,
[37] the agent, method, use, or compound or salt thereof or prodrug thereof of the above-mentioned [36], wherein the dispersion phase particle has the mean particle size of about 0.025 to about 0.7 µm,
[38] the agent, method, use, or compound or salt thereof or prodrug thereof of the above-mentioned [36], wherein the dispersion phase particles and water wherein the dispersion phase particles are dispersed are stable without phase separation,
[39] the agent, method, use, or compound or salt thereof or prodrug thereof of the above-mentioned [35], wherein the emulsion composition does not contain a visibly observed free oil drop,
[40] the agent, method, use, or compound or salt thereof or prodrug thereof of the above-mentioned [34], wherein the oil component is vegetable oil,
[41] the agent, method, use, or compound or salt thereof or prodrug thereof of the above-mentioned [40], wherein the vegetable oil is soybean oil,
[42] the agent, method, use, or compound or salt thereof or prodrug thereof of the above-mentioned [34], wherein the emulsifier is phospholipid,
[43] the agent, method, use, or compound or salt thereof or prodrug thereof of the above-mentioned [42], wherein the phospholipid is egg-yolk lecithin and/or phosphatidylglycerol, [44] the agent, method, use, or compound or salt thereof or prodrug thereof of the above-mentioned [34], wherein the amount of the oil component to be used is about 1 to about 30 wt%, relative to the total of emulsion composition,
[45] the agent, method, use, or compound or salt thereof or prodrug thereof of the above-mentioned [34], wherein the amount of the emulsifier to be used is about 0.1 to about 10%(W/V), relative to the total of emulsion composition,
[46] the agent, method, use, or compound or salt thereof or prodrug thereof of the above-mentioned [43], wherein the phosphatidylglycerol is dimyristoylphosphatidylglycerol,
[47] the agent, method, use, or compound or salt thereof or prodrug thereof of the above-mentioned [34], which comprises soybean oil, phospholipid, glycerol and purified water,
[48] the agent of the above-mentioned [27], the method of the above-mentioned [28], the use of the above-mentioned [29] or the compound of the above-mentioned [30] or a salt thereof or a prodrug thereof, wherein the emulsion composition is for injection, and
[49] the agent of the above-mentioned [27], the method of the above-mentioned [28], the use of the above-mentioned [29] or the compound of the above-mentioned [30] or a salt thereof or a prodrug thereof, which comprises ethyl (6R)-6-[(2-chloro-4-fluoroanilino)sulfonyl]-1-cyclohexene-1-carboxylate or a salt thereof or a prodrug thereof of about 0.001 to about 95 wt%, relative to the total of emulsion composition.

The compound represented by the formula (I) and the formula (II) of the present invention, a salt thereof and a prodrug thereof, and a pharmaceutical composition containing the same are useful as agents for improving prognosis after coronary-artery bypass surgery, particularly as agents for the prophylaxis or treatment and the like of complications after coronary-artery bypass surgery.
In addition, administration of ethyl (6R)-6-[(2-chloro-4-fluoroanilino)sulfonyl]-1-cyclohexene-1-carboxylate (compound 72 of Reference Example B66), or a salt thereof, or a prodrug thereof in a specific dose at a specific administration time can efficiently prevent or treat cardiac diseases, autoimmune diseases, central nervous system diseases, inflammatory diseases, sepsis, severe sepsis, septic shock and complications after coronary-artery bypass surgery.

In the present invention, the above-mentioned compounds represented by the formula (I) and the formula (II), a salt thereof and a prodrug thereof can be used as an active ingredient.

The above-mentioned compounds are explained in detail in the following.
In the specification, R is an aliphatic hydrocarbon group optionally having substituent(s), an aromatic hydrocarbon group optionally having substituent(s), a heterocyclic group optionally having substituent(s), a group represented by the formula: -OR¹ wherein R¹ is a hydrogen atom or an aliphatic hydrocarbon group optionally having substituent(s), or a group represented by the formula:

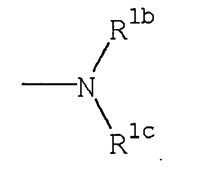

wherein R^{1b} and R^{1c} are the same or different and each is a hydrogen atom or an aliphatic hydrocarbon group optionally having substituent(s), or R and R⁰ in combination form a bond, with particular preference given to the group represented by the formula: -OR¹ wherein R¹ is as defined above.

When R and R⁰ in combination form a bond, the compound represented by the formula (I) can be represented by the formula:

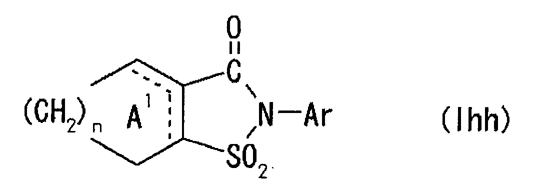

wherein each symbol is as defined above, and specifically can be represented by the formula:

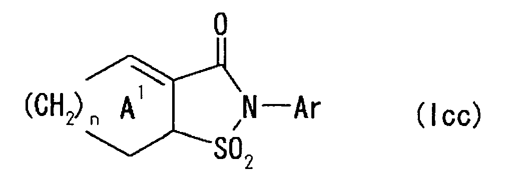

wherein each symbol is as defined above, or

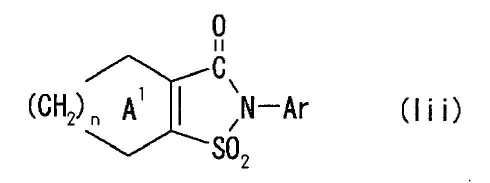

wherein each symbol is as defined above.

When R is a group represented by the formula: -OR¹ wherein R¹ is as defined above, the compound represented by the formula (I) can be represented by the formula:

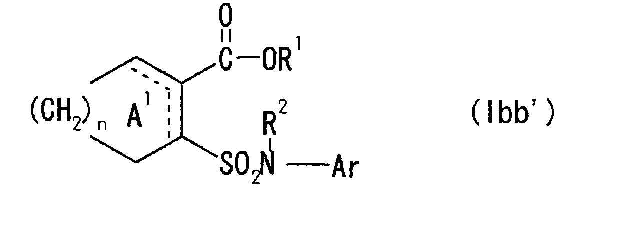

wherein R² is a hydrogen atom or an aliphatic hydrocarbon group, and other symbols are as defined above, and specifically can be represented by the formula:

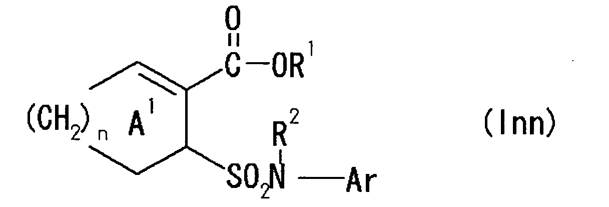

wherein each symbol is as defined above, or

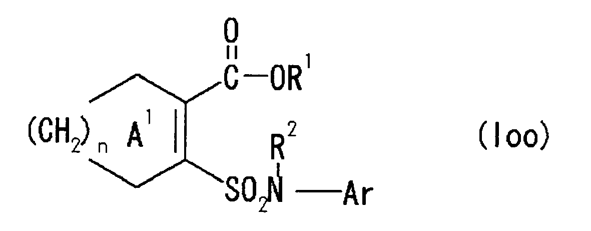

wherein each symbol is as defined above.
As the compound represented by the formula (I), a compound represented by the formula (Icc) or the formula (Inn) is preferable.
R⁰ is a hydrogen atom or an aliphatic hydrocarbon group, or as mentioned above, R⁰ and R in combination form a bond. When R⁰ is a hydrogen atom or an aliphatic hydrocarbon group, R⁰ is preferably a hydrogen atom or a C₁₋₆ alkyl group. Examples of the C₁₋₆ alkyl group for R⁰ include a methyl group, an ethyl group, an n-propyl group, an isopropyl group, an n-butyl group, an isobutyl group, a sec-butyl group, a tert-butyl group and the like, preferably, a methyl group and the like. Preferred as the R⁰ are a hydrogen atom, a methyl group and the like.

As the "aliphatic hydrocarbon group" of the "aliphatic hydrocarbon group optionally having substituent(s)" for R, R¹, R¹¹, R^{1b} or R^{1c} and the "aliphatic hydrocarbon group" for R⁰ or R², for example, an alkyl group, a cycloalkyl group, a cycloalkylalkyl group, an alkenyl group, an alkynyl group, etc. are preferable.
As the alkyl group, for example, a linear or branched alkyl group having 1 to 20 carbon atoms (e.g., a methyl group, an ethyl group, an n-propyl group, an isopropyl group, an n-butyl group, an isobutyl group, a sec-butyl group, a tert-butyl group, a pentyl group, a hexyl group, a heptyl group, an octyl group, a nonyl group, a decyl group, a dodecyl group, etc.) and the like are preferable, and particularly, for example, a lower alkyl group having 1 to 6 carbon atoms (e.g., a methyl group, an ethyl group, an n-propyl group, an isopropyl group, an n-butyl group, an isobutyl group, a sec-butyl group, a tert-butyl group, etc.) and the like are preferable.
As the cycloalkyl group, for example, a cycloalkyl group having 3 to 10 carbon atoms (e.g., a cyclopropyl group, a cyclobutyl group, a cyclopentyl group, a cyclohexyl group, a cycloheptyl group, a cyclooctyl group, etc.) and the like are preferable, and particularly, for example, a cycloalkyl group having 3 to 6 carbon atoms (e.g., a cyclopropyl group, a cyclobutyl group, a cyclopentyl group, a cyclohexyl group, etc.) and the like are preferable.
As the cycloalkylalkyl group, for example, a cycloalkylalkyl group having 4 to 12 carbon atoms (e.g., a cyclopropylmethyl group, a cyclopentylmethyl group, a cyclohexylmethyl group, a cycloheptylmethyl group, etc.) and the like are preferable, and particularly, for example, a cycloalkylalkyl group having 4 to 8 (particularly 4 to 7) carbon atoms (e.g., a cyclopropylmethyl group, a cyclopentylmethyl group, a cyclohexylmethyl group, etc.) and the like are preferable.
As the alkenyl group, for example, a lower alkenyl group having 3 to 6 carbon atoms (e.g., a propenyl group, a butenyl group, a pentenyl group, etc.) and the like are preferable, and particularly, for example, a lower alkenyl group having 3 or 4 carbon atoms (e.g., a propenyl group, a butenyl group, etc.) and the like are preferable.
As the alkynyl group, for example, a lower alkynyl group having 3 to 6 carbon atoms (e.g., a propynyl group, a butynyl group, a pentynyl group, etc.) and the like are preferable, and particularly, for example, a lower alkynyl group having 3 or 4 carbon atoms (e.g., a propynyl group, a butynyl group, etc.) and the like are preferable.

As the "substituent(s)" of the above-mentioned "aliphatic hydrocarbon group optionally having substituent(s)", for example, a heterocyclic group, an oxo group, a hydroxy group, a C₁₋₆ alkoxy group, a C₃₋₁₀ (particularly C₃₋₆) cycloalkyloxy group, a C₆₋₁₀ aryloxy group, a C₇₋₁₉ (particularly C₇₋₁₂) aralkyloxy group, a heterocyclyloxy group, a C₁₋₆ alkylthio group (the sulfur atom may be oxidized), a C₃₋₁₀ (particularly C₃₋₆) cycloalkylthio group (the sulfur atom may be oxidized), a C₆₋₁₀ arylthio group (the sulfur atom may be oxidized), a C₇₋₁₉ (particularly C₇₋₁₂) aralkylthio group (the sulfur atom may be oxidized), a heterocyclylthio group, a heterocyclylsulfinyl group, a heterocyclylsulfonyl group, a nitro group, a halogen atom, a cyano group, a carboxyl group, a C₁₋₁₀ (particularly C₁₋₆) alkoxy-carbonyl group, a C₃₋₆ cycloalkyloxy-carbonyl group, a C₆₋₁₀ aryloxy-carbonyl group, a C₇₋₁₉ (particularly C₇₋₁₂) aralkyloxy-carbonyl group, a heterocyclyloxy-carbonyl group, a C₆₋₁₀ aryl-carbonyl group, a C₁₋₆ alkanoyl group, a C₃₋₅ alkenoyl group, a C₆₋₁₀ aryl-carbonyloxy group, a C₂₋₆ alkanoyloxy group, a C₃₋₅ alkenoyloxy group, a carbamoyl group optionally having substituent(s), a thiocarbamoyl group optionally having substituent(s), a carbamoyloxy group optionally having substituent(s), a C₁₋₆ alkanoylamino group, a C₆₋₁₀ aryl-carbonylamino group, a C₁₋₁₀ (particularly C₁₋₆) alkoxy-carboxamido group, a C₆₋₁₀ aryloxy-carboxamido group, a C₇₋₁₉ (particularly C₇₋₁₂) aralkyloxy-carboxamido group, a C₁₋₁₀ (particularly C₁₋₆) alkoxy-carbonyloxy group, a C₆₋₁₀ aryloxy-carbonyloxy group, a C₇₋₁₉ (particularly C₇₋₁₂) aralkyloxy-carbonyloxy group, a C₃₋₁₀ (particularly C₃₋₆) cycloalkyloxy-carbonyloxy group, a ureido group optionally having substituent(s), a C₆₋₁₀ aryl group optionally having substituent(s), etc. can be used.
These substituents substitute at substitutable positions in the above-mentioned "aliphatic hydrocarbon group", wherein the substituents are not limited to a single substituent but may be the same or different plural (preferably 2 to 4) substituents.

As the "C₁₋₆ alkoxy group", for example, a methoxy group, an ethoxy group, an n-propoxy group, an isopropoxy group, an n-butoxy group, a tert-butoxy group, an n-pentyloxy group, an n-hexyloxy group, etc. can be used. As the "C₃₋₁₀ cycloalkyloxy group", for example, a cyclopropyloxy group, a cyclohexyloxy group, etc. can be used. As the "C₆₋₁₀ aryloxy group", for example, a phenoxy group, a naphthyloxy group, etc. can be used. As the "C₇₋₁₉ aralkyloxy group", for example, a benzyloxy group, a 1-phenylethyloxy group, a 2-phenylethyloxy group, a benzhydryloxy group, a 1-naphthylmethyloxy group, etc. can be used. As the "C₁₋₆ alkylthio group (the sulfur atom may be oxidized)", for example, a methylthio group, an ethylthio group, an n-propylthio group, an n-butylthio group, a methylsulfinyl group, a methylsulfonyl group, etc. can be used. As the "C₃₋₁₀ cycloalkylthio group (the sulfur atom may be oxidized)", for example, a cyclopropylthio group, a cyclohexylthio group, a cyclopentylsulfinyl group, a cyclohexylsulfonyl group, etc. can be used. As the "C₆₋₁₀ arylthio group (the sulfur atom may be oxidized)", for example, a phenylthio group, a naphthylthio group, a phenylsulfinyl group, a phenylsulfonyl group, etc. can be used. As the "C₇₋₁₉ aralkylthio group (the sulfur atom may be oxidized)", for example, a benzylthio group, a phenylethylthio group, a benzhydrylthio group, a benzylsulfinyl group, a benzylsulfonyl group, etc. can be used. As the "halogen atom", a fluorine atom, a chlorine atom, a bromine atom and an iodine atom can be used. As the "C₁₋₁₀ alkoxy-carbonyl group", for example, a methoxycarbonyl group, an ethoxycarbonyl group, an n-propoxycarbonyl group, an isopropoxycarbonyl group, an n-butoxycarbonyl group, an isobutoxycarbonyl group, a tert-butoxycarbonyl group, etc. can be used. As the "C₃₋₆ cycloalkyloxy-carbonyl group", for example, a cyclopropyloxycarbonyl group, a cyclopentyloxycarbonyl group, a cyclohexyloxycarbonyl group, etc. can be used. As the "C₆₋₁₀ aryloxy-carbonyl group", for example, a phenoxycarbonyl group, a naphthyloxycarbonyl group, etc. can be used. As the "C₇₋₁₉ aralkyloxy-carbonyl group", for example, a benzyloxycarbonyl group, a benzhydryloxycarbonyl group, a 2-phenethyloxycarbonyl group, etc. can be used. As the "C₆₋₁₀ aryl-carbonyl group", for example, a benzoyl group, a naphthoyl group, etc. can be used. As the "C₁₋₆ alkanoyl group", for example, a formyl group, an acetyl group, a propionyl group, a butyryl group, a valeryl group, a pivaloyl group, etc. can be used. As the "C₃₋₅ alkenoyl group", for example, an acryloyl group, a crotonoyl group, etc. can be used. As the "'C₆₋₁₀ aryl-carbonyloxy group", for example, a benzoyloxy group, a naphthoyloxy group, etc. can be used. As the "C₂₋₆ alkanoyloxy group", for example, an acetoxy group, a propionyloxy group, a butyryloxy group, a valeryloxy group, a pivaloyloxy group, etc. can be used. As the "C₃₋₅ alkenoyloxy group", for example, an acryloyloxy group, a crotonoyloxy group, etc. can be used.

As the "carbamoyl group optionally having substituent(s)", for example, a carbamoyl group or a cyclic amino (e.g., pyrrolidinyl, piperidinyl, piperazinyl, morpholinyl, etc.)-carbonyl group, which may be substituted by 1 or 2 substituents selected from C₁₋₄ alkyl (e.g., methyl, ethyl, etc.), phenyl, C₁₋₇ acyl (e.g., acetyl, propionyl, benzoyl, etc.), C₁₋₄ alkoxy-phenyl (e.g., methoxyphenyl, etc.), etc., and the like can be used, and specifically, for example, a carbamoyl group, an N-methylcarbamoyl group, an N-ethylcarbamoyl group, an N,N-dimethylcarbamoyl group, an N,N-diethylcarbamoyl group, an N-phenylcarbamoyl group, an N-acetylcarbamoyl group, an N-benzoylcarbamoyl group, an N-(p-methoxyphenyl)carbamoyl group, a 1-pyrrolidinylcarbonyl group, a piperidinocarbonyl group, a 1-piperazinylcarbonyl group, a morpholinocarbonyl group, etc. can be used. As the "thiocarbamoyl group optionally having substituent(s)", for example, a thiocarbamoyl group which may be substituted by 1 or 2 substituents selected from C₁₋₄ alkyl (e.g., methyl, ethyl, etc.), phenyl, etc. can be used, and specifically, for example, a thiocarbamoyl group, an N-methylthiocarbamoyl group, an N-phenylthiocarbamoyl group, etc. can be used. As the "carbamoyloxy group optionally having substituent(s)", for example, a carbamoyloxy group which may be substituted by 1 or 2 substituents selected from C₁₋₄ alkyl (e.g., methyl, ethyl, etc.), phenyl, etc. can be used, and specifically, for example, a carbamoyloxy group, an N-methylcarbamoyloxy group, an N,N-dimethylcarbamoyloxy group, an N-ethylcarbamoyloxy group, an N-phenylcarbamoyloxy group, etc. can be used.

As the "C₁₋₆ alkanoylamino group", for example, an acetamido group, a propionamido group, a butyramido group, a valeramido group, a pivalamido group, etc. can be used. As the "C₆₋₁₀ aryl-carbonylamino group", for example, a benzamido group, a naphthamido group, a phthalimido group, etc. can be used. As the "C₁₋₁₀ alkoxy-carboxamido group", for example, a methoxycarboxamido (CH₃OCONH- group, an ethoxycarboxamido group, a tert-butoxycarboxamido group, etc. can be used. As the "C₆₋₁₀ aryloxy-carboxamido group", for example, a phenoxycarboxamido (C₆H₅OCON group, etc. can be used. As the "C₇₋₁₉ aralkyloxy-carboxamido group", for example, a benzyloxycarboxamido (C₆H₅CH₂OCONH-) group, a benzhydryloxycarboxamido group, etc. can be used. As the "C₁₋₁₀ alkoxy-carbonyloxy group", for example, a methoxycarbonyloxy group, an ethoxycarbonyloxy group, an n-propoxycarbonyloxy group, an isopropoxycarbonyloxy group, an n-butoxycarbonyloxy group, a tert-butoxycarbonyloxy group, an n-pentyloxycarbonyloxy group, an n-hexyloxycarbonyloxy group, etc. can be used. As the ""C₆₋₁₀ aryloxy-carbonyloxy group", for example, a phenoxycarbonyloxy group, a naphthyloxycarbonyloxy group, etc. can be used. As the "C₇₋₁₉ aralkyloxy-carbonyloxy group", for example, a benzyloxycarbonyloxy group, a 1-phenylethyloxycarbonyloxy group, a 2-phenylethyloxycarbonyloxy group, a benzhydryloxycarbonyloxy group, etc. can be used. As the "C₃₋₁₀ cycloalkyloxy-carbonyloxy group", for example, a cyclopropyloxycarbonyloxy group, a cyclohexyloxycarbonyloxy group, etc. can be used.

As the "ureido group optionally having substituent(s)", for example, a ureido group optionally substituted by 1 to 3 (preferably 1 or 2) substituents selected from a C₁₋₄ alkyl group (e.g., a methyl group, an ethyl group, etc.), a phenyl group, etc. can be used, and, for example, a ureido group, a 1-methylureido group, a 3-methylureido group, a 3,3-dimethylureido group, a 1,3-dimethylureido group, a 3-phenylureido group, etc. can be used.

When a heterocyclic group, a heterocyclyloxy group, a heterocyclylthio group, a heterocyclylsulfinyl group, a heterocyclylsulfonyl group or a heterocyclyloxy-carbonyl group is used as the "substituents" of the "aliphatic hydrocarbon group optionally having substituent(s)", the heterocyclic group is a group formed by excluding one hydrogen atom that binds to the heterocycle. It is, for example, a 5- to 8-membered ring (preferably a 5- or 6-membered ring) group containing 1 to several, preferably 1 to 4 hetero atoms such as a nitrogen atom (optionally oxidized), an oxygen atom, a sulfur atom, etc., or its condensed cyclic group. As these heterocyclic groups, for example, a pyrrolyl group, a pyrazolyl group, an imidazolyl group, a 1,2,3-triazolyl group, a 1,2,4-triazolyl group, a tetrazolyl group, a furyl group, a thienyl group, an oxazolyl group, an isoxazolyl group, a 1,2,3-oxadiazolyl group, a 1,2,4-oxadiazolyl group, a 1,2,5-oxadiazolyl group, a 1,3,4-oxadiazolyl group, a thiazolyl group, an isothiazolyl group, a 1,2,3-thiadiazolyl group, a 1,2,4-thiadiazolyl group, a 1,2,5-thiadiazolyl group, a 1,3,4-thiadiazolyl group, a pyridyl group, a pyridazinyl group, a pyrimidinyl group, a pyrazinyl group, an indolyl group, a pyranyl group, a thiopyranyl group, a dioxinyl group, a dioxolyl group, a quinolyl group, a pyrido[2,3-d]pyrimidyl group, a 1,5-, 1,6-, 1,7-, 1,8-, 2,6- or 2,7-naphthyridyl group, a thieno[2,3-d]pyridyl group, a benzopyranyl group, a tetrahydrofuryl group, a tetrahydropyranyl group, a dioxolanyl group, a dioxanyl group, etc. can be used.
These heterocyclic groups may be substituted at substitutable positions by 1 to 3 substituents selected from a C₁₋₄ alkyl (e.g., methyl, ethyl, etc.), a hydroxy, an oxo, a C₁₋₄ alkoxy (e.g., methoxy, ethoxy, etc.), and the like.

As the "C₆₋₁₀ aryl group" of the "C₆₋₁₀ aryl group optionally having substituent(s)", for example, a phenyl group, a naphthyl group, etc. can be used. The C₆₋₁₀ aryl group may be substituted at a substitutable position by a substituent selected from those exemplified as the "substituent" (except for a C₆₋₁₀ aryl group optionally having substituent(s)) of the "aliphatic hydrocarbon group optionally having substituent(s)" described above. Such substituent is not limited to a single substituent, but the same or different, more than one (preferably 2 to 4) substituents may be used.
In the "aliphatic hydrocarbon group optionally having substituent(s)", the substituent may form, together with the aliphatic hydrocarbon group, an optionally substituted fused ring group, and as such fused ring group, an indanyl group, a 1,2,3,4-tetrahydronaphthyl group, etc. can be used. This fused ring group may be substituted at a substitutable position by a substituent selected from those exemplified as the "substituent" of the "aliphatic hydrocarbon group optionally having substituent(s)" described above. Such substituent substitutes at a substitutable position of the fused ring group, wherein the substituent is not limited to a single substituent, but the same or different, more than one (preferably 2 to 4) substituents may be used.

Preferable examples of the above-mentioned "aliphatic hydrocarbon group optionally having substituent(s)" for R, R¹, R¹¹, R^{1b} or R^{1c} include a lower alkyl group having 1 to 6 carbon atoms optionally having substituent(s) (e.g., a methyl group, an ethyl group, an n-propyl group, an isopropyl group, an n-butyl group, an isobutyl group, a tert-butoxycarbonylmethyl group, a hydroxyethyl group etc.), etc. Of these, a methyl group, an ethyl group, an n-propyl group, an isopropyl group, an n-butyl group, an isobutyl group, etc. are preferable. For example, a methyl group, an ethyl group, an n-propyl group and the like are more preferable, and particularly, an ethyl group, etc. are preferable.

As the "aromatic hydrocarbon group" of the "aromatic hydrocarbon group optionally having substituent(s)" for R, an aromatic hydrocarbon group having 6 to 14 carbon atoms (e.g., a phenyl group, a naphthyl group, an anthryl group, an indenyl group etc.) and the like are preferable, and particularly for example, an aryl group having 6 to 10 carbon atoms (e.g., phenyl, naphthyl groups etc.) and the like are preferable and, of these, a phenyl group and the like are particularly preferable.
As the "substituent" of the "aromatic hydrocarbon group optionally having substituent(s)" for R, for example, a halogen atom (a fluorine atom, a chlorine atom, a bromine atom, an iodine atom), a lower (C₁₋₄) alkyl group (e.g., a methyl group, an ethyl group, a propyl group, a butyl group etc.), a lower (C₁₋₄) alkoxy group (e.g., a methoxy group, an ethoxy group, a propoxy group, a butoxy group etc.), a lower (C₁₋₄) alkoxy-carbonyl group (e.g., a methoxycarbonyl group, an ethoxycarbonyl group, a propoxycarbonyl group, a butoxycarbonyl group etc.), a carboxyl group, a nitro group, a cyano group, a hydroxyl group, an acylamino group (e.g., an alkanoylamino group having 1 to 4 carbon atoms such as an acetylamino group, a propionylamino group, a butyrylamino group and the like, etc.), a cycloalkyl group having 3 to 6 carbon atoms (e.g., a cyclopropyl group, a cyclopentyl group etc.), an aryl group having 6 to 10 carbon atoms (e.g., a phenyl group, a naphthyl group, an indenyl group etc.), a halogeno-lower (C₁₋₄) alkyl group (e.g., a trifluoromethyl group, a trifluoroethyl group etc.), a halogeno-lower (C₁₋₄) alkoxy group (e.g., a trifluoromethoxy group, a 1,1,2,2-tetrafluoroethoxy group, a 2,2,3,3,3-pentafluoropropoxy group etc.), a lower (C₁₋₄) alkylthio group (e.g., a methylthio group, an ethylthio group, a propylthio group etc.), a lower (C₁₋₄) alkanesulfonyl group (e.g., a methanesulfonyl group, an ethanesulfonyl group, a propanesulfonyl group etc.), a lower (C₁₋₄) alkanoyl group (e.g., a formyl group, an acetyl group, a propionyl group etc.), a 5-membered aromatic heterocyclic group (e.g., a 1,2,3-triazolyl group, a 1,2,4-triazolyl group, a tetrazolyl group, a thiazolyl group, an isothiazolyl group, an oxazolyl group, an isoxazolyl group, a thiadiazolyl group,
a thienyl group, a furyl group etc.), a carbamoyl group, a lower (C₁₋₄) alkyl-carbamoyl group (e.g., a methylcarbamoyl group, a dimethylcarbamoyl group, a propylcarbamoyl group etc.), a lower (C₁₋₄) alkoxy-carbonyl-lower (C₁₋₄) alkyl-carbamoyl group (e.g., a butoxycarbonylmethylcarbamoyl group, an ethoxycarbonylmethylcarbamoyl group etc.), a 1,3-diacylguanidino-lower (C₁₋₄) alkyl group (e.g., 1,3-diacetylguanidinomethyl, 2,3-bis-(tert-butoxycarbonyl)guanidinomethyl etc.) and the like can be used, and a halogen atom (a fluorine atom, a chlorine atom, a bromine atom, an iodine atom), a lower (C₁₋₄) alkyl group (e.g., a methyl group, an ethyl group, a propyl group, a butyl group etc.) and the like are preferably used, and a fluorine atom, a chlorine atom and a methyl group are more preferably used.
These substituents substitute at substitutable positions of the aromatic hydrocarbon group, and the number of the substituents is preferably 1 to 5, more preferably 1 to 3, most preferably 1 or 2. When two or more of such substituents are present, they may be the same or different.

The "heterocyclic group" of the "heterocyclic group optionally having substituent(s)" for R is, for example, a 5 to 8-membered ring (particularly a 5 or 6-membered ring) group containing 1 to several, preferably 1 to 4, hetero atoms such as nitrogen atom (optionally oxidized), oxygen atom, sulfur atom and the like, and a fused ring group thereof. As such heterocyclic group, for example, a pyrrolyl group, a pyrazolyl group, an imidazolyl group, a 1,2,3-triazolyl group, a 1,2,4-triazolyl group, a tetrazolyl group, a furyl group, a thienyl group, an oxazolyl group, an isoxazolyl group, a 1,2,3-oxadiazolyl group, a 1,2,4-oxadiazolyl group, a 1,2,5-oxadiazolyl group, a 1,3,4-oxadiazolyl group, a thiazolyl group, an isothiazolyl group, a 1,2,3-thiadiazolyl group, a 1,2,4-thiadiazolyl group, a 1,2,5-thiadiazolyl group, a 1,3,4-thiadiazolyl group, a pyridyl group, a pyridazinyl group, pyrimidinyl group, a pyrazinyl group, an indolyl group, a pyranyl group, a thiopyranyl group, a dioxinyl group, a dioxolyl group, a quinolyl group, a pyrido[2,3-d]pyrimidyl group, a 1,5-, 1,6-, 1,7-, 1,8-, 2,6- or 2,7-naphthyridinyl group, a thieno[2,3-d]pyridyl group, a benzopyranyl group, a tetrahydrofuryl group, a tetrahydropyranyl group, a dioxolanyl group, a dioxanyl group and the like can be used.
These heterocyclic groups are optionally substituted by 1 to 3 substituents selected from C₁₋₄ alkyl (e.g., methyl, ethyl etc.), hydroxy, oxo, C₁₋₄ alkoxy (e.g., methoxy, ethoxy etc.) and the like at substitutable positions.

As the "aromatic hydrocarbon group" of the "aromatic hydrocarbon group optionally having substituent(s)" for Ar, an aromatic hydrocarbon group having 6 to 14 carbon atoms (e.g., a phenyl group, a naphthyl group, an anthryl group, an indenyl group etc.) and the like are preferable, and, for example, an aryl group having 6 to 10 carbon atoms (e.g., phenyl, naphthyl groups etc.) and the like are preferable and, of these, a phenyl group and the like are particularly preferable.
As the "substituent" of the "aromatic hydrocarbon group optionally having substituent(s)" for Ar, for example, a halogen atom (a fluorine, a chlorine, a bromine, an iodine atom), a lower (C₁₋₄) alkyl group (e.g., a methyl group, an ethyl group, a propyl group, an isopropyl group, a butyl group etc.), a lower (C₁₋₄) alkoxy group (e.g., a methoxy group, an ethoxy group, a propoxy group, a butoxy group etc.), a lower (C₁₋₄) alkoxy-carbonyl group (e.g., a methoxycarbonyl group, an ethoxycarbonyl group, a propoxycarbonyl group, a butoxycarbonyl group etc.), a carboxyl group, a nitro group, a cyano group, a hydroxyl group, an acylamino group (e.g., an alkanoylamino group having 1 to 4 carbon atoms such as an acetylamino group, a propionylamino group, a butyrylamino group and the like, etc.), a cycloalkyl group having 3 to 6 carbon atoms (e.g., a cyclopropyl group, a cyclopentyl group etc.), an aryl group having 6 to 10 carbon atoms (e.g., a phenyl group, a naphthyl group, an indenyl group etc.), a halogeno-lower (C₁₋₄) alkyl group (e.g., a trifluoromethyl group, a trifluoroethyl group etc.), a halogeno-lower (C₁₋₄) alkoxy group (e.g., a trifluoromethoxy group, a 1,1,2,2-tetrafluoroethoxy group, a 2,2,3,3,3-pentafluoropropoxy group etc.), a lower (C₁₋₄) alkylthio group (e.g., a methylthio group, an ethylthio group, a propylthio group etc.), a lower (C₁₋₄) alkanesulfonyl group (e.g., a methanesulfonyl group, an ethanesulfonyl group, a propanesulfonyl group etc.), a lower (C₁₋₄) alkanoyl group (e.g., a formyl group, an acetyl group, a propionyl group etc.), a 5-membered aromatic heterocyclic group (e.g., a 1,2,3-triazolyl group, a 1,2,4-triazolyl group, a tetrazolyl group, a thiazolyl group, an isothiazolyl group, an oxazolyl group, an isoxazolyl group, a thiadiazolyl group, a thienyl group, a furyl group etc.), a carbamoyl group, a lower (C₁₋₄) alkyl-carbamoyl group (e.g., a methylcarbamoyl group, a dimethylcarbamoyl group, a propionylcarbamoyl group etc.), a lower (C₁₋₄) alkoxy-carbonyl-lower (C₁₋₄) alkyl-carbamoyl group (e.g., a butoxycarbonylmethylcarbamoyl group, a tert-butoxycarbonylmethylcarbamoyl group, an ethoxycarbonylmethylcarbamoyl group etc.), a 1,3-diacylguanidino-lower (C₁₋₄) alkyl group (e.g., 1,3-diacetylguanidinomethyl, 1,3-bis-(tert-butoxycarbonyl)guanidinomethyl etc.) and the like can be used, and a halogen atom (a fluorine atom, a chlorine atom, a bromine atom, an iodine atom), a lower (C₁₋₄) alkyl group (e.g., a methyl group, an ethyl group, a propyl group, a butyl group etc.) and the like are preferably used, and a fluorine atom, a chlorine atom and a methyl group are more preferably used.
These substituents substitute at substitutable positions of the aromatic hydrocarbon group, and the number of the substituents is preferably 1 to 5, more preferably 1 to 3, most preferably 1 or 2. When two or more of such substituents are present, they may be the same or different.

Typically, as Ar, for example, a phenyl group optionally having substituent(s) (e.g., a phenyl group, a halogenophenyl group, a lower (C₁₋₄) alkylphenyl group, a lower (C₁₋₄) alkoxyphenyl group, a lower (C₁₋₄) alkoxy-carbonylphenyl group, a carboxylphenyl group, a nitrophenyl group, a cyanophenyl group, a halogeno-lower (C₁₋₄) alkylphenyl group, a halogeno-lower (C₁₋₄) alkoxyphenyl group, a lower (C₁₋₄) alkanoyl phenyl group, a phenyl group substituted by a 5-membered aromatic heterocyclic group, a lower (C₁₋₄) alkoxy-carbonyl-lower (C₁₋₄) alkyl-carbamoylphenyl group, 1,3-diacylguanidino-lower (C₁₋₄) alkylphenyl group, a phenyl group substituted by a halogen atom and a lower (C₁₋₄) alkyl group, a phenyl group substituted by a halogen atom and a lower (C₁₋₄) alkoxycarbonyl group, a phenyl group substituted by a halogen atom and a cyano group, a phenyl group substituted by a halogen atom and a 5-membered aromatic heterocyclic group, a phenyl group substituted by a halogen atom and a lower (C₁₋₄) alkoxy-carbonyl-lower (C₁₋₄) alkyl-carbamoyl group etc.) and the like can be used.
As Ar, a phenyl group optionally having substituent(s) is preferable. Of these, a halogenophenyl group, a lower (C₁₋₄) alkylphenyl group, a phenyl group substituted by a halogen atom and a lower (C₁₋₄) alkoxycarbonyl group, a phenyl group substituted by a halogen atom and a lower (C₁₋₄) alkyl group and the like are preferably used. Examples of the halogen atom of the substituent for the above-mentioned phenyl group include a fluorine atom, a chlorine atom, a bromine atom and an iodine atom.

As Ar, a group represented by the formula:

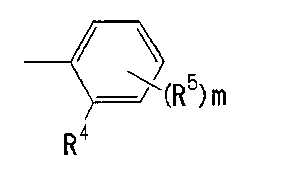

wherein R⁴ and R⁵ are the same or different and each is a halogen atom or a lower (C₁₋₄) alkyl group, and m is an integer of 0 to 2, is more preferable, and a group wherein at least one of R⁴ and R⁵ is a halogen atom is still more preferable.
As the halogen atom (e.g., a fluorine atom, a chlorine atom, a bromine atom, an iodine atom) for R⁴ or R⁵, a fluorine atom and a chlorine atom are preferable.

As the halogenophenyl group, for example, a 2,3-difluorophenyl group, a 2,3-dichlorophenyl group, a 2,4-difluorophenyl group, a 2,4-dichlorophenyl group, a 2,5-difluorophenyl group, a 2,5-dichlorophenyl group, a 2,6-difluorophenyl group, a 2,6-dichlorophenyl group, a 3,4-difluorophenyl group, a 3,4-dichlorophenyl group, a 3,5-difluorophenyl group, a 3,5-dichlorophenyl group, a 2-fluorophenyl group, a 2-chlorophenyl group, a 3-fluorophenyl group, a 3-chlorophenyl group, a 4-fluorophenyl group, a 4-chlorophenyl group, a 4-chloro-2-fluorophenyl group, a 2-chloro-4-fluorophenyl group, a 4-bromo-2-fluorophenyl group, a 2,3,4-trifluorophenyl group, a 2,4,5-trifluorophenyl group, a 2,4,6-trifluorophenyl, 2-bromo-4-fluorophenyl group, 4-bromo-2-chlorophenyl group and the like can be used.
As the lower (C₁₋₄) alkylphenyl group, for example, a 2-ethylphenyl group, a 2,6-diisopropylphenyl group and the like are preferably used. As the lower (C₁₋₄) alkoxyphenyl group, for example, a 4-methoxyphenyl and the like are preferably used.
As the lower (C₁₋₄) alkoxy-carbonylphenyl group, for example, a 2-ethoxycarbonylphenyl group, a 2-methoxycarbonylphenyl group, a 4-methoxycarbonylphenyl group and the like are preferably used. As the halogeno-lower (C₁₋₄) alkylphenyl group, for example, a 2-trifluoromethylphenyl group and the like are preferably used. As the halogeno-lower (C₁₋₄) alkoxyphenyl group, for example, a 2-trifluoromethoxyphenyl group, a 4-(2,2,3,3,3-pentafluoropropoxy)phenyl group and the like are preferably used.
As the lower (C₁₋₄) alkanoylphenyl group, for example, a 2-acetylphenyl group and the like are preferably used. As the phenyl group substituted by a 5-membered aromatic heterocyclic group, for example, a 4-(2H-1,2,3-triazol-2-yl)phenyl group, a 4-(2H-tetrazol-2-yl)phenyl group, a 4-(1H-tetrazol-1-yl)phenyl group, a 4-(1H-1,2,3-triazol-1-yl)phenyl group and the like are preferably used. As the lower (C₁₋₄) alkoxy-carbonyl-lower (C₁₋₄) alkyl-carbamoylphenyl group, for example, a 4-(N-ethoxycarbonylmethylcarbamoyl)phenyl group and the like are preferably used. As the 1,3-diacylguanidino-lower (C₁₋₄) alkylphenyl group, for example, a 4-(1,3-bis-tert-butoxycarbonylguanidinomethyl)phenyl group and the like are preferably used.
As the phenyl group substituted by a halogen atom and a lower (C₁₋₄) alkyl group, for example, a 2-fluoro-4-methylphenyl group, a 2-chloro-4-methylphenyl group, a 4-fluoro-2-methylphenyl group and the like are preferably used. As the phenyl group substituted by a halogen atom and a lower (C₁₋₄) alkoxy-carbonyl group, for example, a 2-chloro-4-methoxycarbonylphenyl group and the like are preferably used. As the phenyl group substituted by a halogen atom and a cyano group, a 2-chloro-4-cyanophenyl group and the like are preferably used. As the phenyl group substituted by a halogen atom and a 5-membered aromatic heterocyclic group, for example, a 2-fluoro-4-(1H-1,2,4-triazol-1-yl)phenyl group and the like are preferably used. As the phenyl group substituted by a halogen atom and a lower (C₁₋₄) alkoxy-carbonyl-lower (C₁₋₄) alkyl-carbamoyl group, for example, a 2-chloro-4-(N-tert-butoxycarbonylmethylcarbamoyl)phenyl group, a 2-chloro-4-(N-ethoxycarbonylmethylcarbamoyl)phenyl group and the like are preferably used.

More specifically, as Ar, a phenyl group, a phenyl group substituted by 1 to 3 (particularly 1 or 2) halogen atoms (e.g., a fluorine atom, a chlorine atom, a bromine atom, an iodine atom) (e.g., a 2,3-difluorophenyl group, a 2,3-dichlorophenyl group, a 2,4-difluorophenyl group, a 2,4-dichlorophenyl group, a 2,5-difluorophenyl group, a 2,5-dichlorophenyl group, a 2,6-difluorophenyl group, a 2,6-dichlorophenyl group, a 3,4-difluorophenyl group, a 3,4-dichlorophenyl group, a 3,5-difluorophenyl group, a 3,5-dichlorophenyl group, a 4-bromo-2-fluorophenyl group, a 2-fluorophenyl group, a 2-chlorophenyl group, a 3-fluorophenyl group, a 3-chlorophenyl group, a 4-fluorophenyl group, a 4-chlorophenyl group, a 2-fluoro-4-chlorophenyl group, a 2-chloro-4-fluorophenyl group, a 2,3,4-trifluorophenyl group, a 2,4,5-trifluorophenyl group, 2-bromo-4-fluorophenyl group, 4-bromo-2-chlorophenyl group etc.), a phenyl group substituted by a halogen atom (e.g., a fluorine atom, a chlorine atom, a bromine atom, an iodine atom) and a lower (C₁₋₄) alkyl group (e.g., a 2-chloro-4-methylphenyl group, a 4-fluoro-2-methylphenyl group, 2-fluoro-4-methylphenyl group etc.), etc. are particularly preferable. Of these, a phenyl group substituted by 1 to 3 (particularly 1 or 2) halogen atoms (e.g., a fluorine atom, a chlorine atom, a bromine atom, an iodine atom) (e.g., a 2,3-dichlorophenyl group, a 2,4-difluorophenyl group, a 2,4-dichlorophenyl group, a 2,6-dichlorophenyl group, a 2-fluorophenyl group, a 2-chlorophenyl group, a 3-chlorophenyl group, a 2-chloro-4-fluorophenyl group, a 2,4,5-trifluorophenyl group etc.), a phenyl group substituted by a halogen atom and a lower (C₁₋₄) alkyl group (e.g., a 2-chloro-4-methylphenyl group, a 4-fluoro-2-methylphenyl group etc.), etc. are preferable. Particularly, a 2,4-difluorophenyl group, a 2-chlorophenyl group, a 2-chloro-4-fluorophenyl group, a 2-chloro-4-methylphenyl group and the like are preferable, and a 2,4-difluorophenyl group, a 2-chloro-4-fluorophenyl group and the like are preferable.

In the present specification, ring A¹ is a cycloalkene optionally having 1 to 4 substituents selected from the group consisting of (i) an aliphatic hydrocarbon group optionally having substituent(s), (ii) an aromatic hydrocarbon group optionally having substituent(s), (iii) a group represented by the formula: -OR¹¹ wherein R¹¹ is a hydrogen atom or an aliphatic hydrocarbon group optionally having substituent(s) and (iv) a halogen atom, preferably a cycloalkene optionally having 1 to 4 substituents selected from the group consisting of (i) an aliphatic hydrocarbon group optionally having substituent(s), (ii) an aromatic hydrocarbon group optionally having substituent(s) and (iv) a halogen atom.
These substituents (i) to (iv) substitute on substitutable carbon atoms in the ring A¹, and when the ring A¹ is substituted by two or more of such substituents, the substituents may be the same or different. A single carbon atom may be substituted by two substituents, and different carbon atoms may be substituted by two or more substituents.
As the "aliphatic hydrocarbon group optionally having substituent(s)" as the substituent on the ring A¹, for example, those similar to the "aliphatic hydrocarbon group optionally having substituent(s)" for R and the like described above may be used.
As the "aromatic hydrocarbon group optionally having substituent(s)" as the substituent on the ring A¹, for example, those similar to the "aromatic hydrocarbon group optionally having substituent(s)" for Ar and the like described above may be used.
As the substituents for the ring A¹, 1 or 2 C₁₋₆ alkyl groups (e.g., a C₁₋₄ alkyl group such as a methyl group, a tert-butyl group, etc.), a phenyl group, a halogen atom (e.g., fluorine, chlorine, bromine, iodine atoms), etc. are preferably used.
As the integer of 1 to 4 for n, 1 to 3 is preferable, and 2 is particularly preferable.

As the compound represented by the formula (I), the compound represented by the formula (Ibb') is preferable, and the compound represented by the formula (Inn) is more preferable.
Furthermore, preferred as a compound represented by the formula (Ibb') or the formula (Inn) is that wherein R¹ is a lower alkyl group optionally having substituent(s) (more preferably R¹ is C₁₋₆ alkyl group (e.g., methyl group, ethyl group, n-propyl group, isopropyl group, n-butyl group,
isobutyl group, sec-butyl group, tert-butyl group etc.)), R² is a hydrogen atom or a lower (C₁₋₆)alkyl group (e.g., methyl group, ethyl group, n-propyl group, isopropyl group, n-butyl group, isobutyl group, sec-butyl group, tert-butyl group etc.), Ar is a phenyl group optionally having substituent(s) (more preferably Ar is phenyl group substituted by 1 or 2 halogen atoms (e.g., fluorine, chlorine, bromine, iodine)), and n is 1, 2 or 3 (more preferably n is 2).
As the compound represented by the formula (I), the compound represented by the formula (Ia):

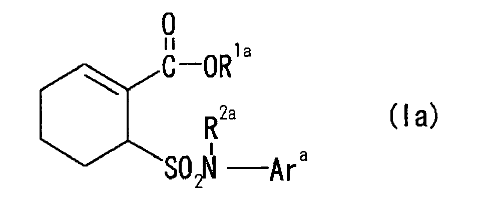

wherein R^{1a} is a C₁₋₆ alkyl group, R^{2a} is a hydrogen atom or a C₁-₆ alkyl group and Ar^{a} is a phenyl group substituted by 1 or 2 halogen atoms is preferable.
Preferable examples of the C₁₋₆ alkyl group for R^{1a} include a methyl group, an ethyl group, an n-propyl group, an isopropyl group, an n-butyl group, an isobutyl group, a sec-butyl group, a tert-butyl group and the like, preferably methyl group, ethyl group, n-propyl group and the like, particularly preferably ethyl group and the like.
Examples of the C₁₋₆ alkyl group for R^{2a} include a methyl group, an ethyl group, an n-propyl group, an isopropyl group, an n-butyl group, an isobutyl group, a sec-butyl group, a tert-butyl group and the like. Preferred as the R^{2a} are a hydrogen atom, a methyl group and the like.
Examples of the "halogen atom" in the "phenyl group substituted by 1 or 2 halogen atoms" for Ar^{a} include a fluorine atom, a chlorine atom, a bromine atom and an iodine atom, preferably a fluorine atom, a chlorine atom and the like. Examples of the "phenyl group substituted by 1 or 2 halogen atoms" for Ar^{a} include a 2,3-dichlorophenyl group, a 2,4-difluorophenyl group, a 2,4-dichlorophenyl group, a 2,6-dichlorophenyl group, a 2-fluorophenyl group, a 2-chlorophenyl group, a 3-chlorophenyl group, a 2-chloro-4-fluorophenyl group and the like, preferably 2,4-difluorophenyl group, 2-chlorophenyl group, 2-chloro-4-fluorophenyl group and the like, more preferably 2,4-difluorophenyl group, 2-chloro-4-fluorophenyl group and the like.
Specifically, as the compound represented by the formula (I), a compound obtained in Reference Example B to be mentioned below and the like is used. Among these,
(1) d-ethyl 6-[N-(2,4-difluorophenyl)sulfamoyl]-1-cyclohexene-1-carboxylate,
(2) ethyl 6-[N-(2-chlorophenyl)sulfamoyl]-1-cyclohexene-1-carboxylate,
(3) ethyl 6-[N-(2-chloro-4-methylphenyl)sulfamoyl]-1-cyclohexene-1-carboxylate, and
(4) ethyl (6R)-6-[(2-chloro-4-fluoroanilino)sulfonyl]-1-cyclohexene-1-carboxylate and salts thereof and the like are preferable.

The compound represented by the formula (II) is explained in detail.
In the present specification, R^{1'} is an aliphatic hydrocarbon group optionally having substituent(s), an aromatic hydrocarbon group optionally having substituent(s), a heterocyclic group optionally having substituent(s), a group represented by the formula: -OR^{1a'} wherein R^{1a'} is a hydrogen atom or an aliphatic hydrocarbon group optionally having substituent(s), or a group represented by the formula:

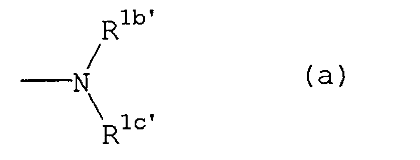

wherein R^{1b'} and R^{1c'} are the same or different and each is a hydrogen atom or an aliphatic hydrocarbon group optionally having substituent(s), particularly preferably a group represented by the formula: -OR^{1a'}.
As the "aliphatic hydrocarbon group optionally having substituent(s)", "aromatic hydrocarbon group optionally having substituent(s)" and "heterocyclic group optionally having substituent(s)" for R^{1'}, those similar to these substituents for R can be used.
As the "aliphatic hydrocarbon group optionally having substituent(s)" for R^{1a'}, for example, those similar to the aforementioned "aliphatic hydrocarbon group optionally having substituent(s)" for R¹ can be used. As R^{1a'}, for example, lower alkyl group having 1 to 6 carbon atoms optionally having substituent(s) (e.g., methyl group, ethyl group, n-propyl group, isopropyl group, n-butyl group, isobutyl group, tert-butoxycarbonylmethyl group, hydroxyethyl group etc.) and the like are preferably used. Of these, for example, a lower alkyl group having a carbon number of 1 to 6 (e.g., methyl group, ethyl group, n-propyl group, isopropyl group, n-butyl group, isobutyl group etc.) and the like are preferably used. Particularly preferred are, for example, a methyl group, an ethyl group, an n-propyl group and the like. Of these, an ethyl group and the like are preferable.

As the "aliphatic hydrocarbon group optionally having substituent(s)" for R^{1b'} and R^{1c'}, for example, those similar to the aforementioned "aliphatic hydrocarbon group optionally having substituent(s)" for R^{1b} and R^{1c} can be used. As R^{1b'} and R^{1c'}, for example, a lower alkyl group having 1 to 6 carbon atoms optionally having substituent(s) (e.g., methyl group, ethyl group, n-propyl group, isopropyl group, n-butyl group, isobutyl group, tert-butoxycarbonylmethyl group, hydroxyethyl group etc.) and the like are preferably used. Of these, for example, methyl group, ethyl group, n-propyl group, isopropyl group, n-butyl group, isobutyl group and the like are preferably used. Particularly, for example, methyl group, ethyl group, n-propyl group and the like are preferable, and ethyl group and the like are specifically preferable.

As R^{1'}, for example, a lower alkyl group having 1 to 6 carbon atoms optionally having substituent(s) (e.g., methyl group, ethyl group, n-propyl group, isopropyl group, n-butyl group, isobutyl group, tert-butoxycarbonylmethyl group, hydroxyethyl group etc.) and the like are preferably used. Of these, for example, methyl group, ethyl group, n-propyl group, isopropyl group, n-butyl group, isobutyl group and the like are preferably used. Particularly, for example, methyl group, ethyl group, n-propyl group and the like are preferable, and ethyl group and the like are specifically preferable.

In the present specification, Y is a methylene group optionally having substituent(s) or NH optionally having substituent(s).
As the "substituent" of the "methylene group optionally having substituent(s)" for Y, for example, C₁₋₆ alkyl group (e.g., methyl group, ethyl group, n-propyl group, isopropyl group, n-butyl group, isobutyl group etc.), hydroxy-substituted-C₁₋₆ alkyl group (e.g., hydroxymethyl group, hydroxyethyl group etc.), C₁₋₄ alkoxy-carbonyl-C₁₋₄ alkyl group (e.g., methoxycarbonylmethyl group, ethoxycarbonylmethyl group, tert-butoxycarbonylmethyl group, methoxycarbonylethyl group, ethoxycarbonylethyl group, tert-butoxycarbonylethyl group etc.) and the like can be mentioned. Of these, methyl group is preferable. Unsubstituted methylene is particularly preferable.
As the "substituent" of the "NH optionally having substituent(s)" for Y, C₁₋₆ alkyl group (e.g., methyl group, ethyl group, n-propyl group, isopropyl group, n-butyl group, isobutyl group etc.), hydroxy-substituted-C₁₋₆ alkyl group (e.g., hydroxymethyl group, hydroxyethyl group etc.), C₁₋₄ alkoxy-carbonyl-C₁₋₄ alkyl group (e.g., methoxycarbonylmethyl group, ethoxycarbonylmethyl group, tert-butoxycarbonylmethyl group, methoxycarbonylethyl group, ethoxycarbonylethyl group, tert-butoxycarbonylethyl group etc.) and the like can be mentioned. Of these, methyl group is preferable. Unsubstituted NH is particularly preferable.

In the "aromatic hydrocarbon group optionally having substituent(s)" for Ar', those similar to the aforementioned "aromatic hydrocarbon group optionally having substituent(s)" for Ar can be used.
Particularly, as Ar', those similar to Ar are preferable. Among others, a group represented by the formula (c):

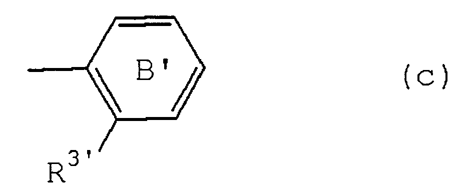

wherein R^{3'} is a halogen atom or a lower alkyl group, and ring B' is optionally further substituted by 1 to 4 halogen atoms is preferable, and a group represented by the formula (c1):

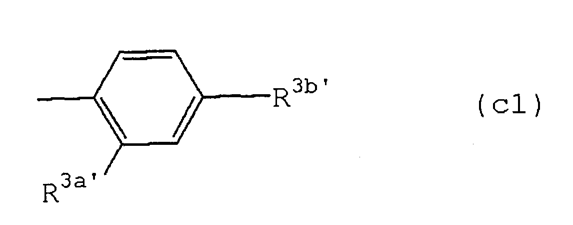

wherein R^{3a'} and R^{3b'} are the same or different and each is a halogen atom is more preferable.
Preferable examples of the Ar' include a phenyl group optionally having 1 or 2 substituents selected from the group consisting of a halogen atom (e.g., fluorine atom, chlorine atom, bromine atom, iodine atom, preferably fluorine atom, chlorine atom) and a C₁₋₆ alkoxy group (e.g., methoxy group etc.).

In the formula (c), examples of the halogen atom for R^{3'} and halogen atom as a substituent for ring B' and halogen atom for R^{3a'} or R^{3b'} in the formula (c1) include a fluorine atom, a chlorine atom, a bromine atom and an iodine atom, preferably a fluorine atom and a chlorine atom. In the formula (c), examples of the lower alkyl group for R^{3'} include a C₁₋₄ alkyl group such as methyl, ethyl, propyl and the like, preferably a methyl group and the like. Of the groups represented by the formula (c), a 2,4-difluorophenyl group, a 2-chloro-4-fluorophenyl group, a 2-methyl-4-chlorophenyl group and the like are preferable. Of the groups represented by the formula (c1), a 2,4-difluorophenyl group, a 2-chloro-4-fluorophenyl group and the like are preferable.
Preferred as the Ar' are also a phenyl group, a 4-methoxyphenyl group and the like.

X is a methylene group, NH, a sulfur atom or an oxygen atom, wherein a methylene group and an oxygen atom are preferable.
When X is a methylene group, Y should be a methylene group optionally having substituent(s).
Ring A' is a 5- to 8-membered ring substituted by a group represented by the formula: -CO-R^{1'}, wherein R^{1'} is as defined above and a group represented by the formula: -SO₂Y-Ar' wherein Y and Ar' are as defined above, and optionally further substituted by 1 to 4 substituents selected from the group consisting of (i) an aliphatic hydrocarbon group optionally having substituent(s), (ii) an aromatic hydrocarbon group optionally having substituent(s), (iii) a group represented by the formula: -OR^{2'} wherein R^{2'} is as defined above and (iv) a halogen atom, with preference given to a 5- to 8-membered ring optionally having 1 to 4 substituents selected from (i) an aliphatic hydrocarbon group optionally having substituent(s), (ii) an aromatic hydrocarbon group optionally having substituent(s) and (iv) a halogen atom.
These substituents are present at substitutable positions on the ring A'. When X constituting the ring is NH or a methylene group, they can substitute the NH and methylene group. When ring A' is substituted by plural substituents, the kinds of such substituents may be the same or different. In addition, two substituents may substitute on the same carbon atom.
As the "aliphatic hydrocarbon group optionally having substituent(s)" and "aromatic hydrocarbon group optionally having substituent(s)", which are substituents of ring A', for example, those similar to the aforementioned groups for R can be mentioned.

As the "aliphatic hydrocarbon group optionally having substituent(s)" for R^{2'}, for example, those similar to the aforementioned groups for R¹¹ can be mentioned.
As the substituent for ring A', 1 or 2 C₁₋₆ alkyl groups (e.g., C₁₋₄ alkyl group such as methyl group, tert-butyl group etc.), phenyl groups, halogen atoms (e.g., fluorine, chlorine, bromine, iodine etc.) and the like are preferably used.
The "s" is an integer of 0 to 2, "t" is an integer of 1 to 3, and the total of "s" and "t" is 4 or less, with preference given to "s" being 1 and "t" being 1.
A group represented by the formula:

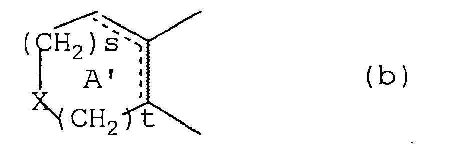

is a group represented by the formula:

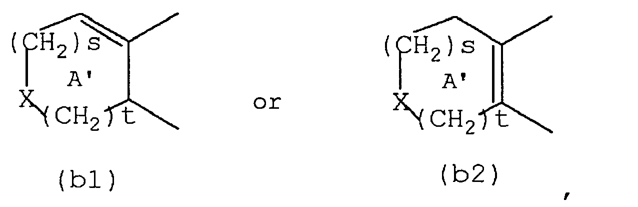

preferably a group represented by the formula (b1).

Preferred as the compound represented by the formula (II) (compound (II)) are the following compounds and the like.
(1) Compound (II) wherein R^{1'} is a group represented by the formula: -OR^{1a'} wherein R^{1a'} is a C₁₋₆ alkyl group, a group represented by the formula:

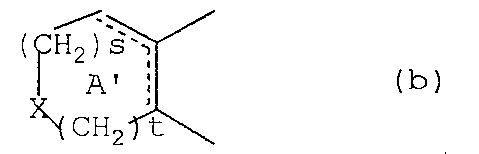

is a group represented by the formula:

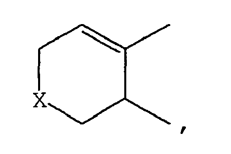

X is a methylene group or an oxygen atom,
Y is a methylene group or -NH-, and
Ar' is a phenyl group optionally having 1 or 2 substituents selected from the group consisting of a halogen atom and a C₁₋₆ alkoxy group, that is, a compound represented by the formula (IIa):

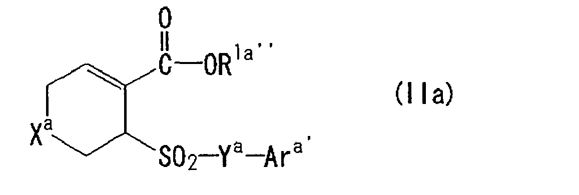

wherein R^{1a''} is a C₁₋₆ alkyl group, X^{a} is a methylene group or an oxygen atom, Y^{a} is a methylene group or -NH-, Ar^{a'} is a phenyl group optionally having 1 or 2 substituents selected from the group consisting of a halogen atom and a C₁₋₆ alkoxy group, provided that when X^{a} is a methylene group, then Y^{a} is a methylene group.
As the C₁₋₆ alkyl group for R^{1a''}, for example, a methyl group, an ethyl group, an n-propyl group, an isopropyl group, an n-butyl group, an isobutyl group and the like are preferably used. Particularly, for example, a methyl group, an ethyl group, an n-propyl group and the like are preferable. Of these, an ethyl group and the like are preferable. Examples of the halogen atom of the substituent for Ar^{a'} include a fluorine atom, a chlorine atom, a bromine atom and an iodine atom, preferably a fluorine atom or a chlorine atom. Examples of the C₁₋₆ alkoxy group of the substituent for Ar^{a'} include a methoxy group and the like, preferably a methoxy group.

(2) Compound (II) wherein R^{1'} is a group represented by the formula: -OR^{1a'}, R^{1a'} is a C₁₋₆ alkyl group (e.g., methyl group, ethyl group, n-propyl group, isopropyl group, n-butyl group, isobutyl group etc, can be mentioned, methyl group, ethyl group, n-propyl group etc. are preferable, and of these, ethyl group and the like are preferable), a group represented by the formula:

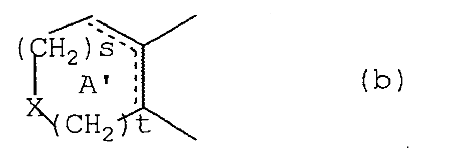

is a group represented by the formula:

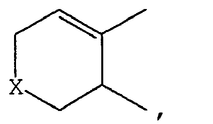

X and Y are each a methylene group, or X is an oxygen atom and Y is -NH-, and Ar' is a phenyl group optionally having two halogen atoms (e.g., fluorine atom, chlorine atom, bromine atom and iodine atom can be mentioned, and fluorine atom and chlorine atom are preferable) (e.g., 2-chloro-4-fluorophenyl group etc).

(3) Ethyl 6-(benzylsulfonyl)-1-cyclohexene-1-carboxylate (compound 1'),
ethyl 6-[(4-methoxybenzyl)sulfonyl]-1-cyclohexene-1-carboxylate (compound 2'),
ethyl 6-[(2,4-difluorobenzyl)sulfonyl]-1-cyclohexene-1-carboxylate (compound 3'),
ethyl 6-[(2-chloro-4-fluorobenzyl)sulfonyl]-1-cyclohexene-1-carboxylate (compound 4'),
ethyl (-)-6-[(2-chloro-4-fluorobenzyl)sulfonyl]-1-cyclohexene-1-carboxylate (compound 5'),
ethyl (+)-6-[(2-chloro-4-fluorobenzyl)sulfonyl]-1-cyclohexene-1-carboxylate (compound 6'),
ethyl 3-[(2,4-difluorophenyl)sulfamoyl]-3,6-dihydro-2H-pyran-4-carboxylate (compound 7'), and
ethyl 3-[(2-chloro-4-fluorophenyl)sulfamoyl]-3,6-dihydro-2H-pyran-4-carboxylate (compound 8').
(4) Ethyl 6-[(2-chloro-4-fluorobenzyl)sulfonyl]-1-cyclohexene-1-carboxylate (compound 4'),
ethyl (+)-6-[(2-chloro-4-fluorobenzyl)sulfonyl]-1-cyclohexene-1-carboxylate (compound 6'), and
ethyl 3-[(2-chloro-4-fluorophenyl)sulfamoyl]-3,6-dihydro-2H-pyran-4-carboxylate (compound 8').

When the compounds represented by the formulas (I) and (II) have stereoisomers, each stereoisomer and a mixture of these stereoisomers are encompassed in the present invention.
Furthermore, when the compound represented by the formula (I) is a compound represented by the formula (Icc) or (Inn), and a group represented by the formula (b) of the compound represented by the formula (II) is a group represented by the formula (b1) and s and t are 1, each has an optical isomer based on the asymmetric carbon in cycloalkene or cyclohexene ring. Such optical isomer and a mixture of such optical isomers are both encompassed in the present invention.

The compounds represented by the formula (I) and formula (II) to be used for the pharmaceutical agent of the present invention (hereinafter to be collectively abbreviated as compound A') may be converted into a salt with an inorganic base, organic base, inorganic acid, organic acid, basic or acidic amino acid, and the like. As the salt with an inorganic base, for example, an alkaline metal salt such as sodium and potassium salts, etc.; an alkaline earth metal salt such as calcium and magnesium salts, etc.; aluminum salt; ammonium salt; and the like are used. As the salt with an organic base, for example, a salt with trimethylamine, triethylamine, pyridine, picoline, ethanolamine, diethanolamine, triethanolamine, dicyclohexylamine, N,N'-dibenzylethylenediamine, etc can be used. As the salt with an inorganic acid, for example, a salt with hydrochloric acid, hydrobromic acid, nitric acid, sulfuric acid, phosphoric acid, etc can be used. As the salt with an organic acid, for example, a salt with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, and the like can be used. As the salt with a basic amino acid, for example, a salt with arginine, lysine, ornithine, etc can be used. As the salt with acidic amino acid, for example, a salt with aspartic acid, glutamic acid, and the like can be used.

A prodrug of compound A' or a salt thereof is a compound which is converted into compound A' or a salt thereof as a result of a reaction with an enzyme, gastric acid etc. under physiological conditions in vivo. Thus, the compound is converted into compound A' or a salt thereof by enzymatical oxidation, reduction, hydrolysis etc., by hydrolysis due to gastric acid etc. A prodrug of compound A' or a salt thereof may be a compound obtained by subjecting an amino group of compound A' or a salt thereof to an acylation, alkylation or phosphorylation (e.g., a compound obtained by subjecting an amino group of compound A' or a salt thereof to an eicosanoylation, alanylation, pentylaminocarbonylation, 2-hydroxypropionylation, 2-acetoxypropionylation, (5-methyl-2-oxo-1,3-dioxolen-4-yl)methoxycarbonylation, tetrahydrofuranylation, pyrrolidylmethylation, pivaloyloxymethylation, tert-butylation, etc.); a compound obtained by subjecting a hydroxy group of compound A' or a salt thereof to an acylation, alkylation, phosphorylation or boration (e.g., a compound obtained by subjecting a hydroxy group of compound A' or a salt thereof to an acetylation, palmitoylation, propanoylation, pivaloylation, succinylation, fumarylation, alanylation, dimethylaminomethylcarbonylation, etc.); a compound obtained by subjecting a carboxyl group of compound A' or a salt thereof to an esterification or amidation (e.g., a compound obtained by subjecting a carboxyl group of compound A' or a salt thereof to an ethyl-esterification, phenyl-esterification, carboxymethylesterification, dimethylaminomethyl-esterification, pivaloyloxymethyl-esterification, ethoxycarbonyloxyethyl-esterification, phthalidyl-esterification, (5-methyl-2-oxo-1,3-dioxolen-4-yl)methyl-esterification, cyclohexyloxycarbonylethyl-esterification and methylamidation, etc.) and the like. Any of these compounds can be produced from compound A' or a salt thereof by a method known per se.
A prodrug of compound A' or a salt thereof may also be one which is converted into compound A' or a salt thereof under a physiological condition, such as those described in "IYAKUHIN no KAIHATSU (Development of Pharmaceuticals)", Vol.7, Design of Molecules, p. 163-198, Published by HIROKAWA SHOTEN (1990) .

The compound represented by the formula (I), a salt thereof and a prodrug thereof can be produced according to a method known per se, for example, a production method described in WO99/46242 or a method analogous thereto. The compound represented by the formula (II), a salt thereof and a prodrug thereof can be produced according to a production method described in WO01/10826 or a method analogous thereto.

When the optically active compound or a salt thereof contains an enantiomer, general separation means may be applied such as diastereomeric salt methods wherein a salt with an optically active acid (e.g., camphor sulfonic acid etc.) or optically active base (e.g., 1-methylbenzylamine etc.) is formed, inclusion compound methods using an optically active host molecule (e.g., 1,6-bis(2-chlorophenyl)-1,6-diphenylhexa-2,4-diyn-1,6-diol), various chromatographies (e.g., liquid chromatography using a chiral column etc.), fractional recrystallization and the like, whereby an optically pure compound can be obtained.
The compound A' or a salt thereof or a prodrug thereof (hereinafter to be comprehensively referred to as compound A) may be a solvate or a non-solvate.
In addition, compound A may be labeled with an isotope (e.g., ³H, ¹⁴C, ³⁵S, ¹²⁵I etc.) and the like.

Compound A in the present invention is highly safe to human body, and can be used as an pharmaceutical agent (e.g., an agent for the prophylaxis or treatment of various diseases), animal drug and the like for mammals (e.g., rat, mouse, guinea pig, monkey, bovine, dog, swine, cat, horse, goat, human etc.)
Compound A in the present invention is low toxic and shows a nitric oxide(NO) production suppressive action, a suppressive action on the production of inflammatory cytokines such as TNF-α, IL-1, IL-6 and the like, a TLR signal inhibitory action (particularly, TLR4 signal inhibitory action) and the like, and can be used as
(i) an agent for improving prognosis after coronary bypass surgery, an agent for improving ischemic reperfusion disorder and postoperative complications, tissue disorders and rejection after organ transplantation and the like, and of these, an agent for the prophylaxis or treatment of complications after coronary-artery bypass surgery (e.g., respiratory disorder, renal failure, cardiac failure, liver failure etc.) in mammals (e.g., cat, bovine, dog, horse, goat, monkey, human etc.),
(ii) an agent for the prophylaxis or treatment of cardiac disease, autoimmune diseases, inflammatory diseases, central nervous system diseases, sepsis, severe sepsis or septic shock in patients who undergoes coronary bypass surgery, and the like.
   In addition, compound A in the present invention can be used as, for example, an agent for the prophylaxis or treatment of coronary heart diseases, ischemic reperfusion disorders during transplantation, postoperative complications (e.g., endotoxemia, reperfusion disorder), tissue disorders and rejection after organ transplantation, inflammatory bowel disease (IBD), acute hepatitis, acute pancreatitis, neuropathy and the like.
   The patient who undergoes coronary bypass surgery may be any of patients before and after coronary bypass surgery.

Compound A can be used as a concomitant drug in combination of other drugs. Examples of such concomitant drug include antibacterial agents, antifungal agents, antivirus drugs, non-steroidal antiinflammatory drugs, steroids, anticoagulants, antithrombotic drugs, thrombolytic drugs, methemoglobin increase preventive drugs, immunomodulators, antiprotozoals, antitussive and expectorant drugs, sedatives, anesthetics, antinarcotics, antiulcer drugs, hyperlipidemia treating agents, therapeutic agents for arteriosclerosis, HDL increasing agents, unstable plaque stabilizing agents, myocardial protecting agent, hypothyroidism treating agent, nephrotic syndrome treating agent, chronic renal failure treating agent, diuretics, hypertension treating agents, cardiac failure treating agents, muscle relaxants, anticonvulsants, cardiacs, vasodilators, vasoconstrictors, antiarrhythmics, antidiabetic drugs, agents for improving prognosis after coronary bypass surgery, hypertensors, tranquilizers, antipsychotics, antiemetics, therapeutic agents for Alzheimer's diseases, anti-Parkinson drugs, therapeutic agents for amyotrophic spinal lateral sclerosis, neurotrophic factors, antidepressants, therapeutic agents for schizophrenia, antitumor drugs, vitamins, vitamin derivatives, therapeutic agents for arthritis, antirheumatics, antiallergic drugs, antiasthmatics, therapeutic agents for atopic dermatitis, therapeutic agents for allergic rhinitis, therapeutic agents for pollakisuria/anischuria, protease drugs, protease inhibitors, anti-SIDS drugs, anti-sepsis drugs, anti-septic shock drugs, endotoxin-antagonists or -antibodies, signal transduction inhibitors, inhibitors of inflammatory mediator activity, antibodies to inhibit inflammatory mediator activity, inhibitors of inflammatory mediator production, inhibitors of anti-inflammatory mediator activity, antibodies to inhibit anti-inflammatory mediator activity, inhibitors of anti-inflammatory mediator production, α1-adrenergic agonists, cytopathy suppressive drug and the like. Of these, antibacterial agents, antifungal agents, antivirus agents, non-steroidal antiinflammatory drugs, steroids, anticoagulants, cytopathy suppressive drugs, anti-sepsis drugs and the like
are preferable. Specifically, the following agents can be mentioned.

### (1) Antibacterial agents

(i) Sulfa drugs
   sulfamethizole, sulfisoxazole, sulfamonomethoxine, sulfamethizole, salazosulfapyridine, silver sulfadiazine and the like.
(ii) Quinoline antibacterial agents
   nalidixic acid, pipemidic acid trihydrate, enoxacin, norfloxacin, ofloxacin, tosufloxacin tosilate, ciprofloxacin hydrochloride, lomefloxacin hydrochloride, sparfloxacin, fleroxacin and the like.
(iii) Antiphthisics
   isoniazid, ethambutol (ethambutol hydrochloride), p-aminosalicylic acid (calcium p-aminosalicylate), pyrazinamide, ethionamide, protionamide, rifampicin, streptomycin sulfate, kanamycin sulfate, cycloserine and the like.
(iv) Antiacidfast bacterium drugs diaphenylsulfone, rifampicin and the like.
(v) Antiviral drugs
   idoxuridine, acyclovir, vidarabine, ganciclovir and the like.
(vi) Anti-HIV agents
   zidovudine, didanosine, zalcitabine, indinavir sulfate ethanolate, ritonavir and the like.
(vii) Antispirocheteles
(viii) Antibiotics
   tetracycline hydrochloride, ampicillin, piperacillin, gentamicin, dibekacin, kanendomycin, lividomycin, tobramycin, amikacin, fradiomycin, sisomycin, tetracycline, oxytetracycline, rolitetracycline, doxycycline, ampicillin, piperacillin, ticarcillin, cephalothin, cephapirin, cephaloridine, cefaclor, cephalexin, cefroxadine, cefadroxil, cefamandole, cefotoam, cefuroxime, cefotiam, cefotiam hexetil, cefuroxime axetil, cefdinir, cefditoren pivoxil, ceftazidime, cefpiramide, cefsulodin, cefmenoxime, cefpodoxime proxetil, cefpirome, cefozopran, cefepime, cefsulodin, cefmenoxime, cefmetazole, cefminox, cefoxitin, cefbuperazone, latamoxef, flomoxef, cefazolin, cefotaxime, cefoperazone, ceftizoxime, moxalactam, thienamycin, sulfazecin, aztreonam or a salt thereof, griseofulvin, lankacidin-group [Journal of Antibiotics (J. Antibiotics), 38, 877-885(1985)] and the like.
(2) Antifungal agents
   (i) polyethylene antibiotics (e.g., amphotericin B, nystatin, trichomycin)
   (ii) griseofulvin, pyrrolnitrin and the like.
   (iii) cytosine metabolism antagonists (e.g., flucytosine)
   (iv) imidazole derivatives (e.g., econazole, clotrimazole, miconazole nitrate, bifonazole, croconazole)
   (v) triazole derivatives (e.g. fluconazole, itraconazole, azole compound [2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone]
   (vi) thiocarbamic acid derivatives (e.g. trinaphthol)
   (vii) echinocandin derivatives (e.g., caspofungin, micafungin, anidulafungin) and the like.
(3) Non-steroidal antiinflammatory drugs
   acetaminophen, phenacetin, ethenzamide, sulpyrine, antipyrine, migrenin, aspirin, mefenamic acid, flufenamic acid, diclofenac sodium, loxoprofen sodium, phenylbutazone, indomethacin, ibuprofen, ketoprofen, naproxen, oxaprozin, flurbiprofen, fenbufen, pranoprofen, floctafenine, epirizole, tiaramide hydrochloride, zaltoprofen, gabexate mesylate, camostat mesylate, urinastatin, colchicine, probenecid, sulfinpyrazone, benzbromarone, allopurinol, gold sodium thiomalate, sodium hyaluronate, sodium salicylate, morphine hydrochloride, salicylic acid, atropine, scopolamine, morphine, pethidine, levorphanol, ketoprofen, naproxen, oxymorphone or a salt thereof, and the like.
(4) Steroids
   dexamethasone, hexestrol, methimazole, betamethasone, triamcinolone, triamcinolone acetonide, fluocinonide, fluocinolone acetonide, prednisolone, methylprednisolone, cortisone acetate, hydrocortisone, fluorometholone, beclometasone propionate, estriol and the like.
(5) Anticoagulants
   heparin sodium, sodium citrate, activated protein C, tissue factor pathway inhibitor, antithrombin III, dalteparin sodium, warfarin potassium, argatroban, gabexate, sodium citrate and the like.

(6) Antithrombotic drugs
   ozagrel sodium, ethyl icosapentate, beraprost sodium, alprostadil, ticlopidine hydrochloride, pentoxifylline, dipyridamole and the like.
(7) Thrombolytic drugs
   tisokinase, urokinase, streptokinase and the like.
(8) methemoglobin increase preventive drugs Methylene blue, ascorbic acid and the like.
(9) Immunomodulators
   cyclosporin, tacrolimus, gusperimus, azathioprine, antilymphocyte serum, dried sulfonated immunoglobulin, erythropoietin, colony-stimulating factor, interleukin, interferon and the like.
(10) Antiprotozoals
   metronidazole, tinidazole, diethylcarbamazine citrate, quinine hydrochloride, quinine sulfate and the like.
(11) Antitussive and expectorant drugs
   ephedrine hydrochloride, noscapine hydrochloride, codeine phosphate, dihydrocodeine phosphate, isoproterenol hydrochloride, ephedrine hydrochloride, methylephedrine hydrochloride, noscapine hydrochloride, alloclamide, chlophedianol, picoperidamine, chloperastine, protokylol, isoproterenol, salbutamol, terbutaline, oximetebanol, morphine hydrochloride, dextromethorphan hydrobromide, oxycodone hydrochloride, dimemorphan phosphate, tipepidine hibenzate, pentoxyverine citrate, clofedanol hydrochloride, benzonatate, guaifenesin, bromhexine hydrochloride, ambroxol hydrochloride, acetylcysteine, ethyl cysteine hydrochloride, carbocysteine and the like.
(12) Sedatives
   chlorpromazine hydrochloride, atropine sulfate, phenobarbital, barbital, amobarbital, pentobarbital, thiopental sodium, thiamylal sodium, nitrazepam, estazolam, flurazepam, haloxazolam, triazolam, flunitrazepam, bromovalerylurea, chloral hydrate, triclofos sodium and the like.
(13) Anesthetics
(13-1) Local anesthetics
   cocaine hydrochloride, procaine hydrochloride, lidocaine, dibucaine hydrochloride, tetracaine hydrochloride, mepivacaine hydrochloride, bupivacaine hydrochloride, oxybuprocaine hydrochloride, ethyl aminobenzoate, oxethazaine and the like.
(13-2) General anesthetics
   (i) inhalation anesthetics (e.g., ether, halothane, nitrous oxide, influrane, enflurane),
   (ii) intravenous anesthetics (e.g., ketamine hydrochloride, droperidol, thiopental sodium, thiamylal sodium, pentobarbital) and the like.
(14) Antinarcotics
   levallorphan, nalorphine, naloxone or a salt thereof and the like.
(15) Antiulcer drugs
   metoclopromide, histidine hydrochloride, lansoprazole, metoclopramide, pirenzepine, cimetidine, ranitidine, famotidine, urogastrone, oxethazaine, proglumide, omeprazole, sucralfate, sulpiride, cetraxate, gefarnate, aldioxa, teprenone, prostaglandin and the like.

(16) Hyperlipidemia treating agents
   HMG-CoA reductase inhibitors (e.g., fluvastatin, cerivastatin, atorvastatin etc.), fibrates (e.g., simfibrate, clofibrate aluminum, clinofibrate, fenofibrate etc.), adsorbents for bile acid (e.g., cholestyramine etc.), nicotinic acid formulations (e.g., nicomol, niceritrol, tocopherol nicotinate etc.), probucol and a derivative thereof, polyvalent unsaturated fatty acid derivatives (e.g., ethyl icosapentate, polyene phosphatidylcholine, melinamide etc.), vegetable sterols (e.g., gamma-oryzanol, soysterol etc.), elastases, sodium dextran sulfate, squalene synthetase inhibitor, squalene epoxidase inhibitor, CETP inhibitor, ethyl 2-chloro-3-[4-(2-methyl-2-phenylpropoxy)phenyl]propionate [chemical and pharmaceutical bulletin (Chem. Pharm. Bull.), 38, 2792, 2796 (1990)], LDL receptor enhancing drug, cholesterol absorption inhibitor (Ezetimibe etc.), MTP inhibitor, ileal bile acid transporter inhibitor, SCAP ligand, FXR ligand and the like.
(17) Therapeutic agents for arteriosclerosis
   MMP inhibitor, chymase inhibitor, ACAT inhibitor(Avasimibe, Eflucimibe etc.), apoAI Milano and an analogue thereof, scavenger receptor inhibitor, 15-lipoxygenase inhibitor, phospholipase A2 inhibitor, ABCA1 activator, LXR ligand, sphingomyelinase inhibitor, paraoxonase activator, estrogen receptor agonist and the like.
(18) HDL increasing agents
   squalene synthetase inhibitors, CETP inhibitors, LPL activators and the like.
(19) Unstable plaque stabilizing agents
   MMP inhibitors, chymase inhibitors, ACAT inhibitors, lipid-rich plaque regressing agents and the like.
(20) Myocardial protecting agents
   cardiac ATP-K oral formulation, endoserine antagonist, urotensin antagonist and the like.
(21) Hypothyroidism treating agents
   dried thyroid gland (thyreoid), levothyroxine sodium (thyradin S), liothyronidin sodium (thyronine, thyromin) and the like.
(22) Nephrotic syndrome treating agents
   prednisolone (Predonine), prednisolone succinate sodium (Predonine), methylprednisolone succinate sodium (Solu medrol), betamethasone (rinderon) and the like.
(23) Chronic renal failure treating agents
   diuretics [e.g., furosemide (Lasix), bumetamide (Lunetron), azosemide (Diart)], hypotensive agent (e.g., ACE inhibitor, (enalapril maleate (Renivase)) and Ca antagonist (manidipine), α-receptor blocker, AII antagonist (candesartan)] and the like.
(24) Diuretics
   thiazide diuretics (benzylhydro-chlorothiazide, cyclopenthiazide, ethiazide, hydrochlorothiazide, hydroflumethiazide, methyclothiazide, penfluthiazide, polythiazide, trichloromethiazide etc.), loop diuretics (clortharidone, clofenamide, indapamide, mefruside, meticrane, sotolazone, tripamide, quinethazone, metolazone, furosemide etc.), potassium retaining diuretics (spironolacton, triamterene etc.).

(25) Hypertension treating agents
   (i) sympathetic nerve suppressants
      α₂ stimulants (e.g., clonidine, guanabenz, guanfacine, methyldopa etc.), ganglionic blocking agents (e.g., hexamethonium, trimethaphan etc.), presynaptic blockers (e.g., alseroxylon, dimethylaminoreserpinate, rescinamine, reserpine, syrosingopine etc.), neuron blockers (e.g., betanidine, guanethidine etc.), α₁ blockers (e.g., bunazosin, doxazocin, prazosin, terazosin, urapidil etc.), β blockers (e.g., propranolol, nadolol, timolol, nipradilol, bunitrolol, indenolol, penbutolol, carteolol, carvedilol, pindolol, acebutolol, atenolol, bisoprolol, metoprolol, labetalol, amosulalol, arotinolol etc.).
   (ii) vasodilators
      calcium channel antagonists (e.g., manidipine, nicardipine, nilvadipine, nisoldipine, nitrendipine, benidipine, amlodipine, aranidipine etc.), phthalazine derivatives (e.g., budralazine, cadralazine, ecarazine, hydralazine, todralazine etc.) and the like.
   (iii) ACE inhibitors
      alacepril, captopril, cilazapril, delapril, enalapril, lisinopril, temocapril, trandolapril, quinapril, imidapril, benazepril, perindopril and the like.
   (iv) AII antagonists
      losartan, candesartan, valsartan, termisartan, irbesartan, forasartan and the like.
   (v) diuretics (e.g., diuretics described above)
(26) Cardiac failure treating agents
   cardiotonic agents (e.g., digitoxin, digoxin, methyldigoxin, lanatoside C, proscillaridine etc.), α, β-stimulants (e.g., epinephrine, norepinephrine, isoproterenol, dopamine, docarpamine, dobutamine, denopamine etc.), phosphodiesterase inhibitors (e.g., amrinone, milrinone, olprinone hydrochloride etc.), calcium channel sensitivity promoters (e.g., pimobendan etc.), nitrate agents (e.g., nitroglycerin, isosorbide nitrate etc.), ACE inhibitors (e.g., ACE inhibitors described above), diuretics (e.g., diuretics described above), carperitide, ubidecarenone, vesnarinone, aminophylline and the like.
(27) Muscle relaxants
   pridinol, tubocurarine, pancuronium, tolperisone hydrochloride, chlorphenesin carbamate, baclofen, chlormezanone, mephenesin, chlorzoxazone, eperisone, tizanidine and the like.
(28) Anticonvulsants
   phenytoin, ethosuximide, acetazolamide, chlordiazepoxide, trimethadione, carbamazepine, phenobarbital, primidone, sulthiame, sodium valproate, clonazepam, diazepam, nitrazepam and the like.
(29) Cardiacs
   trans-π-oxocamphor, terephyllol, aminophyllin, etilefrine, dopamine, dobutamine, denopamine, aminophyllin, vesnarinone, amrinone, pimobendan, ubidecarenone, digitoxin, digoxin, methyldigoxin, lanatoside C, G-strophanthin and the like.
(30) Vasodilators
   oxyfedrine, diltiazem, tolazoline, hexobendine, bamethan, clonidine, methyldopa, guanabenz and the like.
(31) Vasoconstrictors
   dopamine, dobutamine, denopamine and the like.
(32) Antiarrhythmics
   (i) Na channel blockers (e.g., quinidine, procainamide, disopyramide, ajmaline, cibenzoline, lidocaine, diphenylhydantoin, mexiletine, propafenone, flecainide, pilsicainide, phenitoin etc.),
   (ii) β-blockers (e.g., propranolol, alprenolol, bufetolol, oxprenolol, atenolol, acebutolol, metoprolol, bisoprolol, pindolol, carteolol, arotinolol etc.),
   (iii) K channel blockers (e.g., amiodarone etc.),
   (iv) Ca channel blockers (e.g., verapamil, diltiazem etc.) and the like.
(33) Hypertensors dopamine, dobutamine, denopamine, digitoxin, digoxin, methyldigoxin, lanatoside C, G-strophanthin and the like.
(34) Prognosis improving drug after coronary-artery bypass surgery
   E-5564 and the like.
(35) Antidiabetic drugs
   sulfonylureas (e.g., tolbutamide, chlorpropamide, glyclopyramide, acetohexamide, tolazamide, glibenclamide, glybuzole etc.), biguanides (e.g., metformin hydrochloride, buformin hydrochloride etc.), α-glucosidase inhibitors (e.g., voglibose, acarbose etc.), insulin sensitizers (e.g., pioglitazone, rosiglitazone, troglitazone etc.), insulin, glucagon, agents for treating diabetic complications (e.g., epalrestat etc.) and the like.

(36) Tranquilizers diazepam, lorazepam, oxazepam, chlordiazepoxide, medazepam, oxazolam, cloxazolam, clotiazepam, bromazepam, etizolam, fludiazepam, hydroxyzine and the like.
(37) Antipsychotics chlorpromazine hydrochloride, prochlorperazine, trifluoperazine, thioridazine hydrochloride, perphenazine maleate, fluphenazine enanthate, prochlorperazine maleate, levomepromazine maleate, promethazine hydrochloride, haloperidol, bromperidol, spiperone, reserpine, clocapramine hydrochloride, sulpiride, zotepine and the like.
(38) Antiemetic
   phenothiazine derivative, 5-HT3 receptor antagonist and the like.
(39) Therapeutic agents for Alzheimer's diseases
   (i) choline esterase inhibitors such as donepezil, rivastigmine, galanthamine, TAK-147 and the like.
   (ii) cerebral function activators such as Idebenone, Memantine, vinpocetine and the like.
(40) Anti-Parkinson drugs
   L-dopa, Deprenyl, carbidopa+levodopa, Pergolide, Ropinirole, cabergoline, Pramipexol, Entacapone, Lazabemide and the like. (41) Therapeutic agents for amyotrophic spinal lateral sclerosis
   riluzole, mecasermin, Gabapentin and the like.
(42) Antidepressants imipramine, clomipramine, noxiptiline, phenelzine, amitriptyline hydrochloride, nortriptyline hydrochloride, amoxapine, mianserin hydrochloride, maprotiline hydrochloride, sulpiride, fluvoxamine maleate, trazodone hydrochloride and the like.
(43) Therapeutic agents for schizophrenia
   Olanzapine, risperidone, Quetiapine, Iloperidone and the like.
(44) Antitumor drugs
   6-O-(N-chloroacetylcarbamoyl)fumagillol, bleomycin, methotrexate, actinomycin D, mitomycin C, daunorubicin, adriamycin, neocarzinostatin, cytosine arabinoside, fluorouracil, tetrahydrofuryl-5-fluorouracil, picibanil, lentinan, levamisole, bestatin, azimexon, glycyrrhizin, doxorubicin hydrochloride, aclarubicin hydrochloride, bleomycin hydrochloride, peplomycin sulfate, vincristine sulfate, vinblastine sulfate, irinotecan hydrochloride, cyclophosphamide, melphalan, busulphan, thiotepa, procarbazine hydrochloride, cisplatin, azathioprine, mercaptopurine, tegafur, carmofur, cytarabine, methyltestosterone, testosterone propionate, testosterone enanthate, mepitiostane, fosfestol, chlormadinone acetate, leuprorelin acetate, buserelin acetate and the like.
(45) Vitamins
   (i) vitamin A: vitamin A₁, vitamin A₂ and retinol palmitate
   (ii) vitamin D: vitamin D₁, D₂, D₃, D₄ and D₅
   (iii) vitamin E: α-tocopherol, β-tocopherol, γ-tocopherol, δ-tocopherol, dl-α-tocopherol nicotinate
   (iv) vitamin K: vitamin K₁, K₂, K₃ and K₄
   (v) folic acid (vitamin M)
   (vi) vitamin B: vitamin B₁, vitamin B₂, vitamin B₃, vitamin B₅, vitamin B₆ and vitamin B₁₂
   (vii) biotin (vitamin H) and the like.
(46) Vitamin derivatives
   various derivatives of vitamins, for example, ascorbic acid, vitamin D₃ derivatives such as 5,6-trans-cholecalciferol, 2,5-hydroxycholecalciferol, 1-α-hydroxycholecalciferol and the like, vitamin D₂ derivatives such as 5,6-trans-ergocalciferol and the like.

(47) Antiallergic drugs
   diphenhydramine, chlorpheniramine, tripelennamine, metodilamine, clemizole, diphenylpyraline, methoxyphenamine, sodium cromoglycate, tranilast, repirinast, amlexanox, ibudilast, ketotifen, terfenadine, mequitazine, azelastine, epinastine, ozagrel hydrochloride, pranlkast hydrate, seratrodast and the like.
(48) Antiasthmatics isoprenaline hydrochloride, salbutamol sulfate, procaterol hydrochloride, terbutaline sulfate, trimetoquinol hydrochloride, tulobuterol hydrochloride, orciprenaline sulfate, fenoterol hydrobromide, ephedrine hydrochloride, iprotropium bromide, oxitropium bromide, flutropium bromide, theophyline, aminophyllin, sodium cromoglycate, tranilast, repirinast, anrexanone, ibudilast, ketotifen, terfenadine, mequitazine, azelastine, epinastine, ozagrel hydrochloride, pranlkast hydrate, seratrodast, dexamethasone, prednisolone, hydrocortisone, beclometasone dipropionate and the like.
(49) Therapeutic agents for atopic dermatitis sodium cromoglycate and the like.
(50) Therapeutic agents for allergic rhinitis sodium cromoglycate, chlorpheniramine maleate, alimemazine tartrate, clemastine fumarate, homochlorcyclizine hydrochloride, terfenadine, mequitazine and the like.
(51) Therapeutic agents for pollakisuria/anischuria flavoxate hydrochloride and the like.
(52) Anti-sepsis drugs
   peptidic compounds such as rBPI-21 (bactericidal permeability increasing protein), BI-51017 (antithrombin III), SC-59735 (rTFPI), r-PAF acetylhydrase, LY-203638 (r-activated protein C), anti-TNF-α antibody, anti-CD14 antibody, CytoFab, alkaline phosphatase (LPS inactivator) and the like, and non-peptidic compounds such as JTE-607, E-5531, E-5564, S-5920, FR-167653, ONO-1714, ONO-5046 (sivelestat), GW-273629, RWJ-67657, GR-270773, NOX-100, GR-270773, NOX-100, INO-1001 (cytopathy suppressive drug) and the like.
(53) Others
   hydroxycam, diaserine, megestrol acetate, nicerogolin, prostaglandins and the like.

When compound A and other drug are combined, the following effects are afforded.
(1) The doses thereof can be reduced as compared to a single administration of each of compound A and a concomitant drug.
(2) A synergistic treatment effect can be obtained.
(3) A broad treatment effect can be provided on various diseases developed by diseases such as bacterial infection and the like.
(4) The side effects of compound A can be reduced.
   With regard to the combined use, the timing of the administration of compound A and a concomitant drug is not limited. Compound A or a pharmaceutical composition thereof (e.g., the below-mentioned pharmaceutical composition etc., preferably below-mentioned emulsion composition A etc.) and a concomitant drug or a pharmaceutical composition thereof may be simultaneously administered to the administration subject, or may be administered in a staggered manner. The dose of the concomitant drug follows a clinical dose and can be appropriately determined depending on the administration subject, administration route, disease, combination and the like.
   The mode of combined administration is not particularly limited, and compound A and a concomitant drug only need to be combined on administration. Examples of such administration mode include the following:

(1) administration of a single preparation obtained by simultaneously processing compound A or a pharmaceutical composition and the concomitant drug or a pharmaceutical composition, (2) simultaneous administration of two kinds of preparations of compound A or a pharmaceutical composition and the concomitant drug or a pharmaceutical composition, which have been separately produced, by the same administration route, (3) administration of two kinds of preparations of compound A or a pharmaceutical composition and the concomitant drug or a pharmaceutical composition, which have been separately produced, by the same administration route in a staggered manner, (4) simultaneous administration of two kinds of preparations of compound A or a pharmaceutical composition and the concomitant drug or a pharmaceutical composition, which have been separately produced, by different administration routes, (5) administration of two kinds of preparations of compound A or a pharmaceutical composition and the concomitant drug or a pharmaceutical composition, which have been separately produced, by different administration routes in a staggered manner (e.g., administration in the order of compound A or a pharmaceutical composition and the concomitant drug or a pharmaceutical composition, or in the reverse order) and the like.

The mixing ratio of compound A and a concomitant drug in the combination drug of the present invention can be appropriately determined according to the subject of administration, administration route, disease and the like.
For example, the content of compound A of the present invention in the combination agent of the present invention differs depending on the form of a preparation, and is usually from about 0.01 to 99.8% by weight, preferably from about 0.1 to 50% by weight, further preferably from about 0.5 to 20% by weight, based on total of the preparation.
The content of the concomitant drug in the combination agent of the present invention differs depending on the form of a preparation, and is usually from about 0.01 to 99.8% by weight, preferably from about 0.1 to 50% by weight, further preferably from about 0.5 to 20% by weight, based on total of the preparation.
The content of additives such as carrier in the combination agent of the present invention differs depending on the form of a preparation, and is usually from about 1 to 99.98% by weight, preferably from about 10 to 90% by weight, based on total of the preparation.
When compound A and the concomitant drug are independently prepared, the contents thereof may be the same as those mentioned above.

When compound A is administered to a human, it can be safely administered orally or parenterally as it is or in a mixture with an appropriate pharmacologically acceptable carrier, excipient and diluent, in a pharmaceutical composition such as an oral formulation (e.g., powder, granule, tablet, capsule etc.), a parenteral formulation (e.g., injection, external formulation (e.g., nasal formulation, percutaneous formulation etc.) and suppository (e.g., rectal suppository and vaginal suppository etc.).
Any of these formulations may be produced by any method known per se which is employed ordinarily for producing a pharmaceutical formulation. The amount of compound A to be incorporated into a formulation may vary depending on the dosage forms, and is preferably about 10 to 95% by weight in an oral formulation described above and about 0.001 to about 95% by weight in a parenteral formulation described above.

For example, compound A can be prepared into an aqueous injection together with a solubilizer (e.g., β-cyclodextrins etc.), a dispersant (e.g., Tween 80 (manufactured by ATLASPOWDER USA), HCO 60 (manufactured by NIKKO CHEMICALS), carboxymethylcellulose, sodium arginate etc.), a preservative (e.g., methyl paraben, propyl paraben, benzyl alcohol chlorobutanol etc.), an isotonic agent (e.g., sodium chloride, glycerine, sorbitol, glucose etc.) and the like according to a conventional method, or into an oil-based injection by appropriately dissolving, suspending or emulsifying using a vegetable oil (e.g., olive oil, sesame oil, peanut oil, cottonseed oil, corn oil etc.) and propylene glycol and the like.
An oral formulation can be produced by, for example, compressing compound A together with an excipient (e.g., lactose, sucrose, starch etc.), a disintegrant (e.g., starch, calcium carbonate etc.), a binder (e.g., starch, gum arabic, carboxymethyl cellulose, polyvinyl pyrrolidone, hydroxypropyl cellulose etc.), a lubricant (e.g., talc, magnesium stearate, polyethylene glycol 6000 etc.), and the like, followed by, where necessary, a coating process known per se for the purpose of masking a taste, forming an enteric coat, or achieving a sustained release. For such coating agent, for example, hydroxypropylmethyl cellulose, ethyl cellulose, hydroxymethyl cellulose, hydroxypropyl cellulose, polyoxyethylene glycol, Tween 80, Pluronic F68, cellulose acetate phthalate, hydroxypropylmethyl cellulose phthalate, hydroxymethyl cellulose acetate succinate, Eudragit (manufactured by ROHM, Germany, a copolymer of methacrylic acid and acrylic acid), a dye (e.g., titanium oxide, colcothar etc.) and the like may appropriately be used.

Compound A can also be employed as an external formulation in the form of a solid or semi-solid or a liquid.
For example, a solid external formulation may be compound A as it is or can be produced by mixing compound A with an excipient (e.g., glycol, mannitol, starch, crystalline cellulose etc.), a thickening agent (e.g., natural gums, cellulose derivatives, acrylic acid polymers etc.) which is then converted into a powder composition. A semi-solid external formulation may be produced by a standard method and preferably used in the form of an aqueous or oil-based gel or ointment. A liquid external formulation may be produced by a method employed for producing an injection formulation or an analogous method in the form of an oil-based or aqueous suspension.
The solid, semi-solid or liquid external formulation may be supplemented also with a pH modifier (e.g., carbonic acid, phosphoric acid, citric acid, hydrochloric acid, sodium hydroxide etc.), an antiseptic (e.g., p-oxybenzoates, chlorobutanol, benzalkonium chloride etc.) and the like, as appropriate. Typically, an ointment usually containing about 0.1 to 100 mg of compound A per 1 g a vaseline or a lanolin etc. as a formulation base, can be used.
Compound A may be also formulated as an oil or aqueous, solid or semi-solid or liquid suppository. As an oil base in preparing suppository, for example, a high fatty acid glyceride (e.g., cocoa butter, WITEPSOL (manufactured by DYNAMIT NOBEL) etc.), a middle fatty acid (e.g., MYGLYOL (manufactured by DYNAMIT NOBEL) etc.), a vegetable oil (e.g., sesame oil, soybean oil, cottonseed oil etc.) and the like are used as appropriate. An aqueous base may be, for example, polyethylene glycol or propylene glycol, and an aqueous gel base may be, for example, a natural gums, a cellulose derivative, a vinyl polymer, an acrylic polymer and the like.

In the present invention, compound A (particularly, compound 72 (ethyl (6R)-6-[(2-chloro-4-fluoroanilino)sulfonyl]-1-cyclohexene-1-carboxylate) of Reference Example B66 or a salt thereof or a prodrug thereof) is preferably used in the form of an emulsion composition containing the compound and a buffer adjusted to pH about 3.7 to about 5.5 (preferably about 3.7 to about 5.0, more preferably about 4.0 to about 5.0) (hereinafter the composition is abbreviated as emulsion composition A).
According to emulsion composition A, compound A can be effectively used as a component of a composition comprising an emulsifier and the like.
Compound A may be in a liquid form or a solid form in an oil phase, and emulsion composition A can be generally formed as an oil-in-water type (O/W type) or S/O/W type emulsion composition, preferably oil-in-water type (O/W type).
Emulsion composition A can be produced by, for example, using components such as buffer, oil component, emulsifier, water and a mixture thereof and the like. Specific example include a composition comprising dispersion phase particles comprising an oil component, an emulsifier, and compound A,
and water containing buffer wherein dispersion phase particles are dispersed. The dispersion phase particles mean a dispersion phase wherein one of two immiscible liquids is present as fine particles in the other.
Here, compound A (particularly, ethyl (6R)-6-[(2-chloro-4-fluoroanilino)sulfonyl]-1-cyclohexene-1-carboxylate or a salt thereof or a prodrug thereof) is a compound generally stable in the acidic range.
The compound stable in the acidic range in the present specification comprehensively refers to a group of compounds which easily show decomposition of compound, development of analogs and the like in the neutral or basic range exceeding pH about 6, but show less decomposition of compound, development of analogs and the like, and are highly stable in the acidic range (not more than pH about 6). More specifically, the compound stable in the acidic range refers to a compound which shows less decomposition of compound and less development of analogs in the acidic range when the compound in the acidic range and the compound in the neutral or basic range are preserved under the same conditions (temperature, humidity, period etc.) and the quality of the compounds are examined.
As the oil component, any pharmaceutically acceptable fats and oils generally used for the preparation of fat emulsions in the pharmaceutical technical field can be used. Examples of fats and oils include vegetable oil, partially hydrogenated vegetable oil, fats and oils obtained by transesterification reaction (single acid group glyceride (simple glyceride) or mixed acid group glyceride (mixed glyceride)), and middle chain fatty acid glycerol ester and the like, preferably vegetable oil and the like.
The aforementioned fats and oils include fatty acid glycerol ester having a carbon number of about 6 to 30 (preferably about 6 to 22). Examples of the aforementioned fatty acid include saturated fatty acid such as caproic acid, caprylic acid, capric acid, lauric acid, myristic acid, palmitic acid, stearic acid, behenic acid and the like, unsaturated fatty acid such as palmitoleic acid, oleic acid, linoleic acid, arachidonic acid, eicosapentanoic acid, docosahexaenoic acid and the like.
Among vegetable oils, a preferable oil component contains, for example, vegetable oil such as soybean oil, cottonseed oil, rape seed oil, peanut oil, safflower oil, sesame oil, rice bran oil, corn germ oil, sunflower oil, poppy oil, olive oil and the like, and the like. Among these vegetable oils, soybean oil and the like are preferably used.
As fats and oils, a middle chain fatty acid triglyceride having a carbon number of about 6 to 14 (preferably about 8 to 12) can also be used. Preferable middle chain fatty acid glycerol ester includes, for example, caprylic/capric triglycerides such as "miglyo1810", "miglyol 812" (both manufactured by Huls, available from Mitsuba Trading Co., Ltd.) and the like, caprylic acid triglycerides (glycerol tricaprylic acid ester) such as "Panacete 800" (manufactured by NOF Corporation) and the like, and the like.
The amount of the oil component in emulsion composition A to be used is, for example, about 1 to about 30 wt%, preferably about 2 to about 25 wt%, more preferably about 2.5 to about 22.5 wt%, of the whole composition.

As the aforementioned emulsifier, any pharmaceutically acceptable emulsifier can be used. Particularly, pharmaceutically acceptable phospholipids and non-ionic surfactants, particularly phospholipids are preferable. The emulsifier can be used alone or as a mixture of two or more kinds thereof.
Phospholipid includes, for example, naturally occurring phospholipids (e.g., egg-yolk lecithin, soybean lecithin etc.), hydrogenated products thereof, or synthetically obtained phospholipids (e.g., phosphatidylcholine, phosphatidylethanolamines, phosphatidic acid, phosphatidylserine, phosphatidylinositol, phosphatidylglycerol etc.) and the like. Among these phospholipids, egg-yolk lecithin, soybean lecithin, phosphatidyl choline derived from egg-yolk and soybean, and phosphatidyl glycerol are preferable, egg-yolk lecithin and phosphatidylglycerol (of these, the below-mentioned dimyristoylphosphatidylglycerol is preferable) are particularly preferable. A particularly preferable phospholipid is lecithin. Among synthetic phospholipids, anionic phospholipid is preferable. As the anionic synthetic phospholipid, anionic synthetic phospholipids such as dimyristoylphosphatidylglycerol, dipalmitoylphosphatidylglycerol,
distearoylphosphatidylglycerol, dioleoylphosphatidylglycerol, oleoylpalmitoylphosphatidylglycerol, dioctanoylphosphatidic acid, didecanoylphosphatidic acid, dilauroylphosphatidic acid, dimyristoylphosphatidic acid, dipalmitoylphosphatidic acid, diheptadecanoylphosphatidic acid, distearoylphosphatidic acid, dioleoylphosphatidic acid, arachidonylstearoylphosphatidic acid, dipalmitoylphosphatidylserine, dioleoylphosphatidylserine, dimyristoylphosphatidylinositol, dipalmitoylphosphatidylinositol,
distearoylphosphatidylinositol, dioleoylphosphatidylinositol, dimyristoylphosphatidylserine, distearoylphosphatidylserine and the like are specifically used, and dimyristoylphosphatidylglycerol is particularly preferable.
These anionic synthetic phospholipids can be chemically synthesized by a method known per se, or can also be obtained by purification.
As the non-ionic surfactant, a polymer surfactant having a molecular weight of about 800 to 20000, for example, polyoxyethylene-polyoxypropylene copolymer, polyoxyethylene alkyl ether, polyoxyethylenealkylarylether, hydrogenated castor oil polyoxyethylene derivative, sorbitan polyoxyethylene derivative, polyoxyethylene sorbitol derivative, polyoxyethylene alkyl ether sulfate and the like can be mentioned.
The emulsifiers of phospholipid and non-ionic surfactants can be used alone or as a mixture of two or more kinds thereof. Alternatively, commercially available phospholipids may be used.
The total amount of the emulsifier in emulsion composition A to be used is generally about 0.1 to about 10%(W/V), preferably about 0.2 to about 7%(W/V), more preferably about 0.5 to about 5%(W/V), relative to the whole composition. The anionic synthetic phospholipid is in a proportion of about 0.0001 to about 5%(W/V) relative to the whole composition.
In emulsion composition A, the proportion of the emulsifier relative to the oil component is, for example, about 0.1 to about 150 wt%, preferably about 0.5 to about 125 wt%, more preferably about 1 to about 100 wt%. The emulsifier is often used in a proportion of generally about 1 to about 15 wt%, particularly about 1 to about 10 wt%, relative to the oil component.

Water to be used emulsion composition A of the present invention is not particularly limited as long as it is acceptable as a pharmaceutical product and, for example, purified water, water for injection (distilled water for injection) and the like can be mentioned. For production of a product other than pharmaceutical products, water is not particularly limited.
The amount of water in emulsion composition A to be used is generally about 40 to about 99%(W/V), preferably about 55 to about 98.8%(W/V), relative to the whole composition.
Emulsion composition A can be prepared by mixing a dispersion phase component comprising compound A (main drug), an oil component and an emulsifier with water and emulsifying the mixture and a buffer may be added to an aqueous phase before emulsification, or may be added to the emulsion composition after emulsification. Where necessary, additives such as a stabilizer to improve the stability of the aforementioned main drug, an isotonicity agent to control the osmotic pressure, an emulsion aid to improve the emulsifying power, an emulsion stabilizer to improve stability of emulsifier and the like may be added.
Examples of the stabilizer include antioxidants (e.g., ascorbic acid, tocopherol, sorbic acid, retinol etc.), chelating agents (e.g., edetic acid, citric acid, tartaric acid etc., and salts thereof) and the like. The amount of the stabilizer to be used is generally about 0.00001 to about 10%(W/V), preferably about 0.0001 to about 5%(W/V), relative to the whole emulsion composition A.
An isotonicity agent contains, for example, glycerol, sugar alcohol, monosaccharides, disaccharides, amino acid, dextran, albumin and the like. These isotonicity agents can be used in a mixture of one or more kinds thereof.
Examples of the emulsion aid include fatty acid having a carbon number of about 6 to 30, salts of such fatty acid, monoglycerides of the aforementioned fatty acid and the like. The aforementioned fatty acid includes, for example, caproic acid, capric acid, caprylic acid, lauric acid, myristic acid, palmitic acid, stearic acid, behenic acid, palmitoleic acid, oleic acid, linoleic acid, arachidonic acid, eicosapentanoic acid, docosahexaenoic acid and the like, and salts of fatty acid include, for example, alkali metal salts such as sodium salt, potassium salt and the like, calcium salt and the like.
As the emulsion stabilizer, for example, cholesterol, cholesteryl ester, tocopherol, albumin, fatty acid amide derivative, polysaccharides, fatty acid ester derivative of polysaccharides and the like can be used.

While the concentration of compound A of emulsion composition A varies depending on the pharmacological activity or blood kinetics of the compound, it is generally about 0.001 to about 5%(W/V), preferably about 0.01 to about 2%(W/V), more preferably about 0.1 to about 1.5%(W/V). In addition, the content of compound A in emulsion composition A can also be set to about 1 to about 5000 mg, preferably about 10 to about 2000 mg, more preferably about 100 to about 1500 mg, in 100 ml of the composition. In addition, the content of compound A can also be adjusted to about 0.001 to about 95 wt%, preferably about 0.01 to about 30 wt%, more preferably about 0.1 to about 3 wt%, relative to the whole composition.
The proportion (wt%) of compound A relative to the dispersion phase consisting of an oil component and an emulsifier is generally about 0.0047 to about 24%, preferably about 0.047 to about 9.4%.
Emulsion composition A is adjusted to pH about 3.7 to about 5.5, preferably about 3.7 to about 5.0, more preferably about 4.0 to about 5.0.
As a pH adjuster, for example, phosphoric acid, carbonic acid, citric acid, hydrochloric acid, sodium hydroxide and the like are used and hydrochloric acid, sodium hydroxide and the like are particularly preferable.
As the aforementioned buffer, any pharmaceutically acceptable buffer can be used. For example, a buffer containing acetic acid, glacial acetic acid, lactic acid, citric acid, phosphoric acid, carbonic acid, histidine, glycine, barbital, phthalic acid, adipic acid, ascorbic acid, maleic acid, succinic acid, tartaric acid, glutamic acid, benzoic acid, aspartic acid and a salt thereof (e.g.,
potassium, sodium etc.), specifically sodium acetate, sodium lactate, sodium citrate, disodium hydrogen phosphate, sodium dihydrogen phosphate, sodium carbonate, sodium hydrogen carbonate, hydrochloric acid, sodium hydroxide and the like as a constituent component is preferable. Moreover, respective buffers may be used in combination. Particularly, one or more buffers selected from acetate buffer, glacial acetate buffer, lactate buffer, citrate buffer and phosphate buffer are preferable.
As the buffer, a buffer containing (i) a combination of acetic acid or glacial acetic acid and sodium acetate (acetate buffer or glacial acetate buffer), or (ii) a combination of lactic acid and sodium lactate (lactate buffer) as a component, and the like are preferable. Particularly, a buffer containing acetic acid and sodium acetate as components is preferably used.
In the present specification, the "acetic acid" includes glacial acetic acid, and the "acetate buffer" includes glacial acetate buffer.
The concentration of the buffer is preferably not more than about 100 mM, specifically about 0.1mM to about 100 mM, preferably about 0.2 to about 50 mM, more preferably about 5 mM to about 40 mM.
The pH adjuster is an acidic or alkaline compound to be added to adjust the pH of a solution to a desired pH.
The amount of the pH adjuster to be generally added to an injection is trace. The amount of sodium hydroxide as a pH adjuster in a fatty emulsion commercially available in Japan is often not more than about 0.5 mM. While the pH can be adjusted to a desired pH during preparing the solution, the pH of the solution easily changes by the addition of an acid or alkali, and maintenance of pH is difficult.
The buffer is a compound having an action to reduce changes in pH on addition of acid or alkali, namely, a bufferizing action. In many cases, it is a mixed solution of a weak acid and a salt thereof, or a weak base and a salt thereof.
By addition of a buffer, emulsion composition A is not influenced by the development of free fatty acid, and can maintain a constant pH of an emulsion composition during high-pressure vapor sterilization and long-term preservation.
The amount of the buffer to be used for general injections is high with the aim of bufferizing action. For example, the amount of an acetate buffer in a solution injection commercially available in Japan is about 0.2 mM to about 100 mM.

Emulsion composition A is preferably used, for example, as a composition for injection.
Emulsion composition A can be basically produced by a known method or a method according thereto. Particularly, while a conventionally used emulsion technique can be utilized for the emulsion, compound A is preferably dissolved or dispersed in advance in an oil component. To be precise, a composition containing an O/W or S/O/W emulsion can be produced by dispersing a mixture of dispersion phase (1) containing an oil component and an emulsifier, and compound A (2) in water. The buffer may be added to an aqueous phase before emulsification, or added to an emulsion after emulsification during production.
The more preferable method includes, for example, a method of preparing an oil-in-water composition comprising homogenizing an unhomogeneous mixture of a mixture of the main drug, an oil component, an emulsifier and, where necessary, an additive such as isotonicity agent and the like, and water containing a buffer using an emulsifying machine to give a crude emulsion, adding water as necessary, further homogenizing the emulsion using the above-mentioned emulsifying machine, and removing large particles by a filtration means such as a filter and the like. The aforementioned mixture is often warmed to a temperature of, for example, about 30 to about 90°C, preferably about 40 to about 80°C, to dissolve or disperse the main drug. As the emulsifying machine to emulsify an unhomogeneous mixture of the aforementioned mixture and water, conventionally used apparatuses, for example, homogenizers such as a pressurization injection type homogenizer, ultrasonication homogenizer and the like, homomixers such as high-speed rotation type mixer and the like, and the like can be used. To remove large particles having a particle size of not less than about 5 µm, homogenized emulsion is often subjected to a filtration means such as a filter and the like.
Specifically, for example, emulsion composition A can be preferably produced according to the above-mentioned method and using soybean oil as an oil component, phospholipid as an emulsifier, water (particularly, purified water), glycerol used as an isotonicity agent etc. and the like. However, the method is not limited thereto.
In emulsion composition A, the particle size distribution of a dispersion phase, wherein compound A is dissolved, is,
for example, often about 0.01 to about 7 µm, preferably about 0.02 to about 5 µm. From the aspects of the stability of the emulsion and distribution in the body after administration, the mean particle size of the dispersion phase particles, wherein compound A is dissolved, is preferably about 0.025 to about 0.7 µm, more preferably about 0.05 to about 0.4 µm.
The mean particle size used in the present specification means a mean particle size based on the volume distribution and measured by a laser diffraction particle size distribution measurement apparatus, with the laser diffraction·scattering method as a measurement principle.
Pyrogen can be removed from emulsion composition A by a method known *per se*.
Where necessary, after nitrogen gas substitution, emulsion composition A is sterilized and tightly sealed.
Since pH of emulsion composition A is adjusted to about 3.7 to about 5.5 (preferably about 3.7 to about 5.0, more preferably about 4.0 to about 5.0) by adding a buffer, pH of the composition and mean particle size of the dispersion phase particles hardly change even after sterilization by an autoclave etc. or after long-term preservation, and the composition is stable. Therefore, the stability of compound A and emulsion composition A is superior. Moreover, emulsion composition A is free of a visibly observed free oil drop even after sterilization by an autoclave etc. or after long-term preservation, and therefore, phase separation of dispersion phase particles and water wherein the dispersion phase particles are dispersed does not occur, and the composition is stable.
Furthermore, emulsion composition A can increase the concentration of compound A, and control the particle size of the dispersion phase particles. Thus, it can enhance retentivity in blood, blood vessel permeability and transitivity into inflammation site. Therefore, in vivo kinetics or distribution in the body of compound A can be improved and targeting becomes possible, as a result of which administration of an effective drug with suppressed side effects becomes possible. Accordingly, emulsion composition A is particularly useful for the treatment of a target disease by an intravenous administration.

While the dose of compound A varies depending on the age, body weight, symptom, dosage form, administration method, dosing period and the like, it is, for example, generally about 0.01 to about 1000 mg/kg, preferably about 0.01 to about 100 mg/kg, more preferably about 0.1 to about 100 mg/kg, particularly about 0.1 to about 50 mg/kg, especially about 1.5 to about 30 mg/kg, a day in the amount of compound A for a patient (adult, body weight about 60 kg) before or after coronary-artery bypass surgery, which is orally or parenterally administered one to several portions a day. As mentioned above, since the dose varies depending on various conditions, an amount less than the aforementioned dose may be sufficient or administration of an excess amount may be necessary.
Particularly, when ethyl (6R)-6-[(2-chloro-4-fluoroanilino)sulfonyl]-1-cyclohexene-1-carboxylate (compound 72 of Reference Example B66) or a salt thereof or a prodrug thereof is used as compound A, the below-mentioned single administration, multiple administration, continuous drip or a combination of single administration and continuous drip is preferably employed.
While the dose of a combination drug in the present invention varies depending on the kind of compound, the age, body weight, symptom, dosage form, administration method, dosing period and the like, it is, for example, generally about 0.01 to about 1000 mg/kg, preferably about 0.01 to about 100 mg/kg, more preferably about 0.1 to about 100 mg/kg, particularly about 0.1 to about 50 mg/kg, especially about 1.5 to about 30 mg/kg, a day in the amount of each of compound A and a concomitant drug for a patient (adult, body weight about 60 kg) before or after coronary-artery bypass surgery, which is intravenously administered one to several portions a day.
Of course, as mentioned above, since the dose varies depending on various conditions, an amount less than the aforementioned dose may be sufficient or administration of an excess amount may be necessary.
The concomitant drug may be contained in any amount as long as a side effect does not pose a problem. While the daily dose of the combination drug may vary depending on the disease state, the age, sex, body weight and difference in sensitivity of the administration object, timing and interval of administration, characteristics, dispensing and kind of the pharmaceutical preparation, the kind of active ingredient and the like and is not particularly limited, the amount of the drug is generally about 0.001 to 2000 mg, preferably about 0.01 to 500 mg, more preferably about 0.1 to 100 mg, per 1 kg body weight of mammal by oral administration, which is generally administered in 1 to 4 portions during a day.
In administration of a combination drug of the present invention, compound A may be administered after administration of the concomitant drug or the concomitant drug may be administered after administration of compound A, though they may be administered simultaneously. When administered at a time interval, the interval differs depending on the effective ingredient to be administered, drug form and administration method, and for example, when the concomitant drug is administered first, a method in which compound A is administered within time range of from 1 min to 3 days, preferably from 10 min to 1 day, more preferably from 15 min to 1 hr after administration of the concomitant drug is exemplified. When compound A is administered first, a method in which the concomitant drug is administered within time range of from 1 min to 1 day, preferably from 10 min to 6 hrs, more preferably from 15 min to 1 hr after administration of compound A is exemplified.

Furthermore, a preferable method of administering ethyl (6R)-6-[(2-chloro-4-fluoroanilino)sulfonyl]-1-cyclohexene-1-carboxylate (compound 72 of Reference Example B66) or a salt thereof or a prodrug thereof (hereinafter to be comprehensively abbreviated as compound B) using compound B for the prophylaxis or treatment of cardiac diseases, autoimmune diseases, central nervous system diseases, inflammatory diseases, sepsis, severe sepsis, septic shock and complications after coronary-artery bypass surgery is as follows.
As a pharmaceutical composition using compound B, the aforementioned emulsion composition A is preferable.

1. Single administration
   Compound B is administered at a dose of about 0.2 mg/kg body weight to about 2.4 mg/kg body weight for about 15 min to about 240 min, preferably about 15 min to about 120 min to a mammal (e.g., cat, bovine, dog, horse, goat, monkey, human etc.).
   Preferably, compound B is administered to a mammal
   (i) at a dose of about 0.2 mg/kg body weight to about 1.2 mg/kg body weight for about 15 min to about 240 min, preferably about 15 min to about 60 min, particularly preferably about 30 min, or
   (ii) at a dose of about 0.2 mg/kg body weight to about 1.8 mg/kg body weight (preferably, about 1.2 mg/kg body weight to about 1.8 mg/kg body weight) for about 60 min to 240 min, preferably about 60 min to 120 min, particularly preferably about 90 min.
      Particularly preferably, compound B is administered to a mammal at a dose of about 0.2 mg/kg body weight to about 1.8 mg/kg body weight, preferably about 0.6 mg/kg body weight to about 1.8 mg/kg body weight, more preferably about 0.9 mg/kg body weight to about 1.2 mg/kg body weight, particularly preferably about 1.2 mg/kg body weight, for about 30 min to 90 min.

2. Multiple administrations
   Compound B is administered at a dose of about 0.2 mg/kg body weight to about 2.4 mg/kg body weight, preferably about 0.2 mg/kg body weight to about 1.8 mg/kg body weight, more preferably about 0.6 mg/kg body weight to about 1.2 mg/kg body weight, per administration for about 15 min to about 240 min, preferably about 15 min to about 120 min, more preferably about 15 min to about 90 min per administration, 1 to 6 times a day, preferably 1 to 4 times a day, more preferably 2 to 4 times a day, for 1 day to 1 month, preferably 1 to 7 days, more preferably 4 to 6 days, particularly preferably 5 days.
   More preferably, compound B is administered to a mammal at a dose of about 0.6 mg/kg body weight to about 1.2 mg/kg body weight per administration, for about 15 min to about 240 min, preferably about 15 min to about 120 min, more preferably about 15 min to about 90 min per administration, 2 to 4 times a day for 1 day to 1 month, preferably 1 to 7 days, more preferably 4 to 6 days, particularly preferably 5 days.
   Further preferably,
   (1) compound B is administered to a mammal at a dose of about 0.6 mg/kg body weight per administration for about 15 min to about 120 min, more preferably about 15 min to about 90 min per administration, twice a day for 1 to 7 days, more preferably 4 to 6 days, particularly preferably 5 days,
   (2) compound B is administered to a mammal at a dose of about 0.9 mg/kg body weight per administration for about 15 min to about 120 min, more preferably about 15 min to about 90 min per administration, 2 to 4 times a day for 1 to 7 days, more preferably 4 to 6 days, particularly preferably 5 days, or
   (3) compound B is administered to a mammal at a dose of about 1.2 mg/kg body weight per administration for about 15 min to about 120 min, more preferably about 15 min to about 90 min per administration, 2 to 4 times a day for 1 to 7 days, more preferably 4 to 6 days, particularly preferably 5 days.

3. Continuous drip
   Compound B is administered to a mammal by continuous drip at a dose of about 0.01 mg/kg body weight/hr to about 0.3 mg/kg body weight/hr, preferably about 0.05 mg/kg body weight/hr to about 0.15 mg/kg body weight/hr, for about 1 hr to about 1 month, preferably about 1 hr to about 168 hr, more preferably about 1 hr to about 96 hr.
   Particularly preferably, Compound B is administered to a mammal by continuous drip at a dose of about 0.01 mg/kg body weight/hr to about 0.3 mg/kg body weight/hr, for about 1 hr to about 96 hr, preferably about 54 hr to about 96 hr.

4. Combination of single administration and continuous drip
   After the above-mentioned single administration, the above-mentioned continuous drip is performed.
   Specifically, compound B is administered to a mammal (e.g., cat, bovine, dog, horse, goat, monkey, human etc.) at a dose of about 0.2 mg/kg body weight to about 2.4 mg/kg body weight for about 15 min to about 240 min, preferably about 15 min to about 120 min (by single administration), and then, compound B is administered by continuous drip at a dose of about 0.01 mg/kg body weight/hr to about 0.3 mg/kg body weight/hr, preferably about 0.05 mg/kg body weight/hr to about 0.15 mg/kg body weight/hr, for about 1 hr to about 1 month, preferably about 1 hr to about 168 hr, more preferably about 1 hr to about 96 hr.
   More preferably, compound B is administered to a mammal
   (i) at a dose of about 0.2 mg/kg body weight to about 1.2 mg/kg body weight for about 15 min to 240 min, preferably about 15 min to 60 min, particularly preferably about 30 min, or
   (ii) at a dose of about 0.2 mg/kg body weight to about 1.8 mg/kg body weight (preferably about 1.2 mg/kg body weight to about 1.8 mg/kg body weight) for about 60 min to 240 min, preferably about 60 min to 120 min, particularly preferably about 90 min (by single administration), and then, compound B is continuously administered at a dose of about 0.01 mg/kg body weight/hr to about 0.3 mg/kg body weight/hr for about 1 hr to about 96 hr, preferably about 54 hr to about 96 hr.
      Particularly preferably, compound B is administered to a mammal at a dose of about 0.2 mg/kg body weight to about 1.8 mg/kg body weight, preferably about 0.6 mg/kg body weight to about 1.8 mg/kg body weight, more preferably about 0.9 mg/kg body weight to about 1.2 mg/kg body weight, particularly preferably about 1.2 mg/kg body weight, for about 30 min to 90 min (by single administration), and then, compound B is continuously administered at a dose of about 0.01 mg/kg body weight/hr to about 0.3 mg/kg body weight/hr for about 1 hr to about 96 hr, preferably about 54 hr to about 96 hr.

Of the above-mentioned administration methods, the combination of single administration and continuous drip is preferable. In other words, the single administration at the above-mentioned specific dose at specific administration time can improve initial effect, and the continuous drip at the above-mentioned specific dose at specific administration time can afford sustained effect and decrease expression of side effects.
When compound B is administered, it can be prepared in the same manner as in the aforementioned preparation containing compound A into a formulation suitable for the above-mentioned administration method.
In addition, compound B can be used in combination with other drugs as in the above.

### Examples

The present invention is further described in the following by referring to Reference Examples, Examples and Experimental Examples, which are not intended to restrict the invention.
The ¹H-NMR spectrum was determined by a VARIAN GEMINI 200 (200 MHz) spectrometer using tetramethylsilane as an internal standard and represented as the entire δ values in ppm. The number in a bracket when a solvent mixture was employed is the volume ratio of each solvent, wherein % means % by weight unless otherwise specified. The ratio of the solvents in silica gel chromatography shows the volume ratio of the solvents to be admixed.
A high polar diastereomer means a diastereomer having a smaller Rf value, and a low polar diastereomer means a diastereomer having a larger Rf value, when determined by normal phase thin layer chromatography under the same conditions (e.g., using ethyl acetate/hexane etc. as an solvent).
The melting point was measured using melting point measuring apparatus (manufactured by Yanagimoto Mfg. Co., Ltd). The data of the powder X-ray diffraction was measured using RINT2500 (Rigaku Industrial Corporation) using Cu-Kαᵢ ray as a ray source.
Each symbol in examples means the following:
s: singlet d: doublet: t: triplet q: quartet
dd: double doublet tt: triple triplet m: multiplet
br: broad J: coupling constant
The following Reference Examples A can be produced according to Reference Examples of WO99/46242, Reference Example B can be produced according to Examples of WO99/46242, Reference Examples C can be produced according to Reference Examples of WO01/10826, and Reference Examples D can be produced according to Examples of WO01/10826.

### [Reference Examples A]

Reference Example A1 ethyl 2-sulfo-1-cyclohexene-1-carboxylate
Reference Example A2 ethyl 2-chlorosulfonyl-l-cyclohexene-l-carboxylate
Reference Example A3 ethyl 2-chlorosulfonyl-l-cyclopentene-l-carboxylate
Reference Example A4 ethyl 2-chlorosulfonyl-1-cycloheptene-1-carboxylate
Reference Example A5 sodium 6-[N-(4-chloro-2-fluorophenyl)-sulfamoyl]-1-cyclohexene-1-carboxylate
Reference Example A6 1-(3-fluoro-4-nitrophenyl)-1H-1,2,4-triazole
Reference Example A7 1-(4-amino-3-fluorophenyl)-1H-1,2,4-triazole
Reference Example A8 methyl 4-(benzyloxycarbonylamino)-3-chlorobenzoate
Reference Example A9 4-(benzyloxycarbonylamino)-3-chlorobenzoic acid
Reference Example A10 tert-butyl N-(4-benzyloxycarbonylamino-3-chlorobenzoyl)glycinate
Reference Example A11 tert-butyl N-(4-amino-3-chlorobenzoyl)-glycinate
Reference Example A12 6-[N-(2,4-difluorophenyl)sulfamoyl]-1-cyclohexene-1-carboxylic acid
Reference Example A13 ethyl 2-mercapto-5-phenyl-1-cyclohexene-1-carboxylate
Reference Example A14 ethyl 2-chlorosulfonyl-5-phenyl-1-cyclohexene-1-carboxylate
Reference Example A15 ethyl 5-tert-butyl-2-mercapto-1-cyclohexene-1-carboxylate
Reference Example A16 ethyl 5-tert-butyl-2-chlorosulfonyl-1-cyclohexene-1-carboxylate
Reference Example A17 ethyl 5,5-dimethyl-2-mercapto-1-cyclohexene-1-carboxylate
Reference Example A18 ethyl 2-chlorosulfonyl-5,5-dimethyl-1-cyclohexene-1-carboxylate

### [Reference Examples B]

Reference Example B1 ethyl 6-[N-(4-chloro-2-fluorophenyl)-sulfamoyl]-1-cyclohexene-1-carboxylate (compound 1)
Reference Example B2 ethyl 6-[N-(4-chloro-2-fluorophenyl)-N-methylsulfamoyl]-1-cyclohexene-1-carboxylate (compound 2)
Reference Example B3 ethyl 6-[N-(2,4-difluorophenyl)sulfamoyl]-1-cyclohexene-1-carboxylate (compound 3)
Reference Example B4 ethyl 6-[N-(2,6-diisopropylphenyl)-sulfamoyl]-1-cyclohexene-1-carboxylate (compound 4)
Reference Example B5 ethyl 6-[N-(4-nitrophenyl)sulfamoyl]-1-cyclohexene-1-carboxylate (compound 5)
Reference Example B6 ethyl 6-(N-phenylsulfamoyl)-1-cyclohexene-1-carboxylate (compound 6)
ethyl 2-(N-phenylsulfamoyl)-1-cyclohexene-1-carboxylate (compound 7)
Reference Example B7 ethyl 2-[N-(4-chloro-2-fluorophenyl)-sulfamoyl]-1-cyclohexene-1-carboxylate (compound 9)
Reference Example B8 2-(4-methoxyphenyl)-4,5,6,7-tetrahydro-1,2-benzisothiazol-3(2H)-one 1,1-dioxide (compound 67)
ethyl 2-[N-(4-methoxyphenyl)sulfamoyl]-1-cyclohexene-1-carboxylate (compound 8)
Reference Example B9 ethyl 6-[N-(2-fluorophenyl)sulfamoyl]-1-cyclohexene-1-carboxylate (compound 10)
Reference Example B10 ethyl 6-[N-(3-fluorophenyl)sulfamoyl]-1-cyclohexene-1-carboxylate (compound 11)

Reference Example B11 2-(4-fluorophenyl)-4,5,6,7-tetrahydro-1,2-benzisothiazol-3(2H)-one 1,1-dioxide (compound 68)
ethyl 6-[N-(4-fluorophenyl)sulfamoyl]-1-cyclohexene-1-carboxylate (compound 12)
ethyl 2-[N-(4-fluorophenyl)sulfamoyl]-1-cyclohexene-1-carboxylate (compound 18)
Reference Example B12 ethyl 6-[N-(2,6-difluorophenyl)-sulfamoyl]-1-cyclohexene-1-carboxylate (compound 13)
Reference Example B13 ethyl 6-[N-(2,3-difluorophenyl)-sulfamoyl]-1-cyclohexene-1-carboxylate (compound 14)
Reference Example B14 ethyl 6-[N-(2,5-difluorophenyl)-sulfamoyl]-1-cyclohexene-1-carboxylate (compound 15)
Reference Example B15 ethyl 6-[N-(3,4-difluorophenyl)-sulfamoyl]-1-cyclohexene-1-carboxylate (compound 16)
Reference Example B16 ethyl 6-[N-(3,5-difluorophenyl)-sulfamoyl]-1-cyclohexene-1-carboxylate (compound 17)
Reference Example B17 1-ethyl 6-[N-(2,4-difluorophenyl)-sulfamoyl]-1-cyclohexene-1-carboxylate (compound 19)
d-ethyl 6-[N-(2,4-difluorophenyl)sulfamoyl]-1-cyclohexene-1-carboxylate (compound 20)
Reference Example B18 ethyl 6-[N-(2-ethoxycarbonylphenyl)-sulfamoyl]-1-cyclohexene-1-carboxylate (compound 21)
Reference Example B19 methyl 6-[N-(2,4-difluorophenyl)-sulfamoyl]-1-cyclohexene-1-carboxylate (compound 22)
Reference Example B20 propyl 6-[N-(2,4-difluorophenyl)-sulfamoyl]-1-cyclohexene-1-carboxylate (compound 23)

Reference Example B21 methyl 6-[N-(4-chloro-2-fluorophenyl)-sulfamoyl]-1-cyclohexene-1-carboxylate (compound 24)
Reference Example B22 isopropyl 6-[N-(2,4-difluorophenyl)-sulfamoyl]-1-cyclohexene-1-carboxylate (compound 25)
Reference Example B23 ethyl 6-[N-(2-methoxycarbonylphenyl)-sulfamoyl]-1-cyclohexene-1-carboxylate (compound 26)
Reference Example B24 ethyl 6-[N-(2-fluoro-4-methylphenyl)-sulfamoyl]-1-cyclohexene-1-carboxylate (compound 27)
Reference Example B25 ethyl 6-[N-(2-chlorophenyl)sulfamoyl]-1-cyclohexene-1-carboxylate (compound 28)
Reference Example B26 ethyl 6-[N-(2-chloro-4-fluorophenyl)-sulfamoyl]-1-cyclohexene-1-carboxylate (compound 29)
Reference Example B27 ethyl 6-[N-(4-chlorophenyl)sulfamoyl]-1-cyclohexene-1-carboxylate (compound 30)
Reference Example B28 ethyl 6-[N-(2,3,4-trifluorophenyl)-sulfamoyl]-1-cyclohexene-1-carboxylate (compound 31)
Reference Example B29 isobutyl 6-[N-(2,4-difluorophenyl)-sulfamoyl]-1-cyclohexene-1-carboxylate (compound 32)
Reference Example B30 butyl 6-[N-(2,4-difluorophenyl)-sulfamoyl]-1-cyclohexene-1-carboxylate (compound 33)

Reference Example B31 ethyl 6-[N-(4-bromo-2-fluorophenyl)-sulfamoyl]-1-cyclohexene-1-carboxylate (compound 34)
Reference Example B32 ethyl 6-[N-(2,4-dichlorophenyl)-sulfamoyl]-1-cyclohexene-1-carboxylate (compound 35)
Reference Example B33 ethyl 6-[N-(2-acetoxyphenyl)sulfamoyl]-1-cyclohexene-1-carboxylate (compound 36)
Reference Example B34 ethyl 6-[N-(3-chlorophenyl)sulfamoyl]-1-cyclohexene-1-carboxylate (compound 37)
Reference Example B35 ethyl 6-[N-(2,3-dichlorophenyl)-sulfamoyl]-1-cyclohexene-1-carboxylate (compound 38)
Reference Example B36 ethyl 6-[N-(2-ethylphenyl)sulfamoyl]-1-cyclohexene-1-carboxylate (compound 39)
Reference Example B37 ethyl 6-[N-[4-(2H-1,2,3-triazol-2-yl)phenyl]sulfamoyl]-1-cyclohexene-1-carboxylate (compound 40)
Reference Example B38 ethyl 6-[N-(2,5-dichlorophenyl)-sulfamoyl]-1-cyclohexene-1-carboxylate (compound 41)
Reference Example B39 ethyl 6-[N-(2-trifluoromethoxyphenyl)-sulfamoyl]-1-cyclohexene-1-carboxylate (compound 42)
Reference Example B40 ethyl 6-[N-(2,4,5-trifluorophenyl)-sulfamoyl]-1-cyclohexene-1-carboxylate (compound 43)

Reference Example B41 ethyl 6-[N-[4-(2H-tetrazol-2-yl)phenyl]sulfamoyl]-1-cyclohexene-1-carboxylate (compound 44)
Reference Example B42 ethyl 6-[N-(2-chloro-4-methylphenyl)-sulfamoyl]-1-cyclohexene-1-carboxylate (compound 45)
Reference Example B43 ethyl 6-[N-(4-fluoro-2-methylphenyl)-sulfamoyl]-1-cyclohexene-1-carboxylate (compound 46)
Reference Example B44 ethyl 6-[N-(2,6-dichlorophenyl)-sulfamoyl]-1-cyclohexene-1-carboxylate (compound 47)
Reference Example B45 ethyl 6-[N-[4-(1H-tetrazol-1-yl)phenyl]sulfamoyl]-1-cyclohexene-1-carboxylate (compound 48)
Reference Example B46 ethyl 6-[N-(4-(1H-1,2,3-triazol-1-yl)phenyl]sulfamoyl]-1-cyclohexene-1-carboxylate (compound 49)
Reference Example B47 ethyl 6-[N-(2-trifluoromethylphenyl)-sulfamoyl]-1-cyclohexene-1-carboxylate (compound 50)
Reference Example B48 ethyl 6-[N-(4-methoxycarbonylphenyl)-sulfamoyl]-1-cyclohexene-1-carboxylate (compound 51)
Reference Example B49 benzyl 6-[N-(2,4-difluorophenyl)-sulfamoyl]-1-cyclohexene-1-carboxylate(compound 52)
Reference Example B50 ethyl 6-[N-[4-[2,3-bis(tert-butoxycarbonyl)guanidinomethyl]phenyl]sulfamoyl]-1-cyclohexene-1-carboxylate (compound 53)

Reference Example B51 ethyl 6-[N-(2-chloro-4-methoxycarbonylphenyl)sulfamoyl]-1-cyclohexene-1-carboxylate (compound 54)
Reference Example B52 ethyl 6-[N-(2-chloro-4-cyanophenyl)-sulfamoyl]-1-cyclohexene-1-carboxylate (compound 55)
Reference Example B53 2-hydroxyethyl 6-[N-(2,4-difluorophenyl)sulfamoyl]-1-cyclohexene-1-carboxylate (compound 56)
Reference Example B54 ethyl 6-[N-[2-fluoro-4-(1H-1,2,4-triazol-1-yl)phenyl]sulfamoyl]-1-cyclohexene-1-carboxylate (compound 57)
Reference Example B55 ethyl 2-[N-(2,4-difluorophenyl)-sulfamoyl]-1-cyclopentene-1-carboxylate (compound 66)
ethyl 5-[N-(2,4-difluorophenyl)sulfamoyl]-1-cyclopentene-1-carboxylate (compound 58)
Reference Example B56 tert-butyl [6-[N-(2,4-difluorophenyl)-sulfamoyl]-1-cyclohexen-1-yl]carbonyloxyacetate (compound 59)
Reference Example B57 [6-[N-(2,4-difluorophenyl)sulfamoyl]-1-cyclohexen-1-yl]carbonyloxyacetic acid (compound 60)
Reference Example B58 ethyl 7-[N-(2,4-difluorophenyl)-sulfamoyl]-1-cycloheptene-1-carboxylate (compound 61)
Reference Example B59 ethyl 6-[N-[2-chloro-4-(N-tert-butoxycarbonylmethylcarbamoyl)phenyl]sulfamoyl]-1-cyclohexene-1-carboxylate (compound 62)
Reference Example B60 ethyl 6-[N-[2-chloro-4-(N-ethoxycarbonylmethylcarbamoyl)phenyl]sulfamoyl]-1-cyclohexene-1-carboxylate (compound 63)

Reference Example B61 ethyl 5-[N-(2-chloro-4-fluorophenyl)-sulfamoyl]-1-cyclopentene-1-carboxylate (compound 64)
Reference Example B62 2-[4-(2,2,3,3,3-pentafluoropropoxy)-phenyl]-4,5,6,7-tetrahydro-1,2-benzisothiazol-3(2H)-one 1,1-dioxide (compound 69)
Reference Example B63 ethyl 7-[N-(2-chloro-4-fluorophenyl)-sulfamoyl]-1-cycloheptene-1-carboxylate (compound 65)
Reference Example B64 2-(2,4-difluorophenyl)-5,6,7,7a-tetrahydro-1,2-benzisothiazol-3(2H)-one 1,1-dioxide (compound 70)
Reference Example B65 ethyl 6-[N-(2-chloro-4-fluorophenyl)-sulfamoyl]-1-cyclohexene-1-carboxylate ( compound 29)
Reference Example B66 ethyl (6S)-6-[(2-chloro-4-fluoroanilino)sulfonyl]-1-cyclohexene-1-carboxylate (1-ethyl 6-[N-(2-chloro-4-fluorophenyl)sulfamoyl]-1-cyclohexene-1-carboxylate) (compound 71)
ethyl (6R)-6-[(2-chloro-4-fluoroanilino)sulfonyl]-1-cyclohexene-1-carboxylate (d-ethyl 6-[N-(2-chloro-4-fluorophenyl)sulfamoyl]-1-cyclohexene-1-carboxylate) (compound 72)
Reference Example B67 ethyl 6-[N-(2-bromo-4-fluorophenyl)-sulfamoyl]-1-cyclohexene-1-carboxylate (compound 73)
Reference Example B68 ethyl 6-[N-(4-bromo-2-chlorophenyl)-sulfamoyl]-1-cyclohexene-1-carboxylate (compound 74)
Reference Example B69 high polar diastereomer (compound 75) and low polar diastereomer (compound 76) of ethyl 6-[N-(2,4-difluorophenyl)sulfamoyl]-3-phenyl-1-cyclohexene-1-carboxylate
Reference Example B70 high polar diastereomer (compound 77) and low polar diastereomer (compound 78) of ethyl 6-[N-(2-chloro-4-fluorophenyl)sulfamoyl]-3-phenyl-1-cyclohexene-1-carboxylate

Reference Example B71 high polar diastereomer (compound 79) and low polar diastereomer (compound 80) of ethyl 6-[N-(2,4-difluorophenyl)sulfamoyl]-3-tert-butyl-1-cyclohexene-1-carboxylate
Reference Example B72 high polar diastereomer (compound 81) and low polar diastereomer (compound 82) of ethyl 6-[N-(2-chloro-4-fluorophenyl)sulfamoyl]-3-tert-butyl-1-cyclohexene-1-carboxylate
Reference Example B73 ethyl 6-[N-(2,4-difluorophenyl)-sulfamoyl]-3,3-dimethyl-1-cyclohexene-1-carboxylate (compound 83)
Reference Example B74 ethyl 6-[N-(2-chloro-4-fluorophenyl)-sulfamoyl]-3,3-dimethyl-1-cyclohexene-1-carboxylate (compound 84)
Reference Example B75 ethyl 3-bromo-6-[N-(2,4-difluorophenyl)-sulfamoyl]-1-cyclohexene-1-carboxylate (compound 85)
The chemical structures of compounds 1 to 85 are shown in Tables 1 to 12.

**[Table 1]**

| | | | | |
|---|---|---|---|---|
| 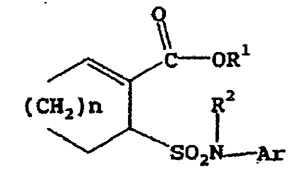 | | | | |

| Compound No. | **R¹** | **R²** | **Ar** | **n** |
|---|---|---|---|---|
| **1** | **C₂H₅** | **H** | 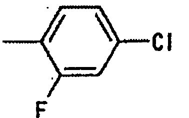 | **2** |
| **2** | **C₂H₅** | **CH₃** | 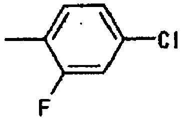 | **2** |
| **3** | **C₂H₅** | **H** | 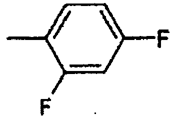 | **2** |
| **4** | **C₂H₅** | **H** | 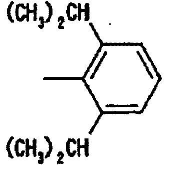 | **2** |
| **5** | **C₂H₅** | **H** | 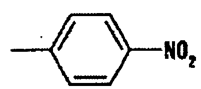 | **2** |
| **6** | **C₂H₅** | **H** | 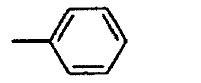 | **2** |
| **10** | **C₂R₅** | **H** | 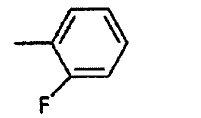 | **2** |

**[Table 2]**

| | | | | |
|---|---|---|---|---|
| **11** | **C₂H₅** | **H** | 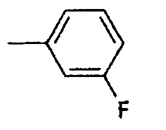 | **2** |
| **12** | **C₂H₅** | **H** | 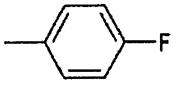 | **2** |
| **13** | **C₂H₅** | **H** | 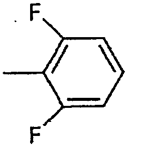 | **2** |
| **14** | **C₂H₅** | **H** | 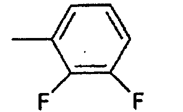 | **2** |
| **15** | **C₂H₅** | **H** | 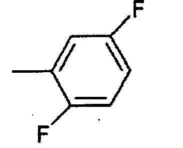 | **2** |
| **16** | **C₂H₅** | **H** | 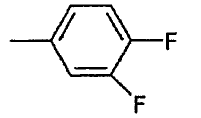 | **2** |
| **17** | **C₂H₅** | **H** | 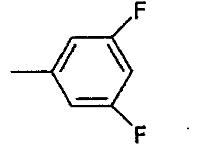 | **2** |
| **19** *(1-form)* | **C₂H₅** | **H** | 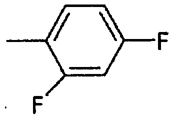 | **2** |
| **20** *(d-form)* | **C₂H₅** | **H** | 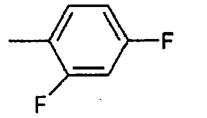 | **2** |

**[Table 3]**

| | | | | |
|---|---|---|---|---|
| **21** | **C₂H₅** | **H** | 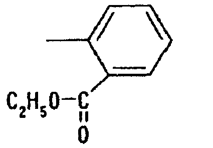 | **2** |
| **22** | **CH₃** | **H** | 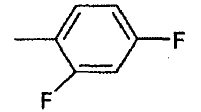 | **2** |
| **23** | **(CH₂)₂CH₃** | **H** | 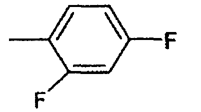 | **2** |
| **24** | **CH₃** | **H** | 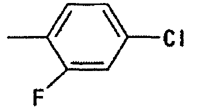 | **2** |
| **25** | **CH(CH₃)₂** | **H** | 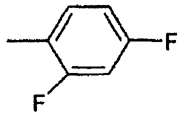 | **2** |
| **26** | **C₂H₅** | **H** | 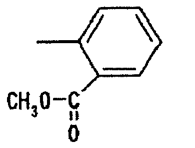 | **2** |
| **27** | **C₂H₅** | **H** | 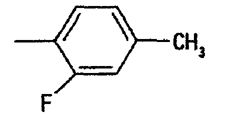 | **2** |
| **28** | **C₂H₅** | **H** | 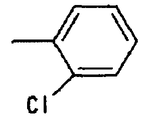 | **2** |
| **29** | **C₂H₅** | **H** | 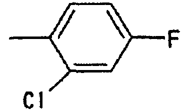 | **2** |
| **30** | **C₂H₅** | **H** | 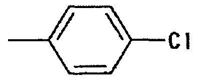 | **2** |

**[Table 4]**

| | | | | |
|---|---|---|---|---|
| **31** | **C₂H₅** | **H** | 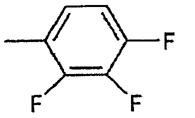 | **2** |
| **32** | **CH₂CH(CH₃)₂** | **H** | 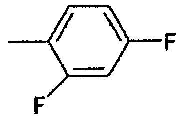 | **2** |
| **33** | **(CH₂)₃CH₃** | **H** | 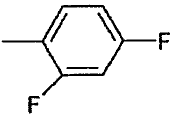 | **2** |
| **34** | **C₂H₅** | **H** | 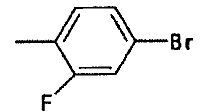 | **2** |
| **35** | **C₂H₅** | **H** | 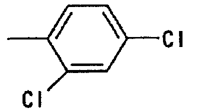 | **2** |
| **36** | **C₂H₅** | **H** | 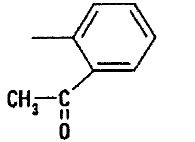 | **2** |
| **37** | **C₂H₅** | **H** | 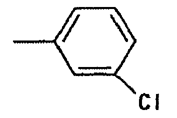 | **2** |
| **38** | **C₂H₅** | **H** | 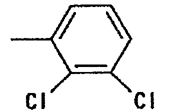 | **2** |
| **39** | **C₂H₅** | **H** | 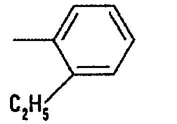 | **2** |
| **40** | **C₂H₅** | **H** | 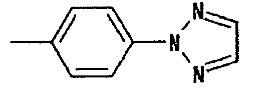 | **2** |

**[Table 5]**

| | | | | |
|---|---|---|---|---|
| **41** | **C₂H₅** | **H** | 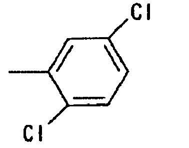 | **2** |
| **42** | **C₂H₅** | **H** | 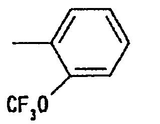 | **2** |
| **43** | **C₂H₅** | **H** | 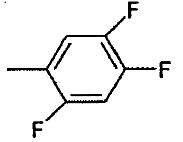 | **2** |
| **44** | **C₂H₅** | **H** | 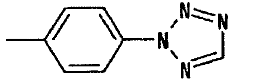 | **2** |
| **45** | **C₂H₅** | **H** | 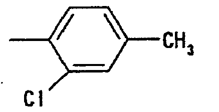 | **2** |
| **46** | **C₂H₅** | **H** | 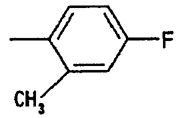 | **2** |
| **47** | **C₂H₅** | **H** | 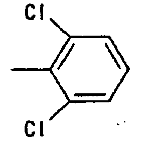 | **2** |
| **48** | **C₂H₅** | **H** | 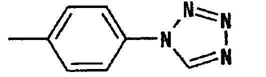 | **2** |
| **49** | **C₂H₅** | **H** | 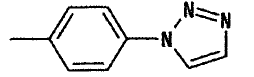 | **2** |
| **50** | **C₂H₅** | **H** | 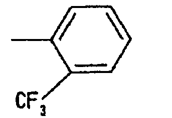 | **2** |

**[Table 6]**

| | | | | |
|---|---|---|---|---|
| **51** | **C₂H₅** | **H** | 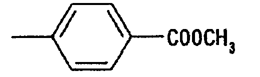 | **2** |
| **52** | 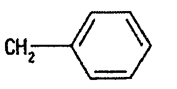 | **H** | 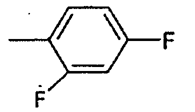 | **2** |
| **53** | **C₂H₅** | **H** | 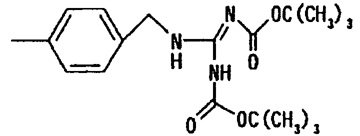 | **2** |
| **54** | **C₂H₅** | **H** | 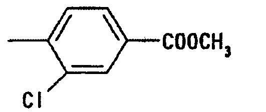 | **2** |
| **55** | **C₂H₅** | **H** | 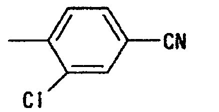 | **2** |
| **56** | **(CH₂)₂OH** | **H** | 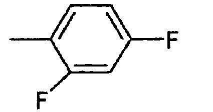 | **2** |
| **57** | **C₂H₅** | **H** | 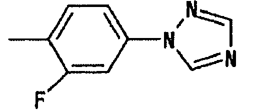 | **2** |
| **58** | **C₂H₅** | **H** | 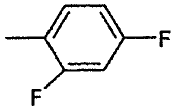 | **1** |
| **59** | **CH₂COOC(CH₃)₃** | **H** | 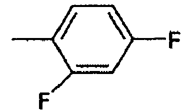 | **2** |
| **60** | **CH₂COOH** | **H** | 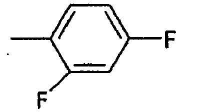 | **2** |

**[Table 7]**

| | | | | |
|---|---|---|---|---|
| **61** | **C₂H₅** | **H** | 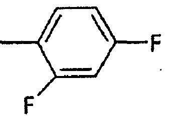 | **3** |
| **62** | **C₂H₅** | **H** | 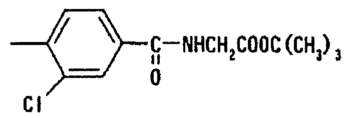 | **2** |
| **63** | **C₂H₅** | **H** | 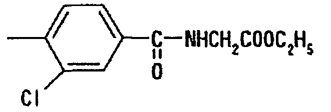 | **2** |
| **64** | **C₂H₅** | **H** | 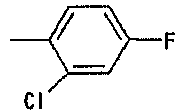 | **1** |
| **65** | **C₂H₅** | **H** | 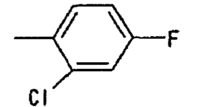 | **3** |
| **71** *(S-form)* | **C₂H₅** | **H** | 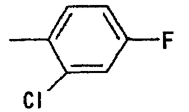 | **2** |
| **72** *(R-form)* | **C₂H₅** | **H** | 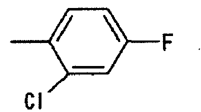 | **2** |
| **73** | **C₂H₅** | **H** | 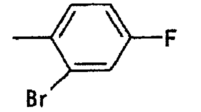 | **2** |
| **74** | **C₂H₅** | **H** | 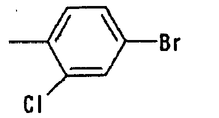 | **2** |

**[Table 8]**

| | | | |
|---|---|---|---|
| 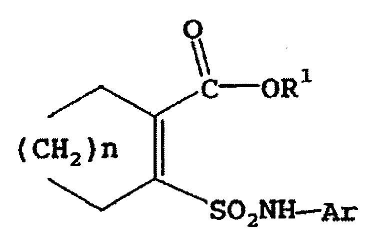 | | | |

| Compound No. | **R¹** | **Ar** | **n** |
|---|---|---|---|
| **7** | **C₂H₅** | 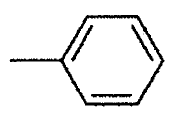 | **2** |
| **8** | **C₂H₅** | 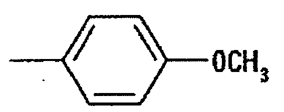 | **2** |
| **9** | **C₂H₅** | 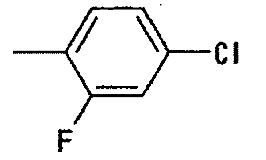 | **2** |
| **18** | **C₂H₅** | 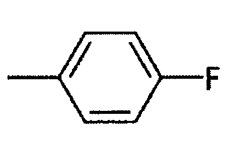 | **2** |
| **66** | **C₂H₅** | 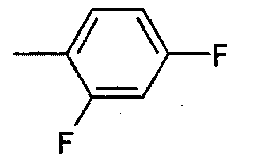 | **1** |

**[Table 9]**

| | | |
|---|---|---|
| 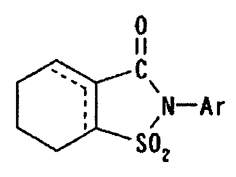 | | |

| Compound No. | 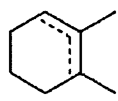 | **Ar** |
|---|---|---|
| **67** | 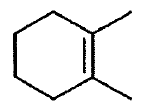 | 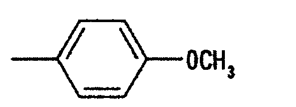 |
| **68** | 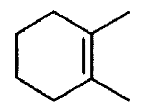 | 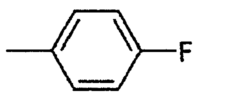 |
| **69** | 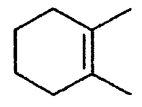 | 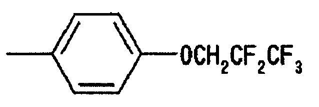 |
| **70** | 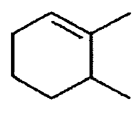 | 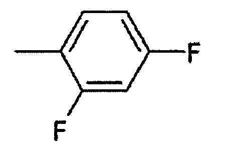 |

**[Table 10]**

| | | | | |
|---|---|---|---|---|
| 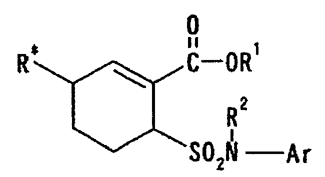 | | | | |

| Compound No. | **R¹** | **R²** | **R^{*}** | **Ar** |
|---|---|---|---|---|
| **75** high polar diastereomer | **C₂H₅ C₂H₅** | **H** | 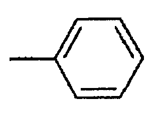 | 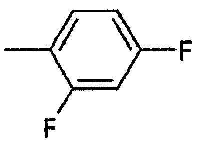 |
| **76** low polar diastereomer | **C₂H₅** | **H** | 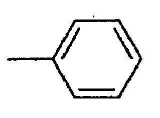 | 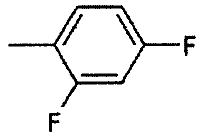 |
| **77** high polar diastereomer | **C₂H₅** | **H** | 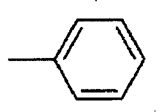 | 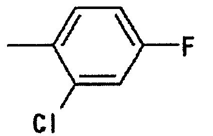 |
| **78** low polar diastereomer | **C₂H₅** | **H** | 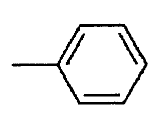 | 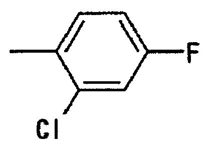 |
| **79** high polar diastereomer | **C₂H₅** | **H** | **C(CH₃)₃** | 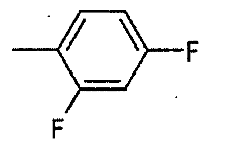 |
| **80** low polar diastereomer | **C₂H₅** | **H** | **C(CH₃)₃** | 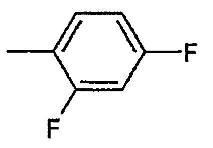 |
| **81** high polar diastereomer | **C₂H₅** | **H** | **C(CH₃)₃** | 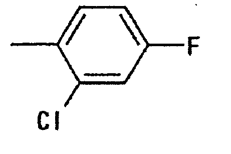 |

**[Table 11]**

| | | | | |
|---|---|---|---|---|
| **82** low polar diastereomer | **C₂H₅** | **H** | **C(CH₃)₃** | 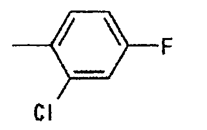 |
| **85** | **C₂H₅** | **H** | **Br** | 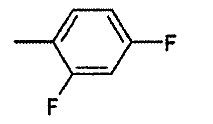 |

**[Table 12]**

| | |
|---|---|
| 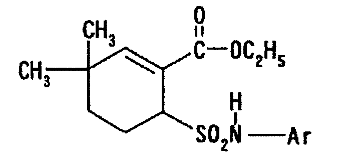 | |

| Compound No. | **Ar** |
|---|---|
| **83** | 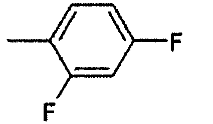 |
| **84** | 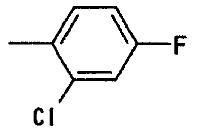 |

### [Reference Examples C]

Reference Example C1 ethyl 6-(benzylsulfanyl)-1-cyclohexene-1-carboxylate
Reference Example C2 ethyl 6-[(4-methoxybenzyl)sulfanyl]-1-cyclohexene-1-carboxylate
Reference Example C3 ethyl 6-[(2,4-difluorobenzyl)sulfanyl]-1-cyclohexene-1-carboxylate
Reference Example C4 ethyl 6-[(2-chloro-4-fluorobenzyl)sulfanyl]-1-cyclohexene-1-carboxylate
Reference Example C5 ethyl 5-hydroxy-3,6-dihydro-2H-pyran-4-carboxylate
Reference Example C6 ethyl 5-sulfanyl-3,6-dihydro-2H-pyran-4-carboxylate
Reference Example C7 4-(ethoxycarbonyl)-5,6-dihydro-2H-pyran-3-sulfonic acid
Reference Example C8 ethyl 5-(chlorosulfonyl)-3,6-dihydro-2H-pyran-4-carboxylate

### [Reference Examples D]

Reference Example D1 ethyl 6-(benzylsulfonyl)-1-cyclohexene-1-carboxylate (compound 1')
Reference Example D2 ethyl 6-[(4-methoxybenzyl)sulfonyl]-1-cyclohexene-1-carboxylate (compound 2')
Reference Example D3 ethyl 6-[(2,4-difluorobenzyl)sulfonyl]-1-cyclohexene-1-carboxylate (compound 3')
Reference Example D4 ethyl 6-[(2-chloro-4-fluorobenzyl)sulfonyl]-1-cyclohexene-1-carboxylate (compound 4')
Reference Example D5 ethyl (-)-6-[(2-chloro-4-fluorobenzyl)sulfonyl]-1-cyclohexene-1-carboxylate (compound 5')
ethyl (+)-6-[(2-chloro-4-fluorobenzyl)sulfonyl]-1-cyclohexene-1-carboxylate (compound 6')
Reference Example D6 ethyl 3-[(2,4-difluorophenyl)sulfamoyl]-3,6-dihydro-2H-pyran-4-carboxylate (compound 7')
Reference Example D7 ethyl 3-[(2-chloro-4-fluorophenyl)sulfamoyl]-3,6-dihydro-2H-pyran-4-carboxylate (compound 8')

The chemical structures of the compounds 1' - 8' are shown in Table 13 and Table 14.

**[Table 13]**

| | |
|---|---|
| 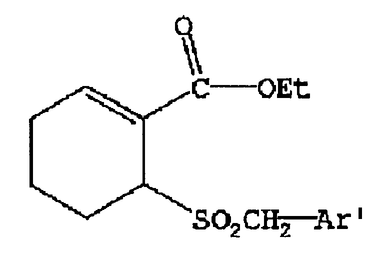 | |

| Compound No. | **Ar'** |
|---|---|
| **1'** | 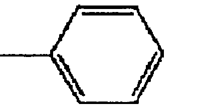 |
| **2'** | 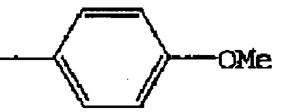 |
| **3'** | 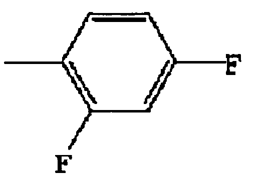 |
| **4'** | 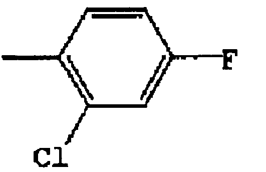 |
| **5'** (-)- form | 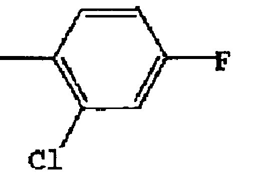 |
| **6'** (+)- form | 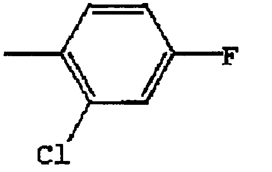 |

**[Table 14]**

| | |
|---|---|
| 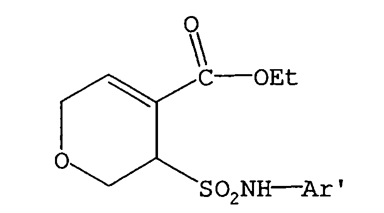 | |

| Compound No. | Ar' |
|---|---|
| 7' | 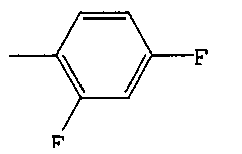 |
| 8' | 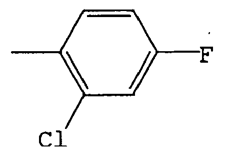 |

### Example 1

**[Table 15]**

| | formulation 1 | control formulation 2 |
|---|---|---|
| compound 72 | 11 g | 17 g |
| soybean oil | 220 g | 340 g |
| egg yolk lecithin | 13.2 g | 20.4 g |
| glycerol | 24.7 g | 38.25 g |
| dimyristoylphosphatidylglycerol | 2.2 g | 3.4 g |
| acetic acid | 11.2 mM | - |
| sodium acetate anhydride | 8.8 mM | - |
| distilled water total amount | 1.1 L | 1.7 L |

Compound 72 (11 g) was dissolved in soybean oil (220 g), and egg-yolk lecithin (13.2 g) and dimyristoylphosphatidylglycerol (2.2 g) were dissolved therein. Glycerol (24.7 g) was dissolved in distilled water, and acetate buffer was added and dissolved to a final concentration of acetic acid 11.2 mM and sodium acetate 8.8 mM. They were mixed, crudely emulsified and finely emulsified by a high-pressure homogenizer at a pressure of 8000 psi. The obtained emulsion composition (20 mL) was filled in a 20 mL vial, purged with nitrogen, tightly sealed with a rubber plug and a plastic cap, and subjected to a sterilization treatment in an autoclave at 121°C or above for 15 min or longer to give an emulsion composition having the composition of the above-mentioned formulation 1.
Then, as a control, compound 72 (17 g) was dissolved in soybean oil (340 g), and egg-yolk lecithin (20.4 g) and dimyristoylphosphatidylglycerol (3.4 g) were dissolved therein. Glycerol (38.25 g) was dissolved in distilled water. They were mixed, crudely emulsified and finely emulsified by a high-pressure homogenizer at a pressure of 8000 psi. The obtained emulsion composition (20 mL) was filled in a 20 mL vial, purged with nitrogen, tightly sealed with a rubber plug and a plastic cap, and subjected to a sterilization treatment in an autoclave at 121°C or above for 15 min or longer to give an emulsion composition having the composition of the above-mentioned control formulation 2.

### Example 2

Emulsion compositions of Formulation 1 and control formulation 2 obtained in Example 1 were preserved at 25°C, and the property, pH and mean particle size were measured over time. The particle size was measured by Malvern Mastersizer S. The results are shown in Table 16.
In formulation 1 containing acetate buffer, pH did not change before and after sterilization, and hardly changed even by a long-term preservation at 25°C, and formulation 1 was stable in the property, pH and mean particle size. In control formulation 2, pH decreased from 4.6 to 4.0 before and after sterilization, and further decreased to 3.6 by preservation at 25°C for 3 months. Due to the decrease in pH, the emulsion composition became unstable, and dispersion phase particles and water wherein the dispersion phase particles are dispersed showed phase separation by preservation at 25°C for 6 months (inadequate property), and the mean particle size increased to 0.8 µm. Thus, by the addition of acetate buffer to an emulsion composition containing compound 72, pH decrease during sterilization and pH decrease during long-term preservation could be improved.

**[Table 16]**

| | | property | pH | average particle size (µm) |
|---|---|---|---|---|
| formulation 1 | before sterilization | adequate¹⁾ | 4.4 | 0.3 |
| | Initial | adequate¹⁾ | 4.4 | 0.3 |
| | 25°C, 3 months | adequate¹⁾ | 4.4 | 0.3 |
| | 25°C, 6 months | adequate¹⁾ | 4.5 | 0.3 |
| control formulation 2 | before sterilization | adequate¹⁾ | 4.6 | 0.3 |
| | Initial | adequate¹⁾ | 4.0 | 0.3 |
| | 25°C, 3 months | adequate¹⁾ | 3.6 | 0.3 |
| | 25°C, 6 months | inadequate²⁾ | 3.6 | 0.8 |

| | | | | |
|---|---|---|---|---|
| 1) Oil drop, creaming and phase separation were not observed. 2) Dispersion phase particles and water wherein the dispersion phase particles were dispersed showed phase separation. | | | | |

### Example 3

**[Table 17]**

| | formulation 3 | control formulation 4 |
|---|---|---|
| compound 72 | 600 g | 600 g |
| soybean oil | 12000 g | 12000 g |
| egg-yolk lecithin | 720 g | 720 g |
| glycerol | 1350 g | 1350 g |
| dimyristoylphosphatidylglycerol | 120 g | 120 g |
| glacial acetic acid | 40.35 g | - |
| sodium acetate trihydrate | 71.85 g | - |
| distilled water total amount | 60 L | 60 L |

Compound 72 (600 g) was dissolved in soybean oil (12 kg), and purified egg-yolk lecithin (720 g) and dimyristoylphosphatidylglycerol (120 g) were dissolved therein at 50 to 60°C. Glycerol (1350 g) was dissolved in distilled water (20 kg), and glacial acetic acid (40.35 g) and sodium acetate trihydrate (71.85 g) were added and dissolved at 50 to 60°C. They were mixed and crudely emulsified by homogenizer Polytron for 5 min. Then, the emulsion was finely emulsified by high-pressure homogenizer at a pressure of 8000 psi, 8 passes. Compound 72 was dissolved and emulsified under a nitrogen stream. The obtained emulsion composition was filtered through a membrane filter having a pore size of 4.5 µm. The obtained emulsion composition (20 mL) was filled in a 20 mL vial, purged with nitrogen, tightly sealed with a rubber plug and a plastic cap, and subjected to a sterilization treatment in an autoclave at 121°C or above for 15 min or longer to give an emulsion composition having the composition of the above-mentioned formulation 3.
Then, as a control, compound 72 (600 g) was dissolved in soybean oil (12 kg), and purified egg-yolk lecithin (720 g) and dimyristoylphosphatidylglycerol (120 g) were dissolved therein at 50 to 60°C. Glycerol (1350 g) was dissolved in distilled water (35 kg) and mixed to dissolve the mixture at 50 to 60°C. They were mixed, and crudely emulsified by homogenizer Polytron for 5 min.
Then, the emulsion was finely emulsified by high-pressure homogenizer at a pressure of 8000 psi, 8 passes. Compound 72 was dissolved and emulsified under a nitrogen stream. The obtained emulsion composition was filtered through a membrane filter having a pore size of 5µm. The obtained emulsion composition (20 mL) was filled in a 20 mL vial, purged with nitrogen, tightly sealed with a rubber plug and a plastic cap, and subjected to a sterilization treatment in an autoclave at 121°C or above for 15 min or longer to give an emulsion composition having the composition of the above-mentioned control formulation 4.

### Example 4

Emulsion compositions of formulation 3 and control formulation 4 obtained in Example 3 were preserved at 25°C, and the property, pH and mean particle size were measured over time. The mean particle size of formulation 3 was measured by Malvern Mastersizer 2000, and that of control formulation 4 was measured by Malvern Mastersizer S.
The results are shown in Table 18. In formulation 3 containing acetate buffer, pH was constant before and after emulsification-sterilization, and pH did not decrease even by a long-term preservation at 25°C for 18 months, further at 25°C for 24 months, and formulation 3 was highly stable with no change in the property and mean particle size.
In control formulation 4, pH decreased after preservation at 25°C for 3 months. After preservation at 25°C for 6 months, free oil drop was observed on the surface of the emulsion composition (inadequate property), and the mean particle size increased to 3.6 µm. Thus, by the addition of 20 mM acetate buffer to an emulsion composition containing compound 72, emulsion composition free of pH decrease during preparation, sterilization and long-term preservation could be prepared. Thus, preparation of a markedly stable emulsion composition became possible.

**[Table 18]**

| | | property | pH | average particle size (µm) |
|---|---|---|---|---|
| formulation 3 | before emulsification - sterilization | adequate¹⁾ | 4.5 | - |
| | initial | adequate¹⁾ | 4.4 | 0.2 |
| | 25°C, 3 months | adequate¹⁾ | 4.5 | 0.2 |
| | 25°C, 6 months | adequate¹⁾ | 4.5 | 0.2 |
| | 25°C, 15 months | adequate¹⁾ | 4.5 | 0.2 |
| | 25°C, 18 months | adequate¹⁾ | 4.5 | 0.2 |
| | 25°C, 24 months | adequate¹⁾ | 4.5 | 0.2 |
| control formulation 4 | before emulsification - sterilization | adequate¹⁾ | 5.2 | - |
| | initial | adequate¹⁾ | 4.0 | 0.3 |
| | 25°C, 3 months | adequate¹⁾ | 3.5 | 0.3 |
| | 25°C, 6 months | inadequate²⁾ | 3.5 | 3.6 |

| | | | | |
|---|---|---|---|---|
| 1) Oil drop, creaming and phase separation were not observed. 2) Free oil drop was visibly observed. | | | | |

### Example 5

To prepare a formulation of total amount 25 mL containing compound 72, soybean oil, egg-yolk lecithin, glycerol, dimyristoylphosphatidylglycerol and distilled water at the same rate with control formulation 2 and further containing various buffers, various concentrations of buffer was added and dissolved in an aqueous phase comprising glycerol and distilled water. As the composition of each buffer, acetate buffer contains acetic acid and sodium acetate, lactate buffer contains lactic acid and sodium lactate, citrate buffer contains citric acid and sodium citrate, phosphate-citrate buffer contains disodium hydrogen phosphate and citric acid, phosphate buffer contains sodium dihydrogen phosphate and disodium hydrogen phosphate, carbonate buffer contains sodium carbonate and sodium hydrogen carbonate, and their concentrations are as described in Tables 19 to 22. Then, compound 72, egg-yolk lecithin and dimyristoylphosphatidylglycerol were dissolved in soybean oil. They were mixed and crudely emulsified by a homogenizer, and an emulsion composition was prepared by a sonicator. The obtained emulsion composition was filtered through a membrane filter having a pore size of 5 µm. The obtained emulsion composition (20 mL) was filled in a 20 mL vial, purged with nitrogen, tightly sealed with a rubber plug and a plastic cap, and subjected to a sterilization treatment in an autoclave at 121°C or above for 15 min or longer to give emulsion compositions containing various buffers. The pH of the emulsion compositions before and after autoclave treatment was examined.
The results are shown in Tables 19 to 22. In emulsion compositions containing compound 72, addition of acetate buffer, lactate buffer, citrate buffer and phosphate - citrate buffer at 5 mM to 32 mM afforded emulsion compositions of pH 4 to 5 which hardly show pH change even by a high-pressure vapor sterilization treatment.
When the pH of an emulsion composition was adjusted to 6 with a buffer, pH decreased markedly by a high-pressure vapor sterilization treatment, even when phosphate buffer, carbonate buffer or citrate buffer was used. With carbonate buffer, pH changed to about 7 before high-pressure vapor sterilization.

**[Table 19]**

| pH of emulsion composition | kind of buffer | buffer concentration | high-pressure vapor sterilization | pH |
|---|---|---|---|---|
| 4 | acetate buffer | 5 mM | before treatment after treatment | 4.1 4.1 |
| | | 10 mM | before treatment after treatment | 4.0 4.0 |
| | | 15 mM | before treatment after treatment | 4.0 4.0 |
| | | 20 mM | before treatment after treatment | 4.0 4.0 |
| | lactate buffer | 5 mM | before treatment after treatment | 4.2 4.1 |
| | | 12.5 mM | before treatment after treatment | 4.2 4.1 |
| | | 25 mM | before treatment after treatment | 4.2 4.1 |
| | citrate buffer | 5 mM | before treatment after treatment | 4.1 4.1 |
| | | 10 mM | before treatment after treatment | 4.0 4.1 |
| | | 15 mM | before treatment after treatment | 4.0 4.1 |
| | | 20 mM | before treatment after treatment | 4.1 4.2 |

**[Table 20]**

| pH of emulsion composition | kind of buffer | buffer concentration | high-pressure vapor sterilization | pH |
|---|---|---|---|---|
| 4.5 | acetate buffer | 5 mM | before treatment after treatment | 4.6 4.5 |
| | | 10 mM | before treatment after treatment | 4.6 4.4 |
| | | 15 mM | before treatment after treatment | 4.5 4.4 |
| | | 20 mM | before treatment after treatment | 4.5 4.4 |
| | lactate buffer | 5 mM | before treatment after treatment | 4.5 4.4 |
| | | 12.5 mM | before treatment after treatment | 4.5 4.4 |
| | | 25 mM | before treatment after treatment | 4.5 4.4 |
| | phosphate - citrate buffer | 8 mM | before treatment after treatment | 4.7 4.3 |
| | | 16 mM | before treatment after treatment | 4.6 4.3 |
| | | 32 mM | before treatment after treatment | 4.6 4.3 |
| | citrate buffer | 5 mM | before treatment after treatment | 4.6 4.7 |
| | | 10 mM | before treatment after treatment | 4.6 4.7 |
| | | 15 mM | before treatment after treatment | 4.5 4.7 |
| | | 20 mM | before treatment after treatment | 4.5 4.7 |

**[Table 21]**

| pH of emulsion composition | kind of buffer | buffer concentration | high-pressure vapor sterilization | pH |
|---|---|---|---|---|
| 5 | acetate buffer | 5 mM | before treatment after treatment | 5.0 4.8 |
| | | 10 mM | before treatment after treatment | 5.0 4.8 |
| | | 15 mM | before treatment after treatment | 4.9 4.8 |
| | | 20 mM | before treatment after treatment | 5.0 4.9 |
| | citrate buffer | 5 mM | before treatment after treatment | 5.1 5.1 |
| | | 10 mM | before treatment after treatment | 5.1 5.2 |
| | | 15 mM | before treatment after treatment | 5.1 5.1 |
| | | 20 mM | before treatment after treatment | 5.0 5.0 |

**[Table 22]**

| pH of emulsion composition | kind of buffer | buffer concentration | high-pressure vapor sterilization | pH |
|---|---|---|---|---|
| 6 | citrate buffer | 5 mM | before treatment after treatment | 6.3 5.6 |
| | | 10 mM | before treatment after treatment | 6.3 5.7 |
| | | 15 mM | before treatment after treatment | 6.3 5.7 |
| | | 20 mM | before treatment after treatment | 6.3 5.6 |
| | phosphate buffer | 5 mM | before treatment after treatment | 6.1 4.6 |
| | | 10 mM | before treatment after treatment | 6.1 4.7 |
| | | 15 mM | before treatment after treatment | 6.0 5.0 |
| | | 20 mM | before treatment after treatment | 6.0 5.3 |
| | carbonate buffer | 5 mM | before treatment after treatment | 7.0 4.3 |
| | | 10 mM | before treatment after treatment | 7.0 4.3 |
| | | 15 mM | before treatment after treatment | 7.0 5.0 |
| | | 20 mM | before treatment after treatment | 7.1 5.6 |

### Comparative Example 1

To prepare a formulation (total amount 25 mL) containing compound 72, soybean oil, egg-yolk lecithin, glycerol, dimyristoylphosphatidylglycerol and distilled water at the same rate with control formulation 2 and further containing various concentrations of sodium hydroxide as a pH adjuster, sodium hydroxide was dissolved in an aqueous phase consisting of glycerol and distilled water to final concentrations of 0, 0.5, 0.75, 1, 1.5 and 2.0 mM. Then, compound 72, egg-yolk lecithin and dimyristoylphosphatidylglycerol were dissolved in soybean oil. They were mixed and crudely emulsified by a homogenizer, and an emulsion composition was prepared by a sonicator. The obtained emulsion composition was filtered through a membrane filter having a pore size of 5 µm. The obtained emulsion composition (20 mL) was filled in a 20 mL vial, purged with nitrogen, tightly sealed with a rubber plug and a plastic cap, and subjected to a sterilization treatment in an autoclave at 121°C or above for 15 min or longer to give emulsion compositions containing various concentrations of sodium hydroxide as a pH adjuster. The pH of each emulsion compositions before and after autoclave treatment was examined.
The results are shown in Table 23. The emulsion compositions containing compound 72 showed remarkable pH decrease after sterilization when any concentration of sodium hydroxide, including that free of sodium hydroxide, was added.

**[Table 23]**

| sodium hydroxide concentration | high-pressure vapor sterilization | pH |
|---|---|---|
| 0 mM | before treatment after treatment | 5.4 4.6 |
| 0.5 mM | before treatment after treatment | 6.5 4.5 |
| 0.75 mM | before treatment after treatment | 6.7 4.5 |
| 1 mM | before treatment after treatment | 6.7 4.5 |
| 1.5 mM | before treatment after treatment | 6.7 4.6 |
| 2.0 mM | before treatment after treatment | 6.9 4.7 |

This application is based on a patent application No. 2006-135883 filed in Japan, the contents of which are incorporated in full herein by this reference.
Although the present invention have been presented or described by referring to preferred embodiments of this invention, it will, however, be understood by those of ordinary skill in the art that various modifications may be made to the forms and details without departing from the scope of the invention as set forth in the appended claims. All patents, patent publications and other publications indicated or cited in the Specification are hereby incorporated in their entireties by reference.

## Claims

1. An agent for the prophylaxis or treatment of complications after coronary-artery bypass surgery, which comprises a compound represented by the formula (I): 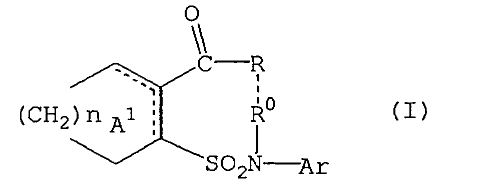 wherein
R is an aliphatic hydrocarbon group optionally having substituent(s), an aromatic hydrocarbon group optionally having substituent(s), a heterocyclic group optionally having substituent(s), a group represented by the formula: -OR¹ wherein R¹ is a hydrogen atom or an aliphatic hydrocarbon group optionally having substituent(s), or a group represented by the formula: 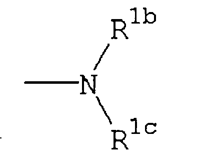 wherein R^{1b} and R^{1c} are the same or different and each is a hydrogen atom or an aliphatic hydrocarbon group optionally having substituent(s),
R⁰ is a hydrogen atom or an aliphatic hydrocarbon group, or R and R⁰ in combination form a bond,
ring A¹ is a cycloalkene optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR¹¹ wherein R¹¹ is a hydrogen atom or an aliphatic hydrocarbon group optionally having substituent(s), and (4) a halogen atom,
Ar is an aromatic hydrocarbon group optionally having substituent(s),
a group represented by the formula: 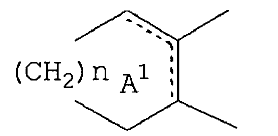 is a group represented by the formula: 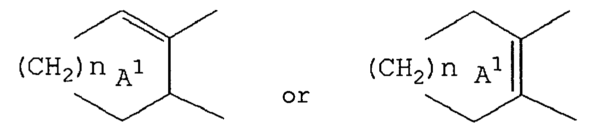 and n is an integer of 1 to 4,
or a salt thereof or a prodrug thereof, or
a compound represented by the formula (II): 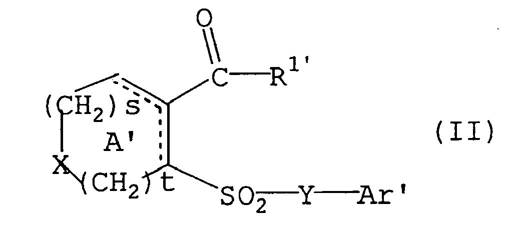 wherein
R^{1'} is an aliphatic hydrocarbon group optionally having substituent(s), an aromatic hydrocarbon group optionally having substituent(s), a heterocyclic group optionally having substituent(s), a group represented by the formula: -OR^{1a'} wherein R^{1a'} is a hydrogen atom or an aliphatic hydrocarbon group optionally having substituent(s), or a group represented by the formula: 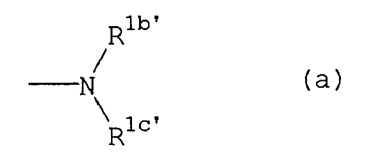 wherein R^{1b'} and R^{1c'} are the same or different and each is a hydrogen atom or an aliphatic hydrocarbon group optionally having substituent(s),
X is a methylene group, NH, a sulfur atom or an oxygen atom,
Y is a methylene group optionally having substituent(s) or NH optionally having substituent(s),
ring A' is a 5- to 8-membered ring optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR^{2'} wherein R^{2'} is a hydrogen atom or an aliphatic hydrocarbon group optionally having substituent(s), and (4) a halogen atom, Ar' is an aromatic hydrocarbon group optionally having substituent(s),
a group represented by the formula: 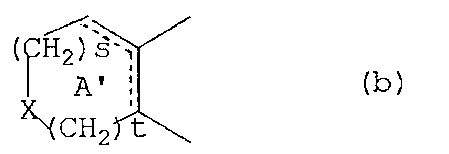 is a group represented by the formula: 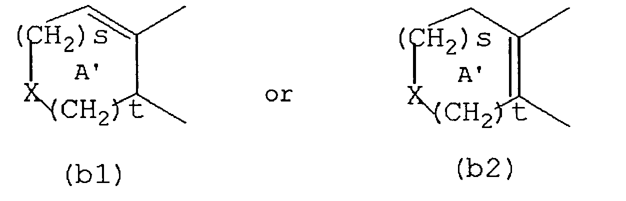 s is an integer of 0 to 2,
t is an integer of 1 to 3, and
the total of s and t is 4 or less;
provided that when X is a methylene group, then Y should be a methylene group optionally having substituent(s), a salt thereof and a prodrug thereof.

2. An agent for the prophylaxis or treatment of cardiac diseases, autoimmune diseases, central nervous system diseases, inflammatory diseases, sepsis, severe sepsis or septic shock in a patient who undergoes coronary-artery bypass surgery, which comprises a compound represented by the formula (I): 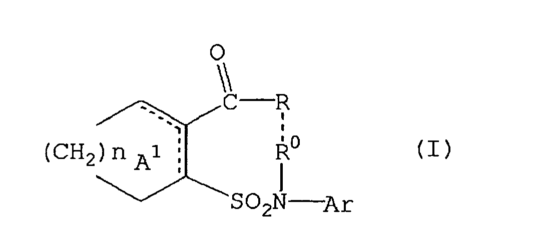 wherein
R is an aliphatic hydrocarbon group optionally having substituent(s), an aromatic hydrocarbon group optionally having substituent(s), a heterocyclic group optionally having substituent(s), a group represented by formula: -OR¹ wherein R¹ is a hydrogen atom or an aliphatic hydrocarbon group optionally having substituent(s), or a group represented by the formula: 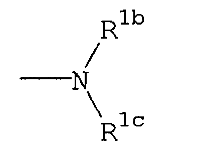 wherein R^{1b} and R^{1c} are the same or different and each is a hydrogen atom or an aliphatic hydrocarbon group optionally having substituent(s),
R⁰ is a hydrogen atom or an aliphatic hydrocarbon group, or R and R⁰ in combination form a bond,
ring A¹ is a cycloalkene optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR¹¹ wherein R¹¹ is a hydrogen atom or an aliphatic hydrocarbon group optionally having substituent(s), and (4) a halogen atom,
Ar is an aromatic hydrocarbon group optionally having substituent(s),
a group represented by the formula: 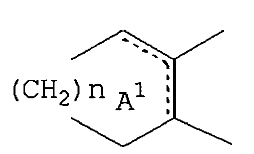 is a group represented by the formula: 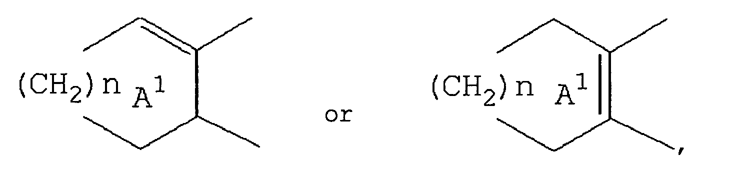 and n is an integer of 1 to 4,
or a salt thereof or a prodrug thereof, or
a compound represented by the formula (II): 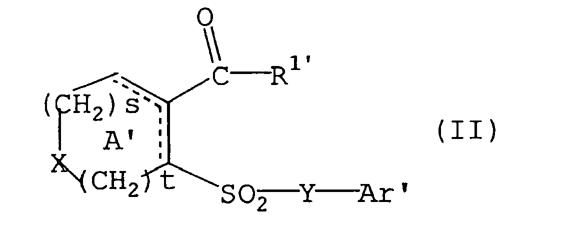 wherein
R^{1'} is an aliphatic hydrocarbon group optionally having substituent(s), an aromatic hydrocarbon group optionally having substituent(s), a heterocyclic group optionally having substituent(s), a group represented by the formula: -OR^{1a'} wherein R^{1a'} is a hydrogen atom or an aliphatic hydrocarbon group optionally having substituent(s), or a group represented by the formula: 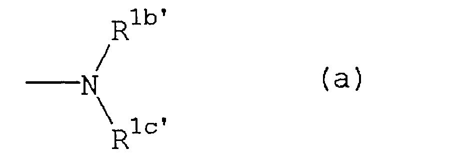 wherein R^{1b'} and R^{1c'} are the same or different and each is a hydrogen atom or an aliphatic hydrocarbon group optionally having substituent(s),
X is a methylene group, NH, a sulfur atom or an oxygen atom,
Y is a methylene group optionally having substituent(s) or NH optionally having substituent(s),
ring A' is a 5- to 8-membered ring optionally having 1 to 4 substituents selected from the group consisting of (1) an aliphatic hydrocarbon group optionally having substituent(s), (2) an aromatic hydrocarbon group optionally having substituent(s), (3) a group represented by formula: -OR^{2'} wherein R^{2'} is a hydrogen atom or an aliphatic hydrocarbon group optionally having substituent(s), and (4) a halogen atom, Ar' is an aromatic hydrocarbon group optionally having substituent(s),
a group represented by the formula: 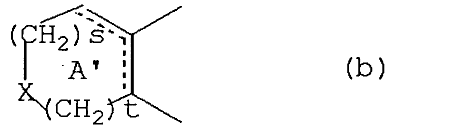 is a group represented by the formula: 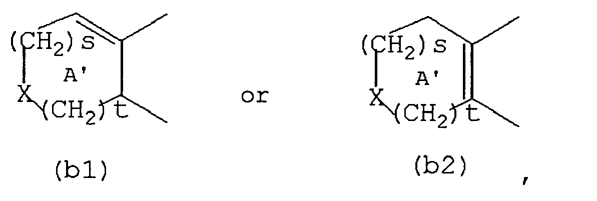 s is an integer of 0 to 2,
t is an integer of 1 to 3, and
the total of s and t is 4 or less;
provided that when X is a methylene group, then Y should be a methylene group optionally having substituent(s), a salt thereof and a prodrug thereof.

3. The agent of claim 1 or 2, wherein the formula (I) is the formula (Ia): 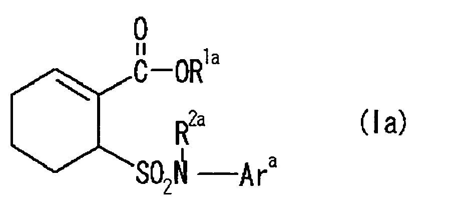 wherein R^{1a} is a C₁₋₆ alkyl group, R^{2a} is a hydrogen atom or a C₁₋₆ alkyl group, Ar^{a} is a phenyl group substituted by 1 or 2 halogen atoms, and the formula (II) is the formula (IIa): 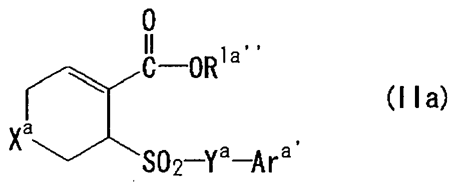 wherein R^{1a"} is a C₁₋₆ alkyl group, X^{a} is a methylene group or an oxygen atom, Y^{a} is a methylene group or -NH-, Ar^{a'} is a phenyl group optionally having 1 or 2 substituents selected from the group consisting of a halogen atom and a C₁₋₆ alkoxy group, provided that when X^{a} is a methylene group, then Y^{a} should be a methylene group.

4. The agent of claim 1 or 2, which is used in combination with at least one drug selected from the group consisting of antibacterial agents, antifungal agents, antivirus drugs, non-steroidal antiinflammatory drugs, steroids, anticoagulants, cytopathy suppressive agents and anti-sepsis drugs.

5. A method for preventing or treating complications after coronary-artery bypass surgery, which comprises administering an effective amount of a compound represented by the formula (I) : 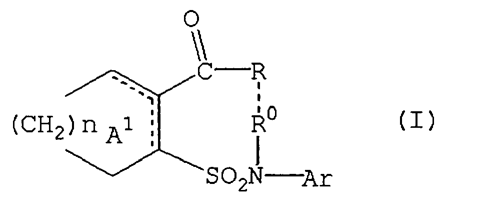 wherein
R is an aliphatic hydrocarbon group optionally having substituent(s), an aromatic hydrocarbon group optionally having substituent(s), a heterocyclic group optionally having substituent(s), a group represented by the formula: -OR¹ wherein R¹ is a hydrogen atom or an aliphatic hydrocarbon group optionally having substituent(s), or a group represented by the formula: 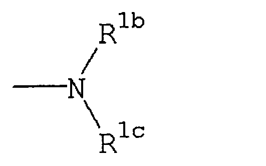 wherein R^{1b} and R^{1c} are the same or different and each is a hydrogen atom an aliphatic hydrocarbon group optionally having substituent(s),
R⁰ is a hydrogen atom or an aliphatic hydrocarbon group, or R and R⁰ in combination form a bond,
ring A¹ is a cycloalkene optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR¹¹ wherein R¹¹ is a hydrogen atom or an aliphatic hydrocarbon group optionally having substituent(s), and (4) a halogen atom,
Ar is an aromatic hydrocarbon group optionally having substituent(s),
a group represented by the formula: 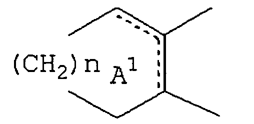 is a group represented by the formula: 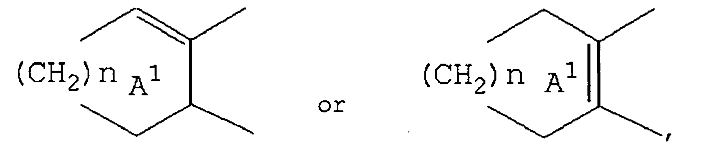 and n is an integer of 1 to 4,
or a salt thereof or a prodrug thereof, or
a compound represented by the formula (II): 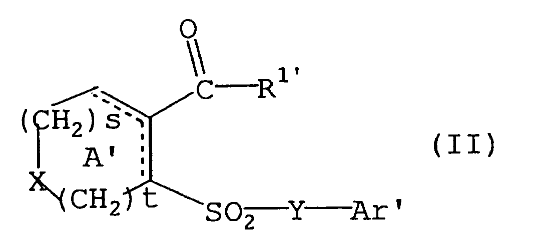 wherein
R^{1'} is an aliphatic hydrocarbon group optionally having substituent(s), an aromatic hydrocarbon group optionally having substituent(s), a heterocyclic group optionally having substituent(s), a group represented by the formula: -OR^{1a'} wherein R^{1a'} is a hydrogen atom or an aliphatic hydrocarbon group optionally having substituent(s), or a group represented by the formula: 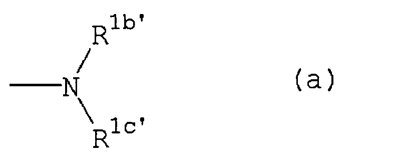 wherein R^{1b'} and R^{1c'} are the same or different and each is a hydrogen atom or an aliphatic hydrocarbon group optionally having substituent(s),
X is a methylene group, NH, a sulfur atom or an oxygen atom,
Y is a methylene group optionally having substituent(s) or NH optionally having substituent(s),
ring A' is a 5- to 8-membered ring optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR^{2'} wherein R^{2'} is a hydrogen atom or an aliphatic hydrocarbon group optionally having substituent(s), and (4) a halogen atom, Ar' is a hydrocarbon group optionally having substituent(s), a group represented by the formula: 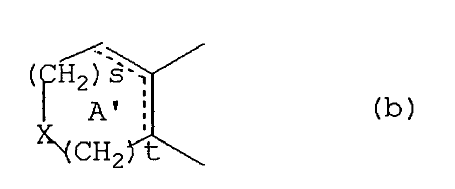 is a group represented by the formula: 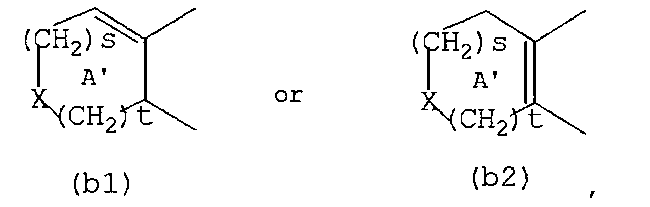 s is an integer of 0 to 2,
t is an integer of 1 to 3, and
the total of s and t is 4 or less;
provided that when X is a methylene group, then Y should be a methylene group optionally having substituent(s), or a salt thereof or a prodrug thereof, to a mammal.

6. Use of a compound represented by the formula (I): 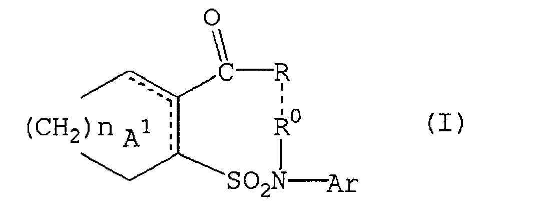 wherein
R is an aliphatic hydrocarbon group optionally having substituent(s), an aromatic hydrocarbon group optionally having substituent(s), a heterocyclic group optionally having substituent(s), a group represented by the formula: -OR¹ wherein R¹ is a hydrogen atom or an aliphatic hydrocarbon group optionally having substituent(s), or a group represented by the formula: 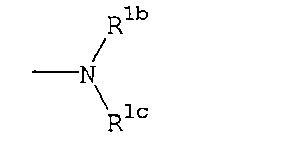 wherein R^{1b} and R^{1c} are the same or different and each is a hydrogen atom or an aliphatic hydrocarbon group optionally having substituent(s),
R⁰ is a hydrogen atom or an aliphatic hydrocarbon group, or R and R⁰ in combination form a bond,
ring A¹ is a cycloalkene optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR¹¹ wherein R¹¹ is a hydrogen atom or an aliphatic hydrocarbon group optionally having substituent(s), and (4) a halogen atom,
Ar is an aromatic hydrocarbon group optionally having substituent(s),
a group represented by the formula: 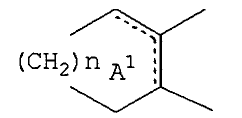 is a group represented by the formula: 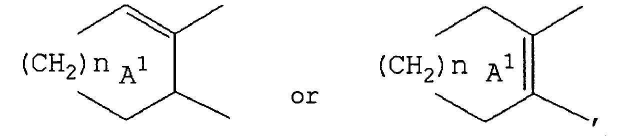 and n is an integer of 1 to 4,
or a salt thereof or a prodrug thereof, or
a compound represented by the formula (II): 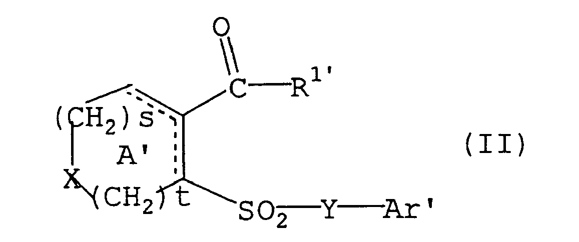 wherein
R^{1'} is an aliphatic hydrocarbon group optionally having substituent(s), an aromatic hydrocarbon group optionally having substituent(s), a heterocyclic group optionally having substituent(s), a group represented by the formula: -OR^{1a'} wherein R^{1a'} is a hydrogen atom or an aliphatic hydrocarbon group optionally having substituent(s), or a group represented by the formula: 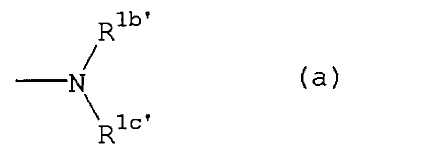 wherein R^{1b'} and R^{1c'} are the same or different and each is a hydrogen atom or an aliphatic hydrocarbon group optionally having substituent(s),
X is a methylene group, NH, a sulfur atom or an oxygen atom,
Y is a methylene group optionally having substituent(s) or NH optionally having substituent(s),
ring A' is a 5- to 8-membered ring optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR^{2'} wherein R^{2'} is a hydrogen atom or an aliphatic hydrocarbon group optionally having substituent(s), and (4) a halogen atom, Ar' is an aromatic hydrocarbon group optionally having substituent(s),
a group represented by the formula: 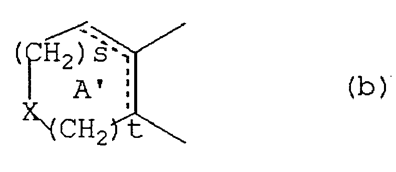 is a group represented by the formula: 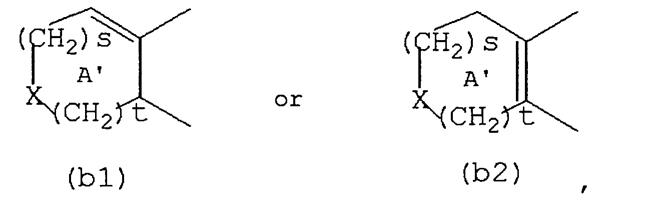 s is an integer of 0 to 2,
t is an integer of 1 to 3, and
the total of s and t is 4 or less;
provided that when X is a methylene group, then Y should be a methylene group optionally having substituent(s),
or a salt thereof or a prodrug thereof,
for the production of an agent for the prophylaxis or treatment of complications after coronary-artery bypass surgery.

7. A method for the prophylaxis or treatment of cardiac disease, autoimmune diseases, central nervous system diseases, inflammatory disease, sepsis, severe sepsis or septic shock, which comprises administering an effective amount of a compound represented by the formula (I): 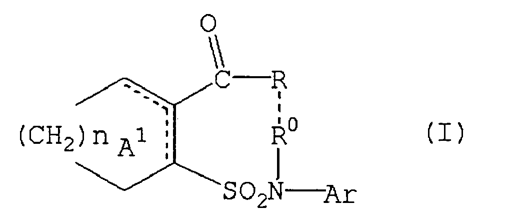 wherein
R is an aliphatic hydrocarbon group optionally having substituent(s), an aromatic hydrocarbon group optionally having substituent(s), a heterocyclic group optionally having substituent(s), a group represented by the formula: -OR¹ wherein R¹ is a hydrogen atom or an aliphatic hydrocarbon group optionally having substituent(s), or a group represented by the formula: 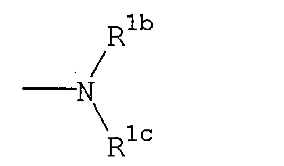 wherein R^{1b} and R^{1c} are the same or different and each is a hydrogen atom or an aliphatic hydrocarbon group optionally having substituent(s),
R⁰ is a hydrogen atom or an aliphatic hydrocarbon group, or R and R⁰ in combination form a bond,
ring A¹ is a cycloalkene optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR¹¹ wherein R¹¹ is a hydrogen atom or an aliphatic hydrocarbon group optionally having substituent(s), and (4) a halogen atom,
Ar is an aromatic hydrocarbon group optionally having substituent(s),
a group represented by the formula: 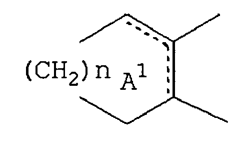 is a group represented by the formula: 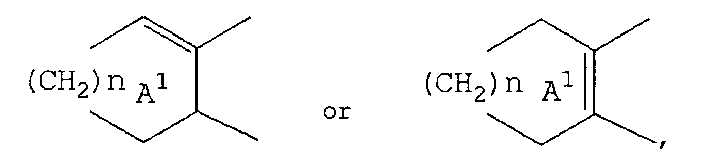 and n is an integer of 1 to 4,
or a salt thereof or a prodrug thereof, or
a compound represented by the formula (II): 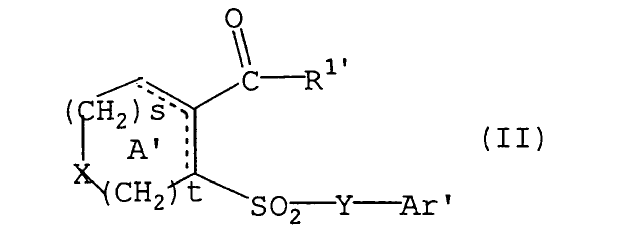 wherein
R^{1'} is an aliphatic hydrocarbon group optionally having substituent(s), an aromatic hydrocarbon group optionally having substituent(s), a heterocyclic group optionally having substituent(s), a group represented by the formula: -OR^{1a'} wherein R^{1a'} is a hydrogen atom or an aliphatic hydrocarbon group optionally having substituent(s), or a group represented by the formula: 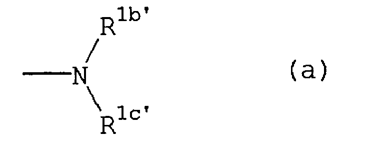 wherein R^{1b'} and R^{1c'} are the same or different and each is a hydrogen atom or an aliphatic hydrocarbon group optionally having substituent(s),
X is a methylene group, NH, a sulfur atom or an oxygen atom,
Y is a methylene group optionally having substituent(s) or NH optionally having substituent(s),
ring A' is a 5- to 8-membered ring optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR^{2'} wherein R^{2'} is a hydrogen atom or an aliphatic hydrocarbon group optionally having substituent(s), and (4) a halogen atom, Ar' is an aromatic hydrocarbon group optionally having substituent(s),
a group represented by the formula: 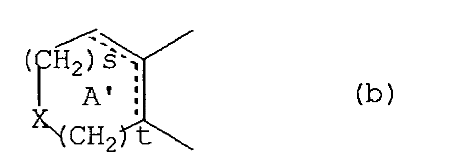 is a group represented by the formula: 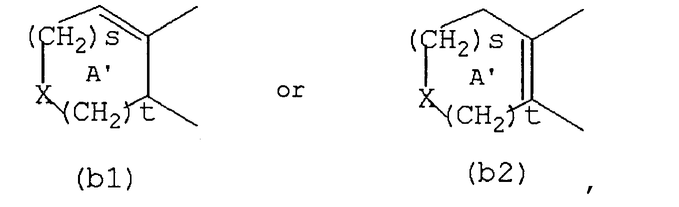 s is an integer of 0 to 2,
t is an integer of 1 to 3, and
the total of s and t is 4 or less;
provided that when X is a methylene group, then Y should be a methylene group optionally having substituent(s), or a salt thereof or a prodrug thereof, to a patient who undergoes coronary-artery bypass surgery.

8. Use of a compound represented by the formula (I): 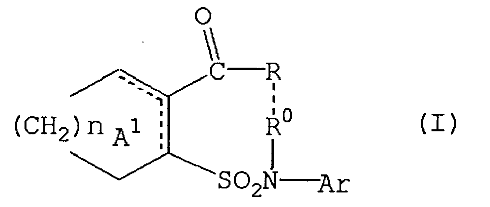 wherein
R is an aliphatic hydrocarbon group optionally having substituent(s), an aromatic hydrocarbon group optionally having substituent(s), a heterocyclic group optionally having substituent(s), a group represented by the formula: -OR¹ wherein R¹ is a hydrogen atom or an aliphatic hydrocarbon group optionally having substituent(s), or a group represented by the formula: 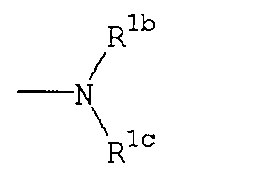 wherein R^{1b} and R^{1c} are the same or different and each is a hydrogen atom or an aliphatic hydrocarbon group optionally having substituent(s),
R⁰ is a hydrogen atom or an aliphatic hydrocarbon group, or R and R⁰ in combination form a bond,
ring A¹ is a cycloalkene optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR¹¹ wherein R¹¹ is a hydrogen atom or an aliphatic hydrocarbon group optionally having substituent(s), and (4) a halogen atom,
Ar is an aromatic hydrocarbon group optionally having substituent(s),
a group represented by the formula: 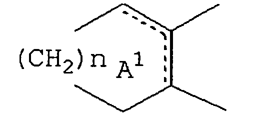 is a group represented by the formula: 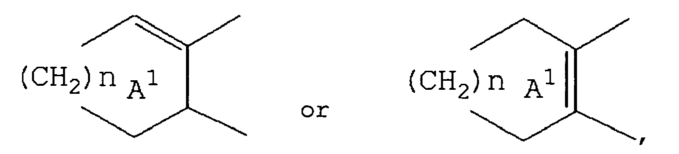 and n is an integer of 1 to 4,
or a salt thereof or a prodrug thereof, or
a compound represented by the formula (II): 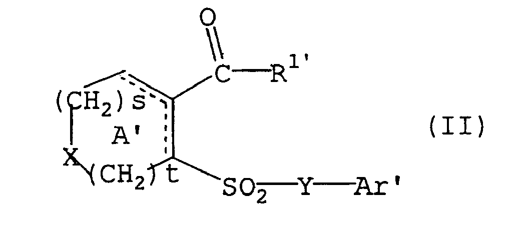 wherein
R^{1'} is an aliphatic hydrocarbon group optionally having substituent(s), an aromatic hydrocarbon group optionally having substituent(s), a heterocyclic group optionally having substituent(s), a group represented by the formula: -OR^{1a'} wherein R^{1a'} is a hydrogen atom or an aliphatic hydrocarbon group optionally having substituent(s), or a group represented by the formula: 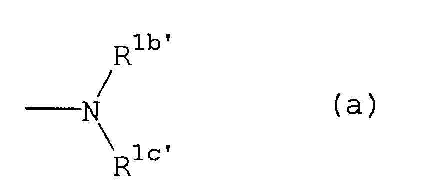 wherein R^{1b'} and R^{1c'} are the same or different and each is a hydrogen atom or an aliphatic hydrocarbon group optionally having substituent(s),
X is a methylene group, NH, a sulfur atom or an oxygen atom,
Y is a methylene group optionally having substituent(s) or NH optionally having substituent(s),
ring A' is a 5- to 8-membered ring optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR^{2'} wherein R^{2'} is a hydrogen atom or an aliphatic hydrocarbon group optionally having substituent(s), and (4) a halogen atom, Ar' is an aromatic hydrocarbon group optionally having substituent(s),
a group represented by the formula: 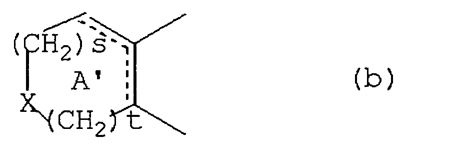 is a group represented by the formula: 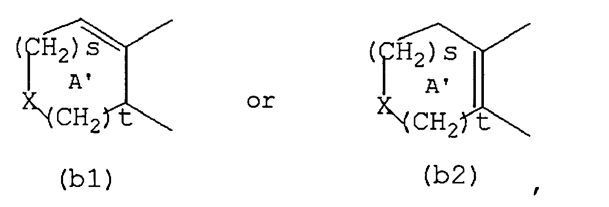 s is an integer of 0 to 2,
t is an integer of 1 to 3, and
the total of s and t is 4 or less;
provided that when X is a methylene group, then Y should be a methylene group optionally having substituent(s),
or a salt thereof or a prodrug thereof,
for the production of an agent for the prophylaxis or
treatment of cardiac disease, autoimmune diseases, central nervous system diseases, inflammatory disease, sepsis, severe sepsis or septic shock in a patient who undergoes coronary-artery bypass surgery.

9. An agent for the prophylaxis or treatment of cardiac disease, autoimmune disease, central nervous system disease, inflammatory disease, sepsis, severe sepsis, septic shock or complications after coronary-artery bypass surgery, comprising ethyl (6R)-6-[(2-chloro-4-fluoroanilino)sulfonyl]-1-cyclohexene-1-carboxylate or a salt thereof or a prodrug thereof, which is prepared for administration of ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight for about 15 min to about 240 min.

10. A method for the prophylaxis or treatment of cardiac disease, autoimmune diseases, central nervous system diseases, inflammatory disease, sepsis, severe sepsis, septic shock or complications after coronary-artery bypass surgery, which comprises administering ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight for about 15 min to about 240 min, to a mammal.

11. Use of ethyl (6R)-6-[(2-chloro-4-fluoroanilino)sulfonyl]-1-cyclohexene-1-carboxylate or a salt thereof or a prodrug thereof, for the production of an agent for the prophylaxis or treatment of cardiac disease, autoimmune diseases, central nervous system diseases, inflammatory disease, sepsis, severe sepsis, septic shock or complications after coronary-artery bypass surgery, wherein the agent is prepared for administration of ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight for about 15 min to about 240 min.

12. An agent for the prophylaxis or treatment of cardiac disease, autoimmune diseases, central nervous system diseases, inflammatory disease, sepsis, severe sepsis, septic shock or complications after coronary-artery bypass surgery, comprising ethyl (6R)-6-[(2-chloro-4-fluoroanilino)sulfonyl]-1-cyclohexene-1-carboxylate or a salt thereof or a prodrug thereof, which is prepared for administration of ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight per administration for about 15 min to about 240 min per administration, 1 to 6 times a day, for 1 day to 1 month.

13. A method for the prophylaxis or treatment of cardiac disease, autoimmune diseases, central nervous system diseases, inflammatory disease, sepsis, severe sepsis, septic shock or complications after coronary-artery bypass surgery, which comprises administering ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight per administration for about 15 min to about 240 min per administration, 1 to 6 times a day, for 1 day to 1 month, to a mammal.

14. Use of ethyl (6R)-6-[(2-chloro-4-fluoroanilino)sulfonyl]-1-cyclohexene-1-carboxylate or a salt thereof or a prodrug thereof, for the production of an agent for the prophylaxis or treatment of cardiac disease, autoimmune diseases, central nervous system diseases, inflammatory disease, sepsis, severe sepsis, septic shock or complications after coronary-artery bypass surgery, wherein the agent is prepared for administration of ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight per administration for about 15 min to about 240 min per administration, 1 to 6 times a day, for 1 day to 1 month.

15. An agent for the prophylaxis or treatment of cardiac disease, autoimmune diseases, central nervous system diseases, inflammatory disease, sepsis, severe sepsis, septic shock or complications after coronary-artery bypass surgery, comprising ethyl (6R)-6-[(2-chloro-4-fluoroanilino)sulfonyl]-1-cyclohexene-1-carboxylate or a salt thereof or a prodrug thereof, which is prepared for continuous drip of ethyl (6R)-6-[(2-chloro-4-fluoroanilino)sulfonyl]-1-cyclohexene-1-carboxylate or a salt thereof or a prodrug thereof at a dose of about 0.01 mg/kg body weight/hr to about 0.3 mg/kg body weight/hr for about 1 hr to about 1 month.

16. A method for the prophylaxis or treatment of cardiac disease, autoimmune diseases, central nervous system diseases, inflammatory disease, sepsis, severe sepsis, septic shock or complications after coronary-artery bypass surgery, which comprises giving continuous drip of ethyl (6R)-6-[(2-chloro-4-fluoroanilino)sulfonyl]-1-cyclohexene-1-carboxylate or a salt thereof or a prodrug thereof at a dose of about 0.01 mg/kg body weight/hr to about 0.3 mg/kg body weight/hr for about 1 hr to about 1 month, to a mammal.

17. Use of ethyl (6R)-6-[(2-chloro-4-fluoroanilino)sulfonyl]-1-cyclohexene-1-carboxylate or a salt thereof or a prodrug thereof, for the production of an agent for the prophylaxis or treatment of cardiac disease, autoimmune diseases, central nervous system diseases, inflammatory disease, sepsis, severe sepsis, septic shock or complications after coronary-artery bypass surgery, wherein the agent is prepared for continuous drip of ethyl (6R)-6-[(2-chloro-4-fluoroanilino)sulfonyl]-1-cyclohexene-1-carboxylate or a salt thereof or a prodrug thereof at a dose of about 0.01 mg/kg body weight/hr to about 0.3 mg/kg body weight/hr for about 1 hr to about 1 month.

18. An agent for the prophylaxis or treatment of cardiac disease, autoimmune diseases, central nervous system diseases, inflammatory disease, sepsis, severe sepsis, septic shock or complications after coronary-artery bypass surgery, comprising ethyl (6R)-6-[(2-chloro-4-fluoroanilino)sulfonyl]-1-cyclohexene-1-carboxylate or a salt thereof or a prodrug thereof, which is prepared for administration of ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight for about 15 min to about 240 min, and then for continuous drip of ethyl (6R)-6-[(2-chloro-4-fluoroanilino)sulfonyl]-1-cyclohexene-1-carboxylate or a salt thereof or a prodrug thereof at a dose of about 0.01 mg/kg body weight/hr to about 0.3 mg/kg body weight/hr for about 1 hr to about 1 month.

19. A method for the prophylaxis or treatment of cardiac disease, autoimmune diseases, central nervous system diseases, inflammatory disease, sepsis, severe sepsis, septic shock or complications after coronary-artery bypass surgery, which comprises administering ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight for about 15 min to about 240 min, and then giving continuous drip of ethyl (6R)-6-[(2-chloro-4-fluoroanilino)sulfonyl]-1-cyclohexene-1-carboxylate or a salt thereof or a prodrug thereof at a dose of about 0.01 mg/kg body weight/hr to about 0.3 mg/kg body weight/hr for about 1 hr to about 1 month, to a mammal.

20. Use of ethyl (6R)-6-[(2-chloro-4-fluoroanilino)sulfonyl]-1-cyclohexene-1-carboxylate or a salt thereof or a prodrug thereof, for the production of an agent for the prophylaxis or treatment of cardiac disease, autoimmune diseases, central nervous system diseases, inflammatory disease, sepsis, severe sepsis, septic shock or complications after coronary-artery bypass surgery, wherein the agent is prepared for administration of ethyl (6R)-6-[(2-chloro-4-fluoroanilino)sulfonyl]-1-cyclohexene-1-carboxylate or a salt thereof or a prodrug thereof at a dose of about 0.2 mg/kg body weight to about 2.4 mg/kg body weight for about 15 min to about 240 min, and then for continuous drip of ethyl (6R)-6-[(2-chloro-4-fluoroanilino)sulfonyl]-1-cyclohexene-1-carboxylate or a salt thereof or a prodrug thereof at a dose of about 0.01 mg/kg body weight/hr to about 0.3 mg/kg body weight/hr for about 1 hr to about 1 month.

21. The agent of claim 9, 12, 15 or 18, wherein ethyl (6R)-6-[(2-chloro-4-fluoroanilino)sulfonyl]-1-cyclohexene-1-carboxylate or a salt thereof or a prodrug thereof is used in the form of an emulsion composition having a pH adjusted to about 3.7 to about 5.5, which comprises the compound and a buffer.

22. The method of claim 10, 13, 16 or 19, wherein ethyl (6R)-6-[(2-chloro-4-fluoroanilino)sulfonyl]-1-cyclohexene-1-carboxylate or a salt thereof or a prodrug thereof is used in the form of an emulsion composition having a pH adjusted to about 3.7 to about 5.5, which comprises the compound and a buffer.

23. The use of claim 11, 14, 17 or 20, wherein ethyl (6R)-6-[(2-chloro-4-fluoroanilino)sulfonyl]-1-cyclohexene-1-carboxylate or a salt thereof or a prodrug thereof is used in the form of an emulsion composition having a pH adjusted to about 3.7 to about 5.5, which comprises the compound and a buffer.
